# EUROPEAN PATENT APPLICATION

(11) **EP 4 786 458 A1**
(43) Date of publication of application: **05.08.2026**
(21) Application number: 24871010.5
(22) Date of filing: 27.09.2024
(51) Int. Cl.: C07D 333/16, A61K 31/341, A61K 31/381, A61P 25/04

(54) **ANALGESIC COMPOUND, PREPARATION METHOD THEREFOR, AND USE THEREOF**

(30) Priority: 28.09.2023 CN 202311275169
(71) Applicant: West China Hospital, Sichuan University, Chengdu, Sichuan 610041 (CN)
(72) Inventor: ZHANG, Wensheng, Chengdu, Sichuan 610041 (CN); DENG, Chaoyi, Chengdu, Sichuan 610041 (CN); LUO, Xudong, Chengdu, Sichuan 610041 (CN); DENG, Yu, Chengdu, Sichuan 610041 (CN); LIU, Jin, Chengdu, Sichuan 610041 (CN); LI, Chenyang, Chengdu, Sichuan 610041 (CN); LU, Junyu, Chengdu, Sichuan 610041 (CN); ZHU, Tao, Chengdu, Sichuan 610041 (CN); LUO, Fengming, Chengdu, Sichuan 610041 (CN)
(74) Representative: Altmann Stößel Dick Patentanwälte PartG mbB
(86) International application number: PCT/CN2024/121889
(87) International publication number: WO 2025/067475

(57) **Abstract**

Provided are an analgesic compound, a preparation method therefor, and the use thereof, which relate to the field of pharmaceutical chemistry. The compound is a compound represented by formula I, or a stereoisomer thereof, or a pharmaceutically acceptable salt thereof, or a solvate thereof, or a crystal form thereof, or a prodrug thereof, or a metabolite thereof, or a deuterated derivative thereof. The compound has a uniquely advantageous analgesic effect, is highly safe when used, has only mild toxic side effects, and no dependence develops during use. Therefore, the compound has wide application prospects in the preparation of analgesic pharmaceuticals, and provides a new option when clinically preparing a drug with an analgesic effect.

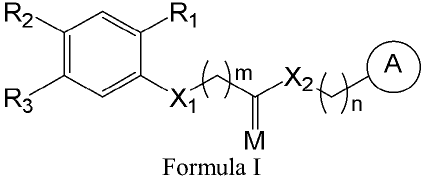

## Description

### Field of the invention

The present invention relates to the field of medicinal chemistry, and specifically to a class of analgesic compounds, preparation methods therefor, and uses thereof.

### Background of the invention

The Global Burden of Disease reports that pain and pain-related diseases are the primary causes of global disability and disease burden. The report indicates that over 80% of patients who undergo surgery experience acute postoperative pain, and less than half of these patients receive adequate postoperative pain relief. Among them, nearly 80% have moderate to severe pain scores, and 10% to 50% of patients develop chronic postoperative pain. Therefore, there is still an urgent need for analgesic drugs in clinical practice. In the field of pain treatment, traditional opioids remain the most effective and commonly used analgesic drugs for treating moderate to severe pain. Opioids play their analgesic effect by acting on opioid receptors in the G protein-coupled receptor family, primarily activating the downstream Gi/o protein pathway.

However, while opioids exert potent analgesic effects, they are also accompanied by numerous adverse reactions, such as common respiratory depression, deep sedation, nausea and vomiting, constipation, etc., as well as tolerance and decreased pain sensation caused by long-term use, which can even lead to drug abuse, addiction, and severe social harm. Opioids acting on µ receptors (such as remifentanil) can produce dose-dependent respiratory depression by direct action on the respiratory center of the brainstem. Studies have shown that the average addiction rate of opioids is between 8% and 12%. Patients with physical dependence or addiction to opioids often abuse opioids to avoid withdrawal symptoms.

Although researchers have successively developed many new opioid analgesics and even non-opioid analgesics over the past century, no particularly significant progress has been made. For example, Oliceridine (TRV130), a targeted µ-receptor analgesic designed based on the concept of G protein bias, was approved for marketing by the U.S. Food and Drug Administration (FDA) in 2020 for the treatment of moderate to severe pain. However, it still carries a "black box warning" emphasizing its potential opioid-related side effects. Many known analgesics based on other non-opioid receptor targets have not achieved analgesic effects comparable to morphine or remifentanil due to limitations in analgesic targets or pain models, or have failed to produce consistent results in multiple clinical studies compared to animal experiments.

In summary, although traditional opioids used clinically are the most effective drugs for treating moderate to severe pain, the accompanying adverse reactions and drug dependency limit the effectiveness of pain treatment. While reducing safety, they also lead to serious social issues such as drug abuse. Therefore, designing a novel compound that retains the analgesic efficacy of opioids while avoiding their severe adverse reactions, as a new type of non-opioid analgesic drug, holds significant clinical importance and broad market prospects.

### Summary of the invention

The objective of the present invention is to provide a class of analgesic compounds, preparation methods therefor, and uses thereof.

The present invention provides a compound represented by formula I, or a stereoisomer thereof, or a pharmaceutically acceptable salt thereof, or a solvate thereof, or a crystal form thereof, or a prodrug thereof, or a metabolite thereof, or a deuterated derivative thereof: 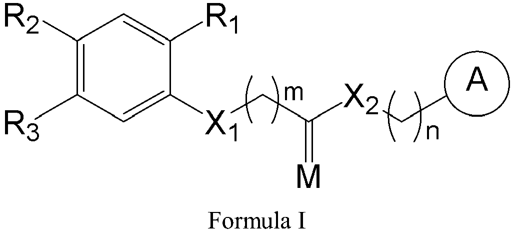 wherein,
R₁ and R₃ are each independently selected from H or NR₄R₅, and only one of R₁ and R₃ is selected from NR₄R₅;
R₂ is selected from the group consisting of H, substituted or unsubstituted C₁-C₆ alkyl, halogen, and hydroxyl;
R₄ and R₅ are each independently selected from the group consisting of substituted or unsubstituted C₁-C₆ alkyl, and substituted or unsubstituted C₁-C₆ alkoxy;
X₁ and X₂ are each independently selected from the group consisting of O, S, and NR₆;
R₆ is selected from the group consisting of H and substituted or unsubstituted C₁-C₆ alkyl;
m is selected from an integer of 1 to 5; n is selected from an integer of 0 to 5;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms ranges from 1 to 5.

Further, the compound is as represented by formula II: 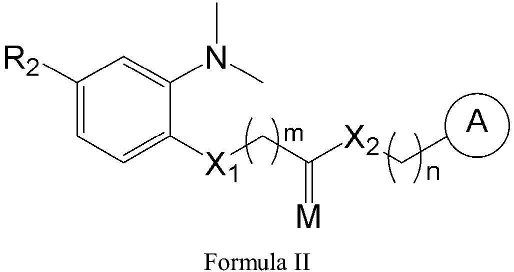 wherein,
R₂ is selected from the group consisting of H, substituted or unsubstituted C₁-C₆ alkyl, halogen, and hydroxyl;
X₁ and X₂ are each independently selected from the group consisting of O, S, and NR₆;
R₆ is selected from the group consisting of H and substituted or unsubstituted C₁-C₆ alkyl;
m is selected from 1, 2, 3, 4, or 5; n is selected from 0, 1, 2, 3, 4, or 5;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

Further, the compound is as represented by formula III: 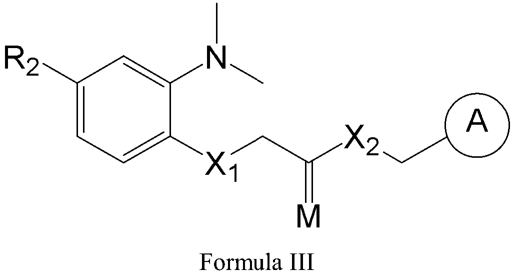 wherein,
R₂ is selected from the group consisting of H, substituted or unsubstituted C₁-C₆ alkyl, halogen, and hydroxyl;
X₁ and X₂ are each independently selected from the group consisting of O, S, and NR₆;
R₆ is selected from the group consisting of H and substituted or unsubstituted C₁-C₆ alkyl;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

Further, the compound is as represented by formula Iva, formula IVb, or formula IVc: 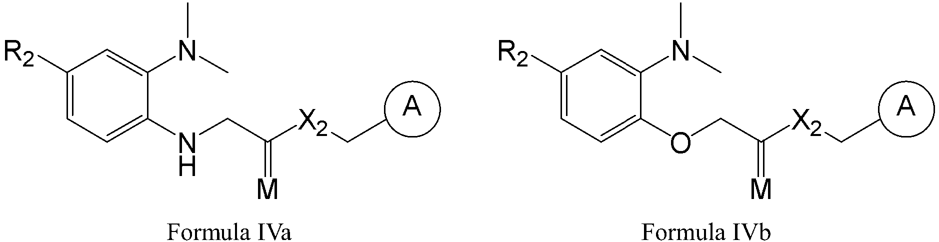 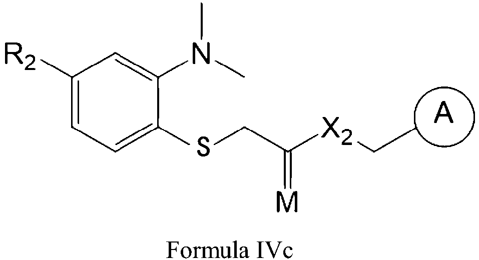 wherein,
R₂ is selected from the group consisting of H, substituted or unsubstituted C₁-C₆ alkyl, halogen, and hydroxyl;
X₂ is selected from the group consisting of O, S, and NR₆;
R₆ is selected from the group consisting of H and substituted or unsubstituted C₁-C₆ alkyl;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

Further, the compound is as represented by formula V: 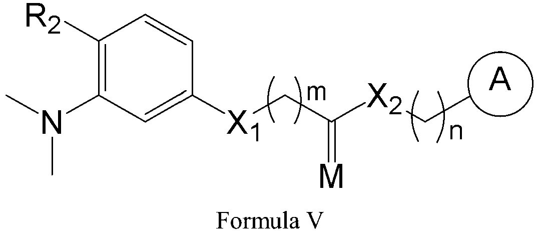 wherein,
R₂ is selected from the group consisting of H, substituted or unsubstituted C₁-C₆ alkyl, halogen, and hydroxyl;
X₁ and X₂ are each independently selected from the group consisting of O, S, and NR₆;
R₆ is selected from the group consisting of H and substituted or unsubstituted C₁-C₆ alkyl;
m is selected from 1, 2, 3, 4, or 5; n is selected from 0, 1, 2, 3, 4, or 5;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

Further, the compound is as represented by formula VI: 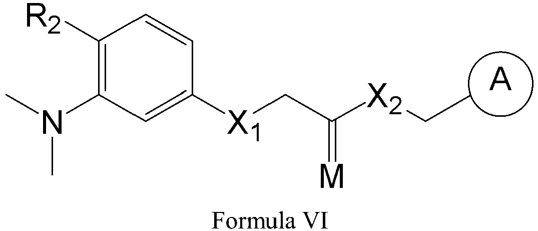 wherein,
R₂ is selected from the group consisting of H, substituted or unsubstituted C₁-C₆ alkyl, halogen, and hydroxyl;
X₁ and X₂ are each independently selected from the group consisting of O, S, and NR₆;
R₆ is selected from the group consisting of H and substituted or unsubstituted C₁-C₆ alkyl;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

Further, the compound is as represented by formula VIIa, formula VIIb, or formula VIIc: 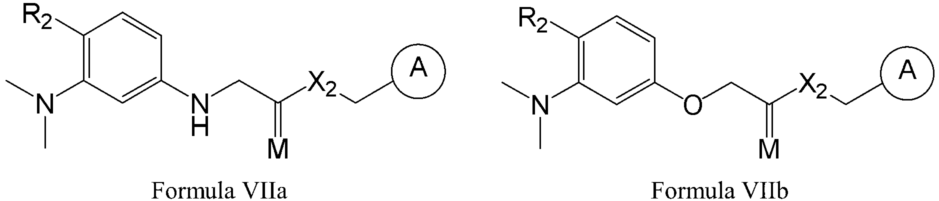 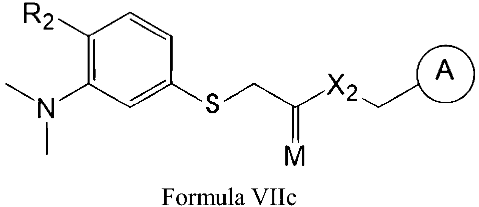 wherein,
R₂ is selected from the group consisting of H, substituted or unsubstituted C₁-C₆ alkyl, halogen, and hydroxyl;
X₂ is selected from the group consisting of O, S, and NR₆;
R₆ is selected from the group consisting of H and substituted or unsubstituted C₁-C₆ alkyl;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

Further,
X₂ is selected from the group consisting of O, S, and NH;
and/or, M is selected from the group consisting of O, S, and NH.

Further, ring A is selected from the group consisting of following substituted or unsubstituted groups: 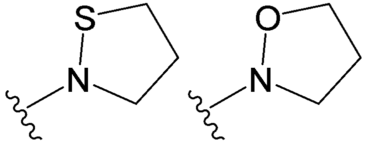 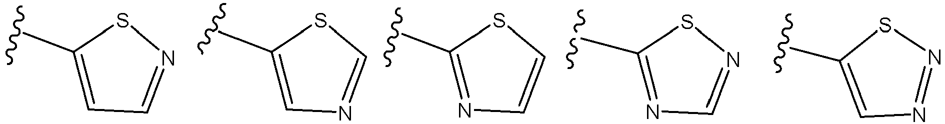 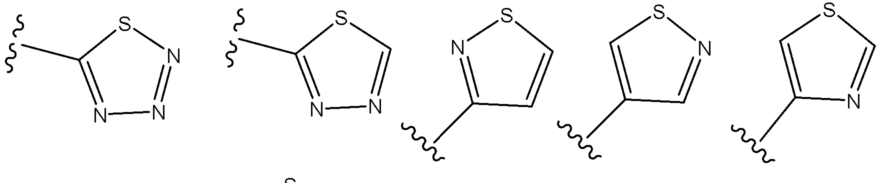 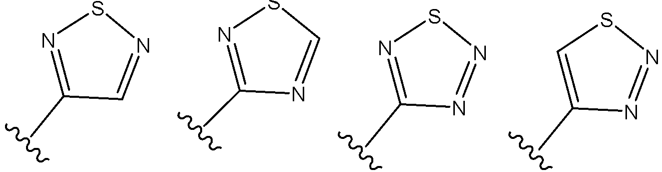 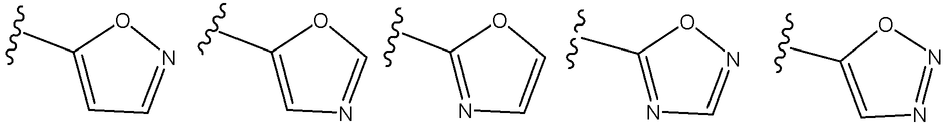 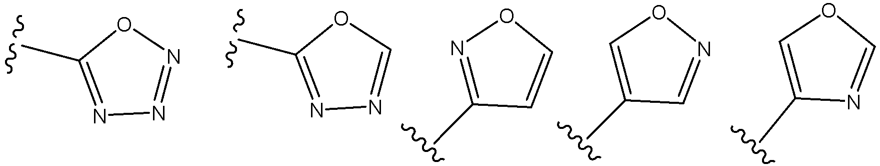 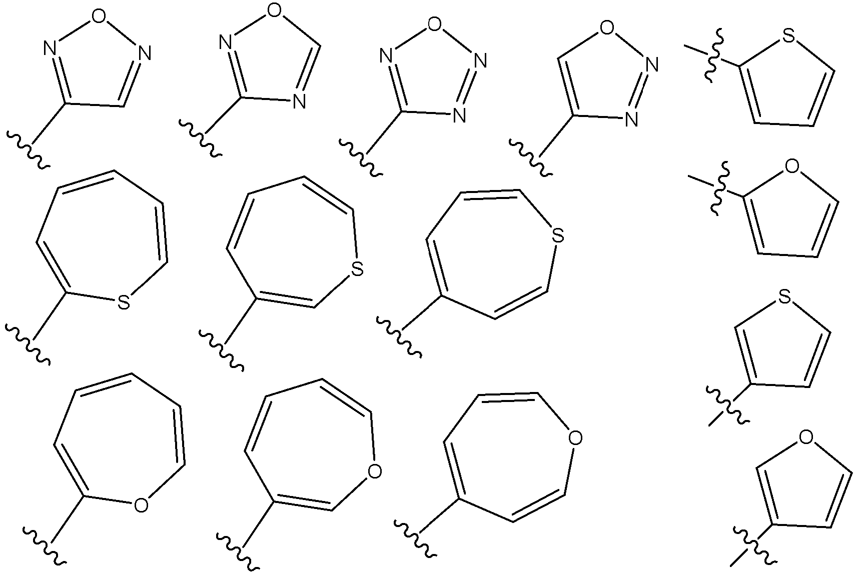 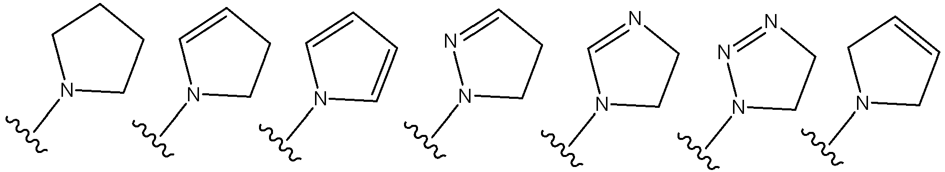 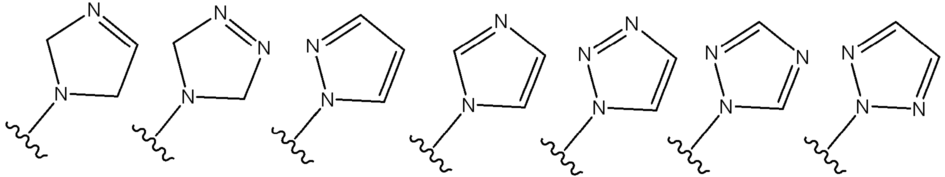 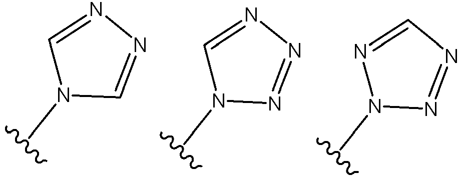 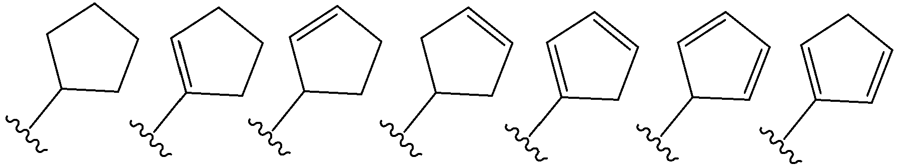 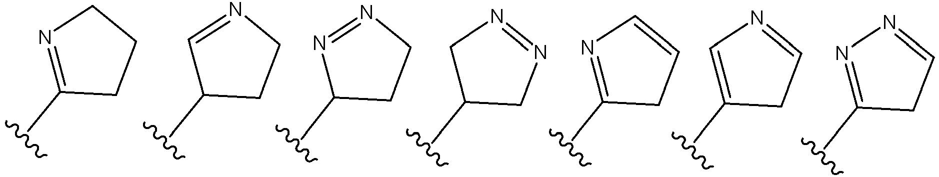 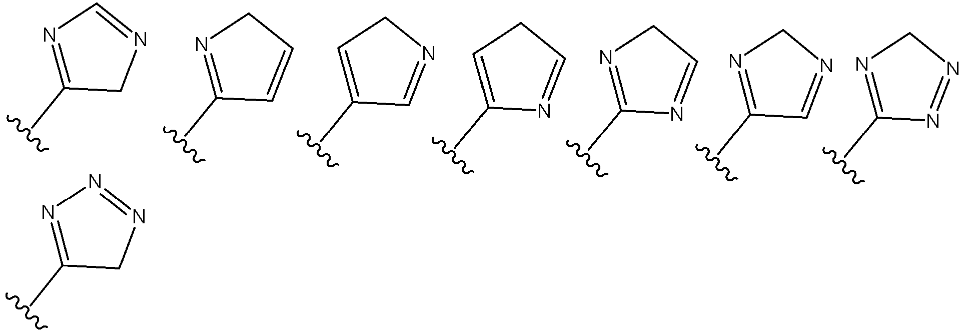 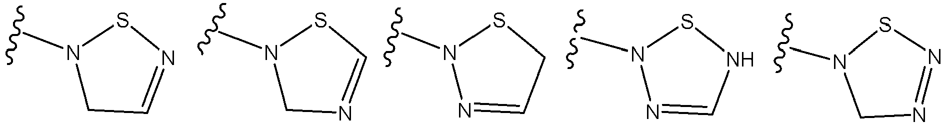 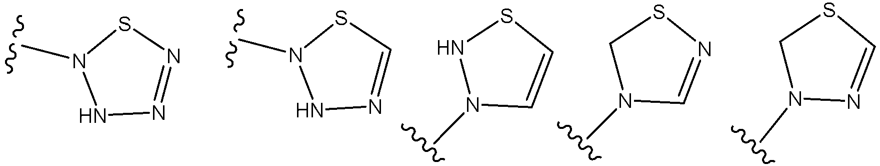 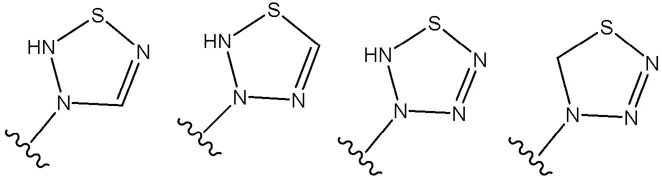 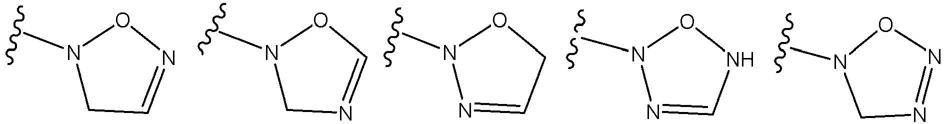 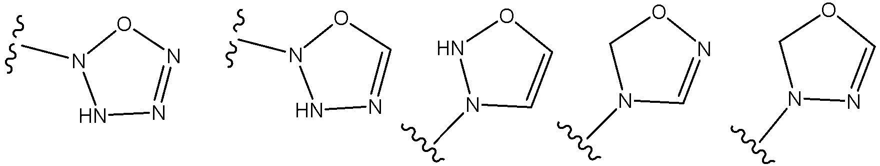 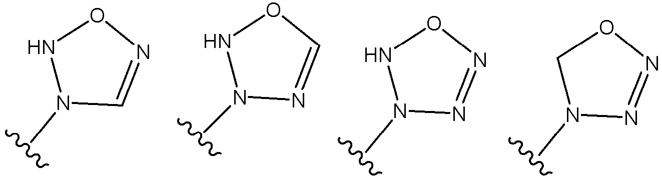 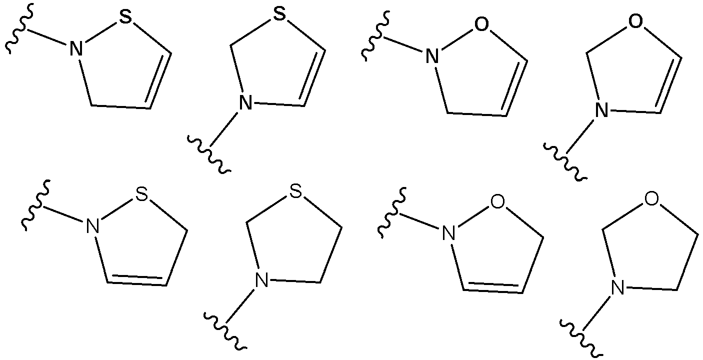 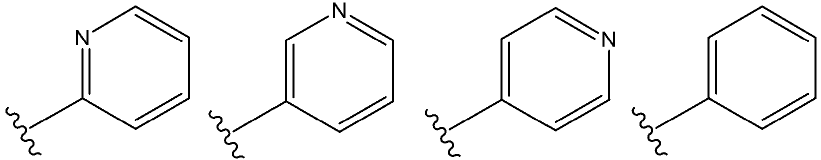 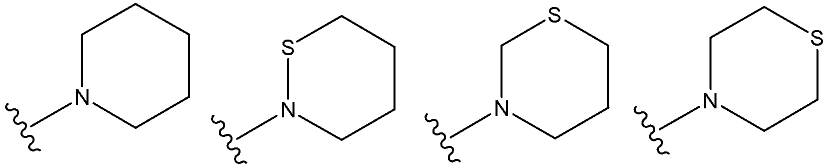 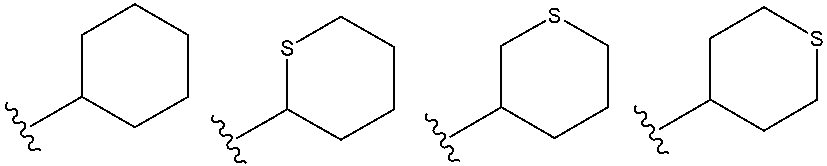 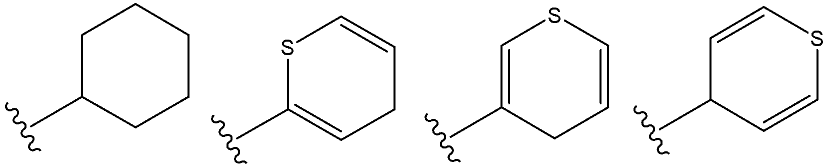 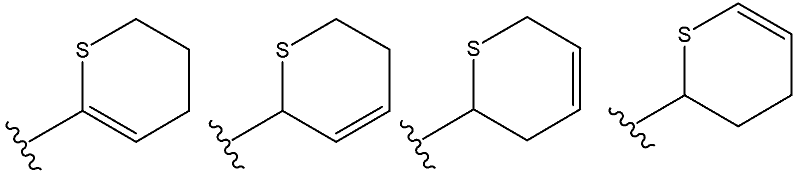 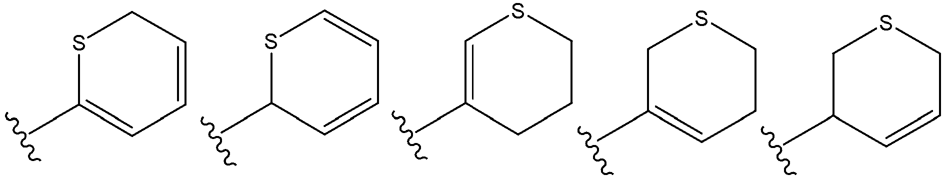 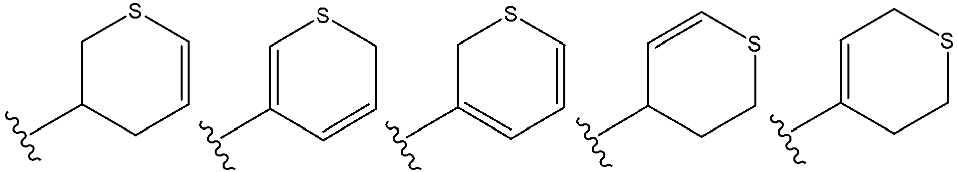 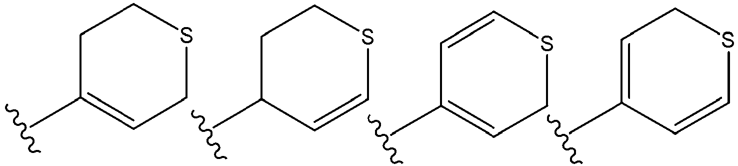 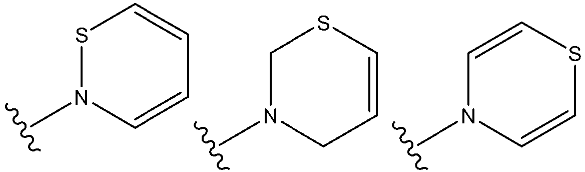 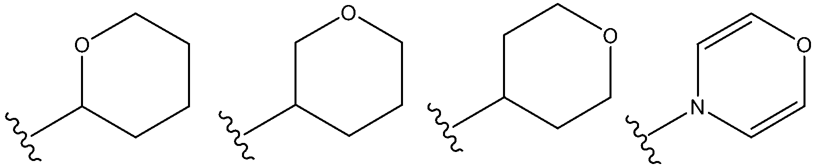 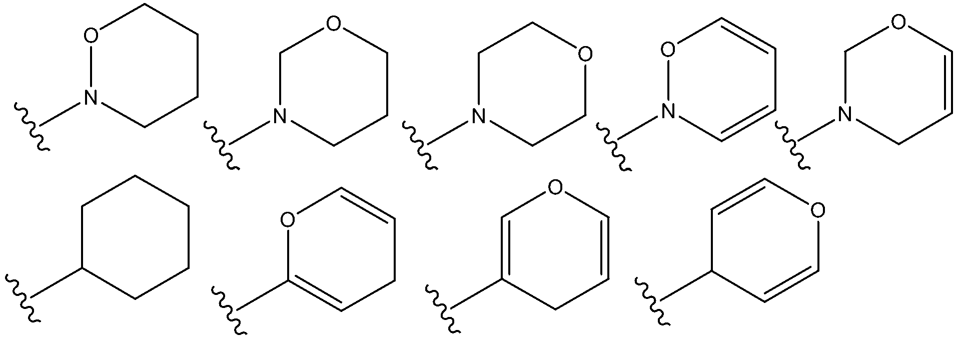 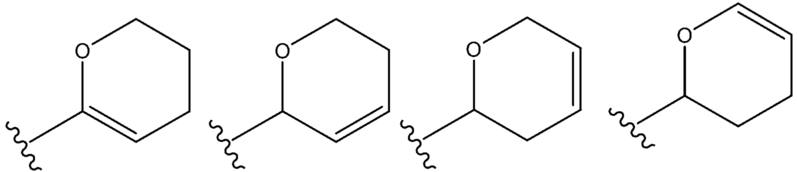 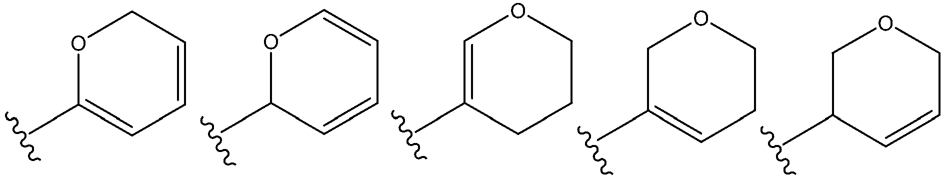 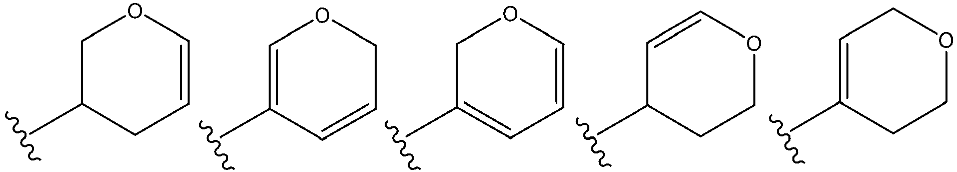 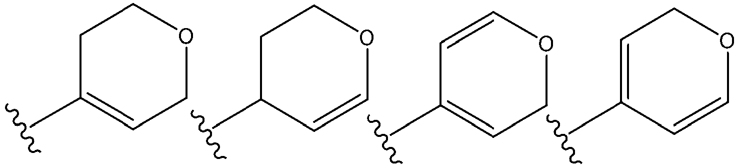 ;
the substituents of said ring A are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the substituents of said alkyl and alkoxy are selected from the group consisting of deuterium, halogen, amino, nitro, cyano, carboxyl, hydroxyl, and mercapto;

Further,
the substituents of said ring A are selected from the group consisting of deuterium, halogen, amino, nitro, cyano, carboxyl, hydroxyl, mercapto, substituted or unsubstituted C₁-C₄ alkyl, and substituted or unsubstituted C₁-C₄ alkoxy;
the substituents of said alkyl and alkoxy are selected from the group consisting of deuterium, halogen, amino, nitro, cyano, carboxyl, hydroxyl, and mercapto

Further, said compound is selected from the group consisting of following compounds:

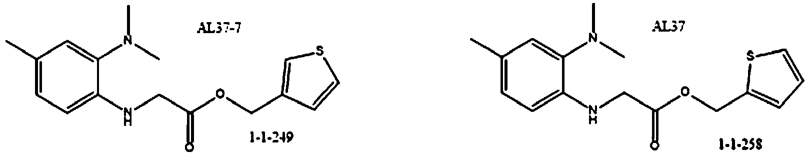

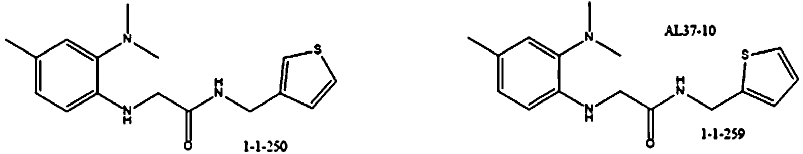

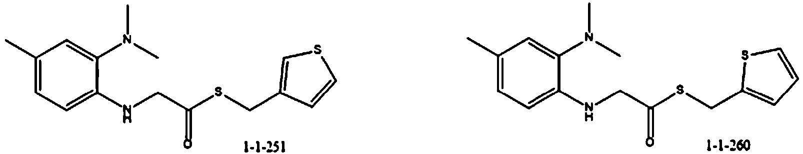

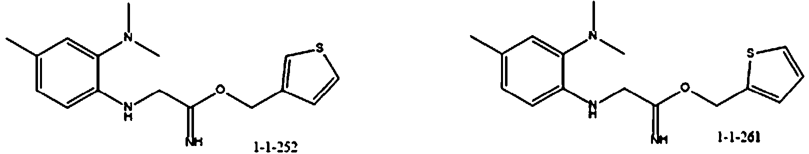

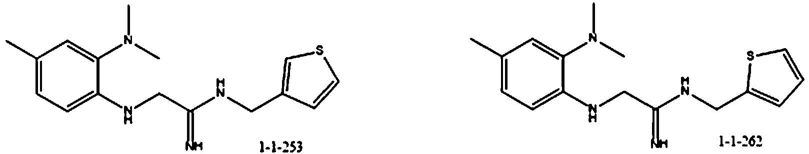

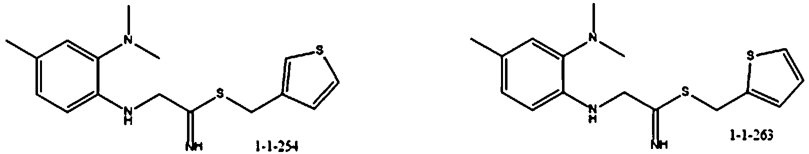

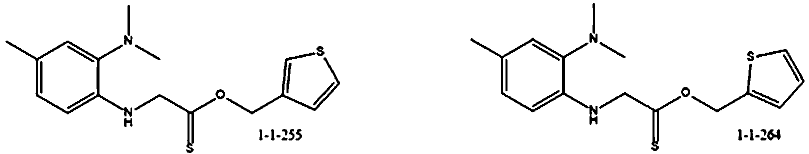

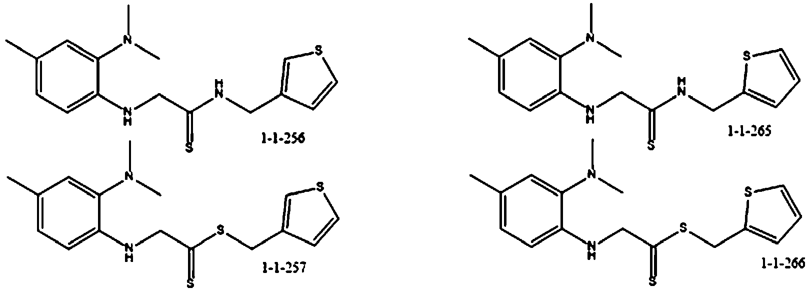

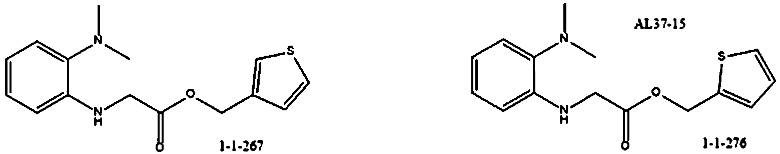

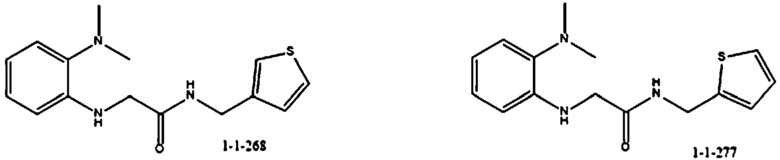

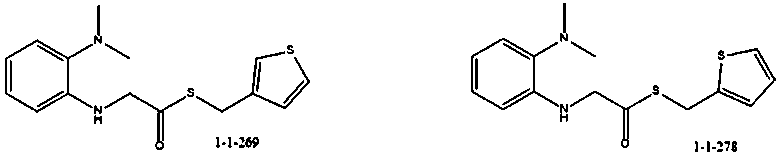

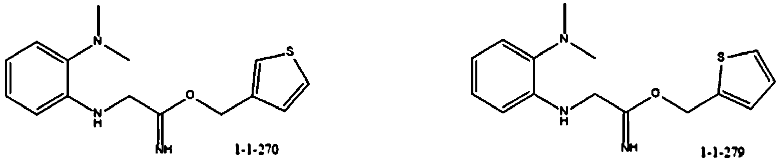

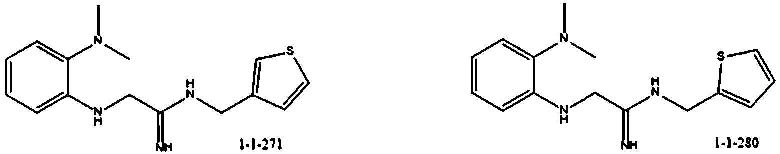

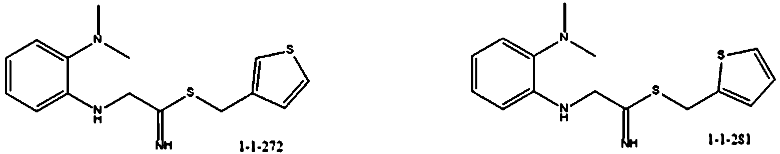

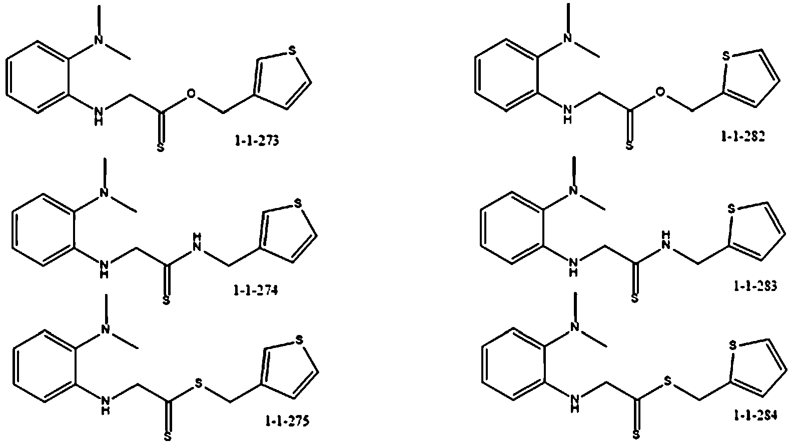

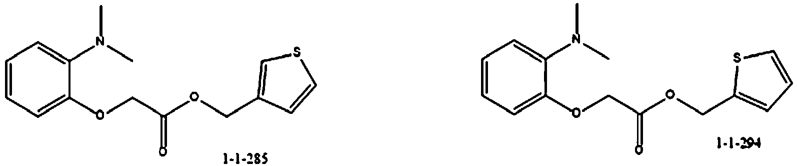

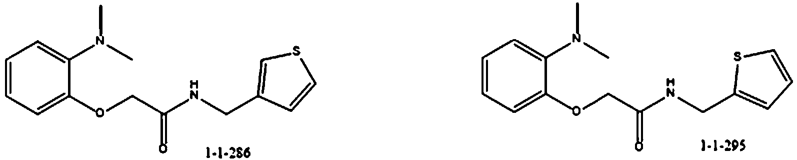

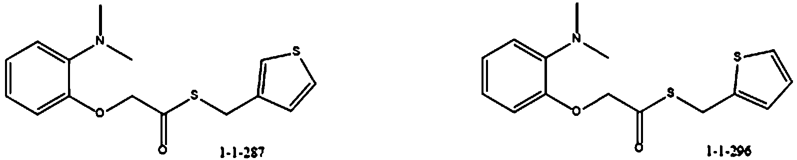

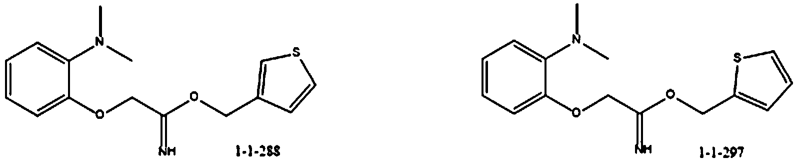

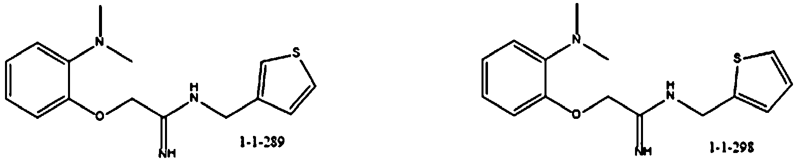

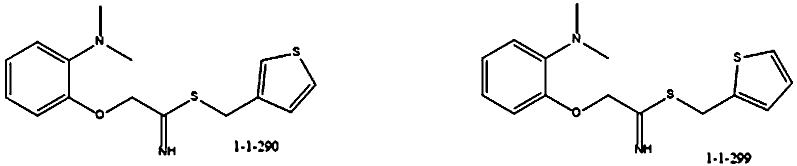

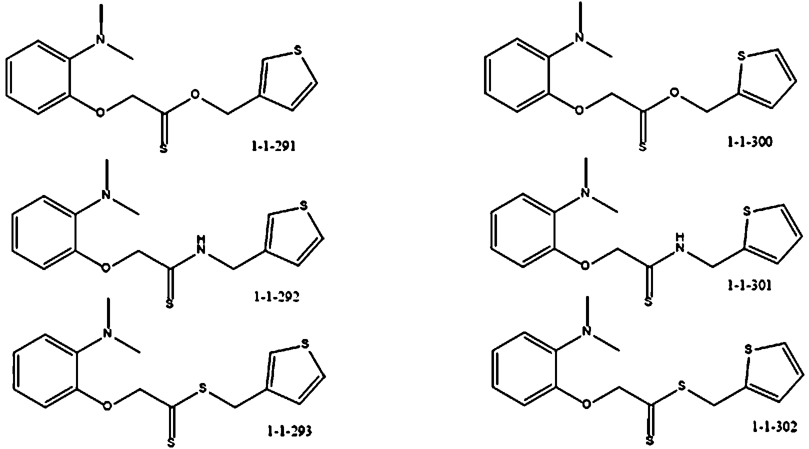

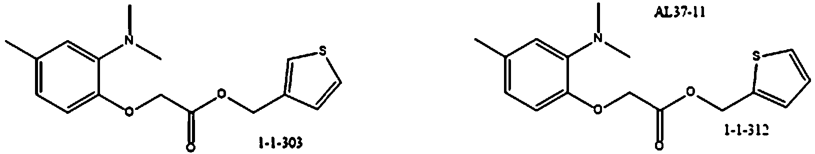

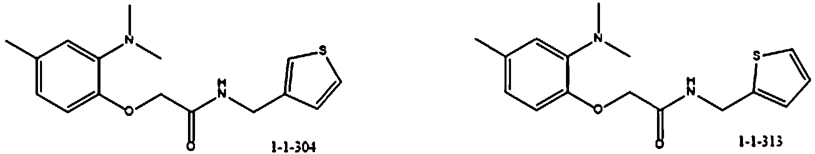

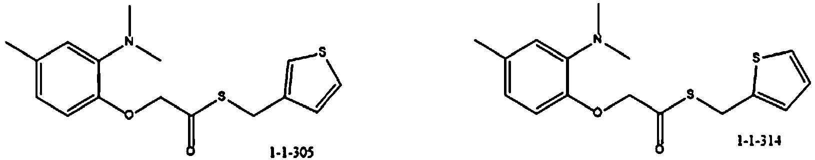

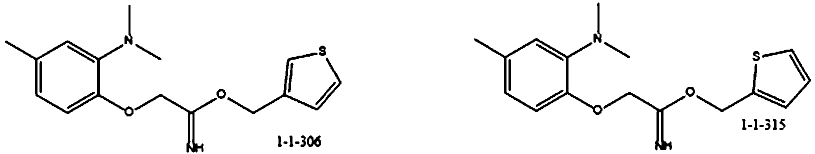

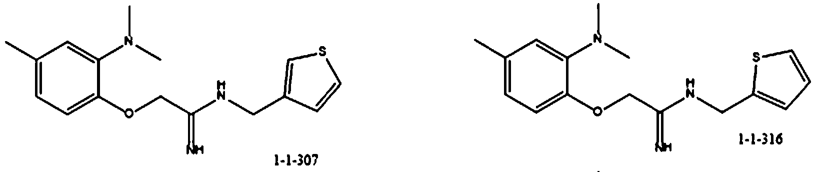

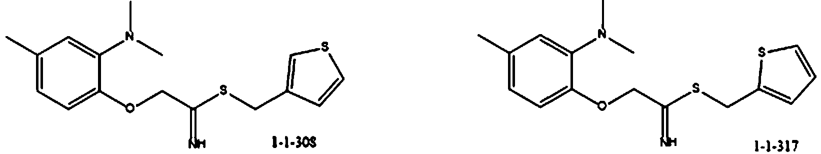

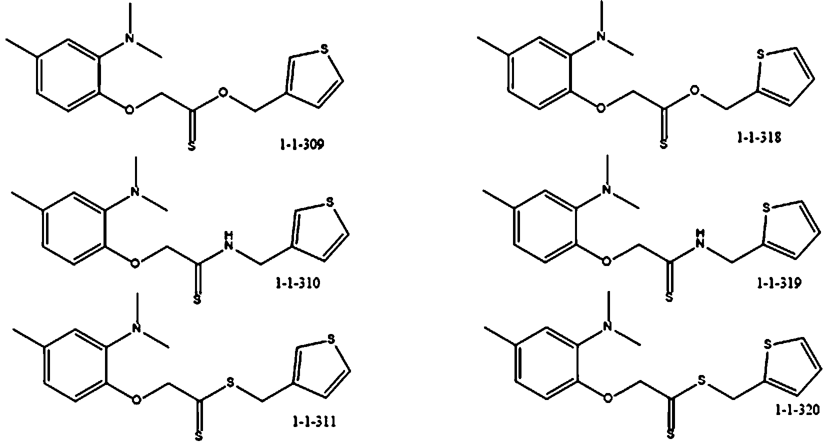

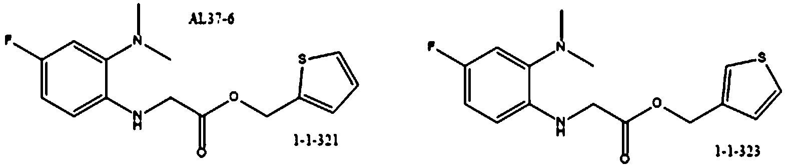

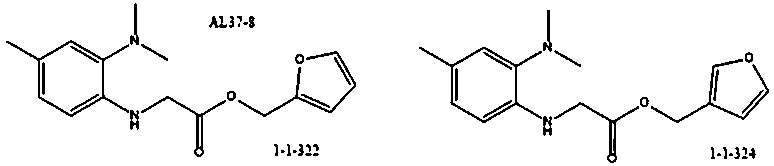

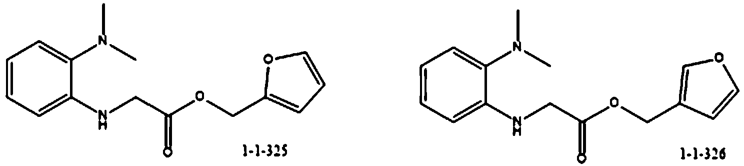

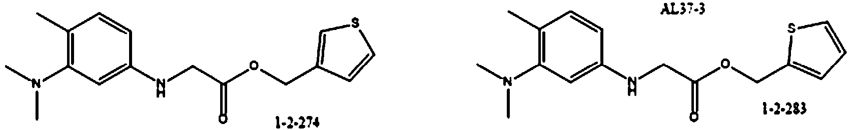

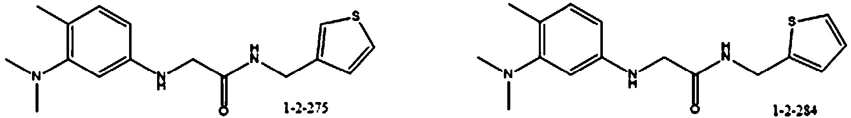

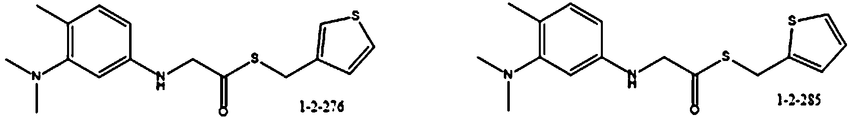

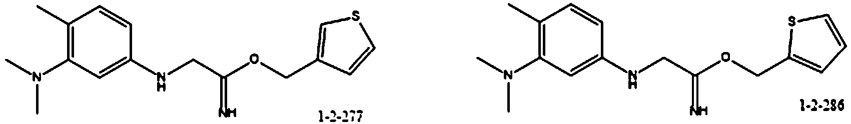

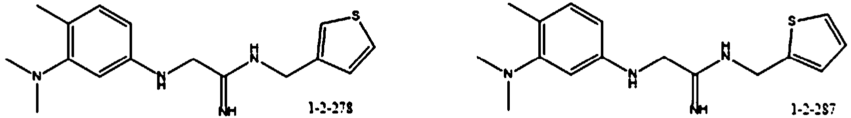

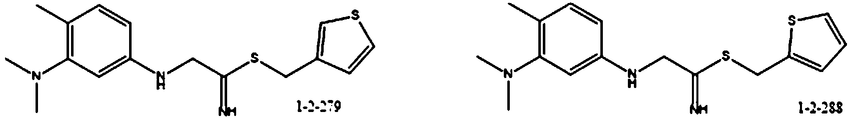

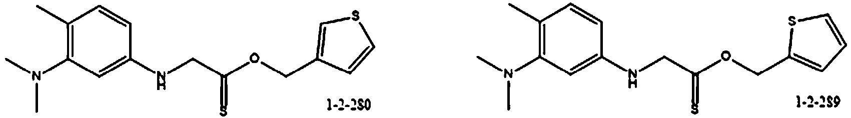

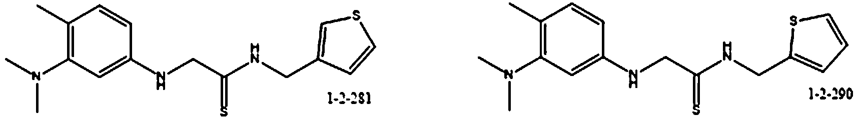

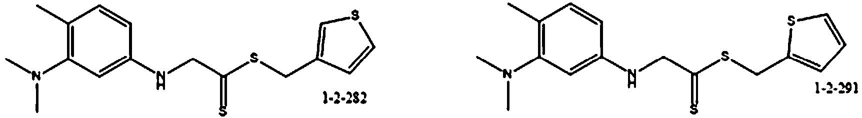

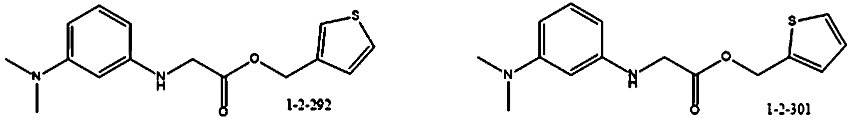

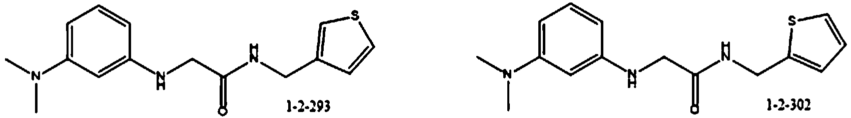

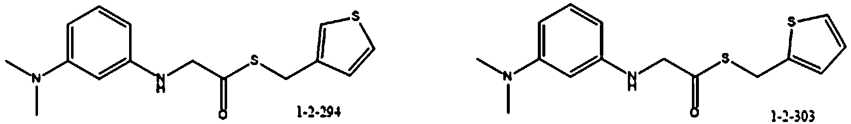

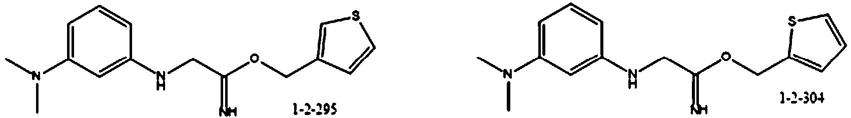

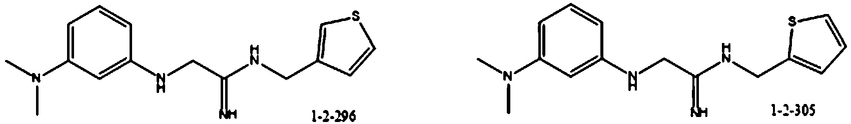

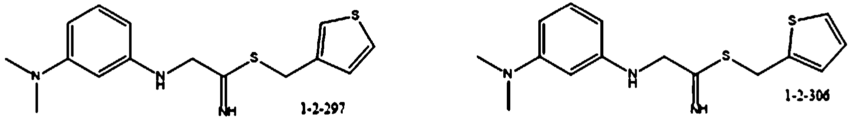

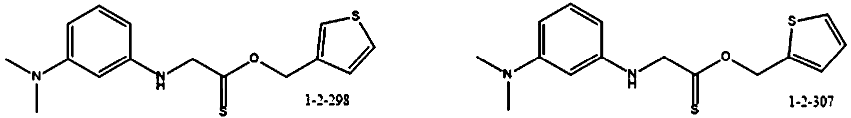

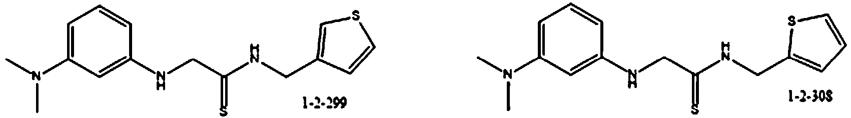

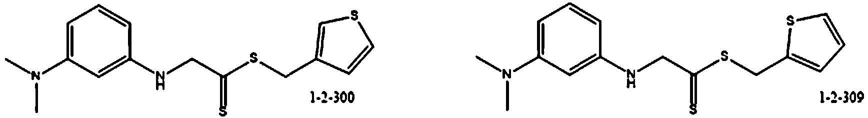

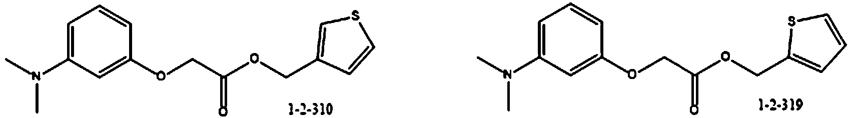

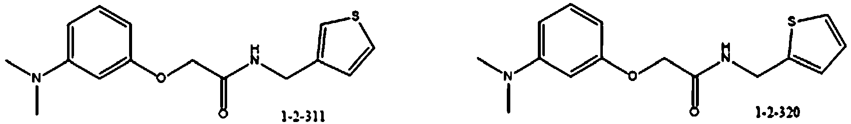

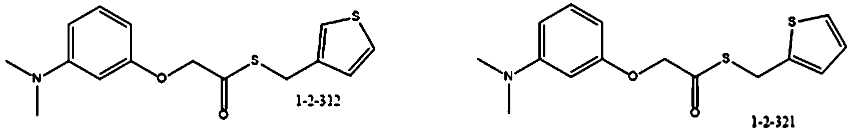

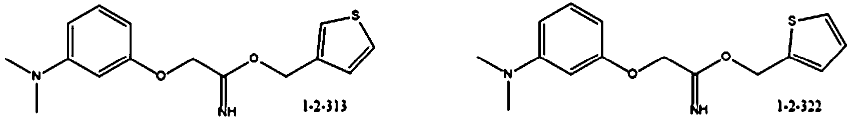

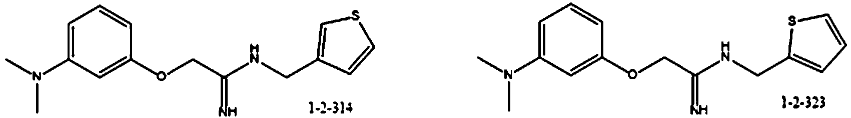

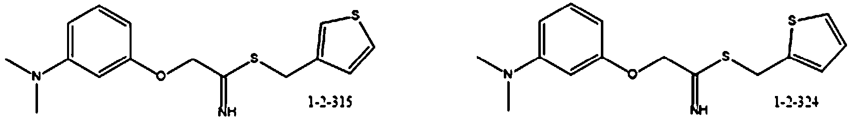

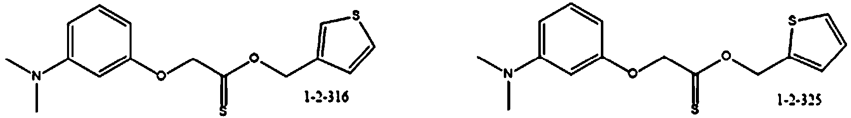

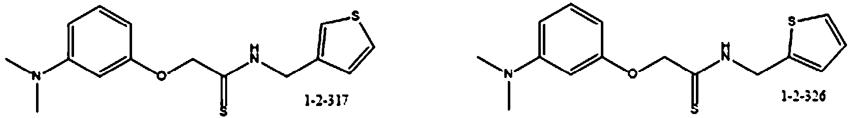

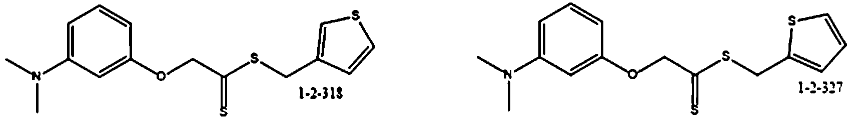

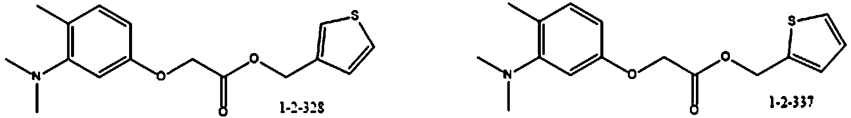

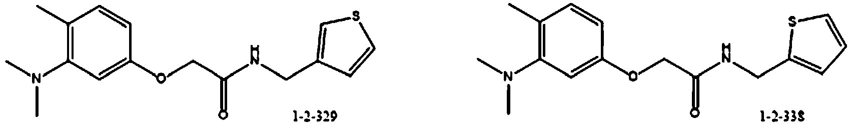

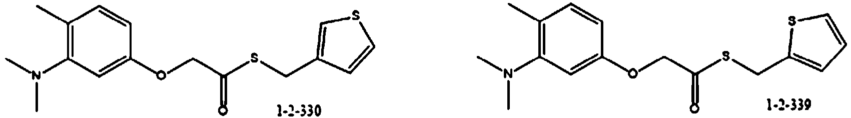

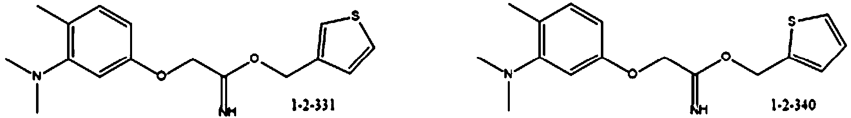

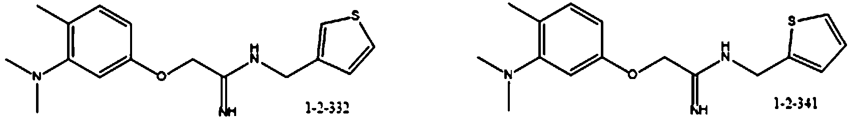

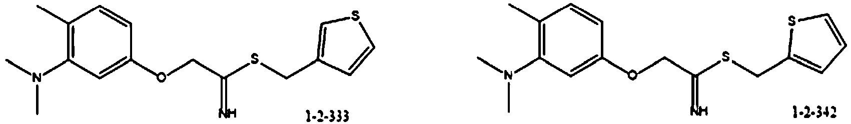

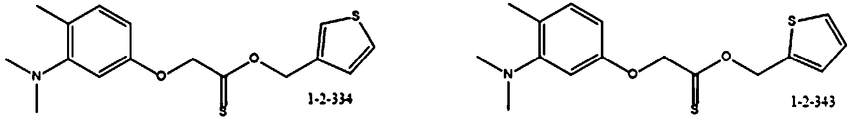

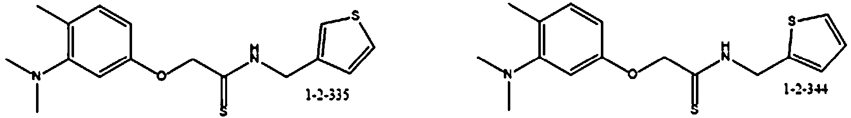

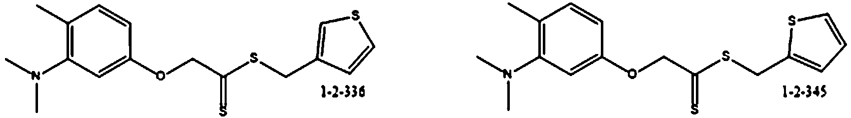

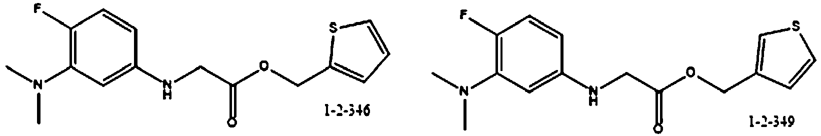

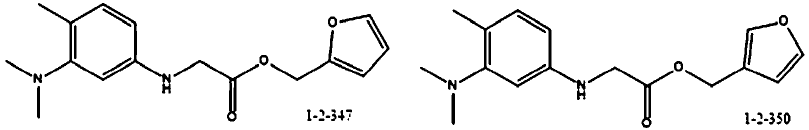

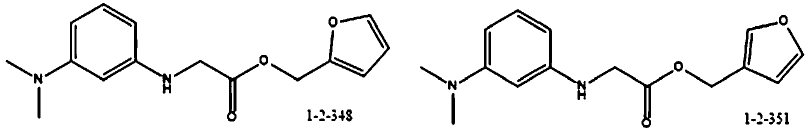

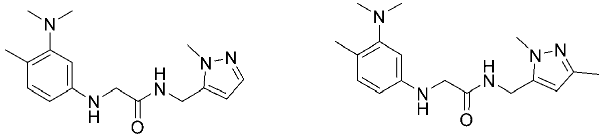

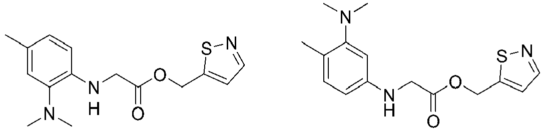

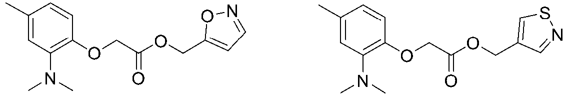

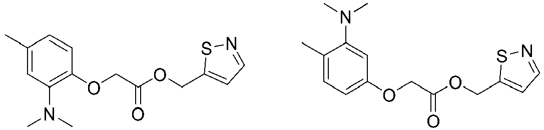

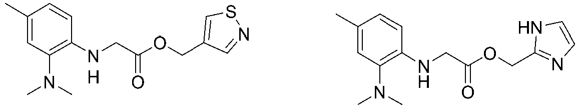

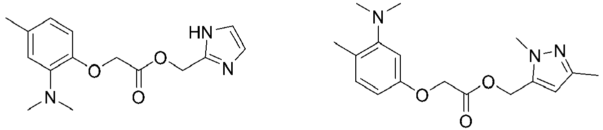

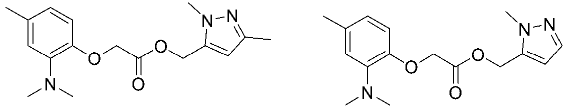

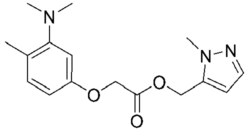

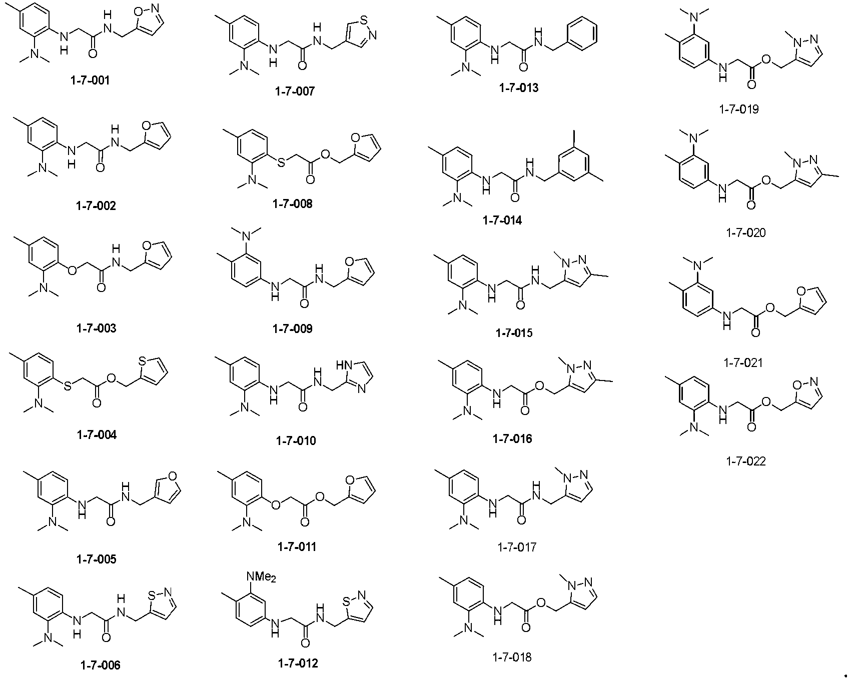

The present invention also provides a method for preparing the above compound, or a stereoisomer thereof, or a pharmaceutically acceptable salt thereof, or a solvate thereof, or a crystal form thereof, or a prodrug thereof, or a metabolite thereof, or a deuterated derivative thereof, which comprises the following steps:

### Step 1:

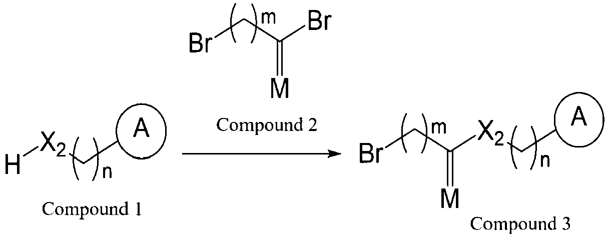

In a solvent, compound **1,** an organic base, and compound **2** react to obtain compound **3;** preferably, the organic base is pyridine;
wherein, n, m, X₂, A, and M are as defined above;

### Step 2:

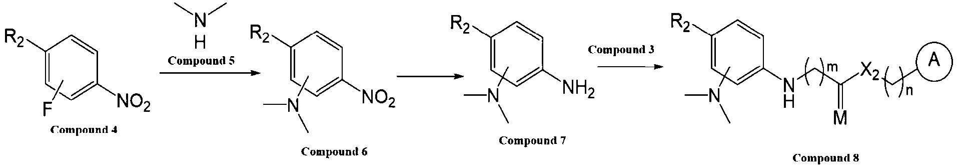

(1) In a solvent, compound **4** reacts with compound 5 to yield compound **6;**
(2) In a solvent, compound **6** reacts with Pd/C in a hydrogen atmosphere to yield compound **7;**
(3) In a solvent, compound **7,** an organic base, and compound **3** react to yield compound **8;** preferably, the organic base is DIPEA;
   wherein, R₂, n, m, X₂, A, and M are as defined above; 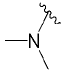 is in the ortho or meta position of R₂;
   alternatively, 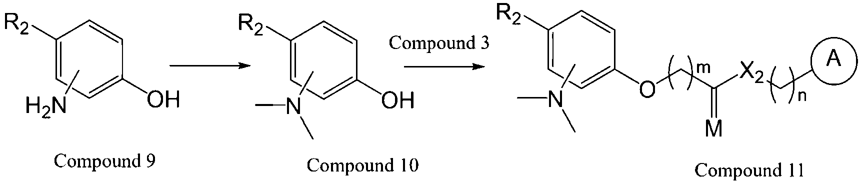
      (A) In a solvent, a compound, formaldehyde aqueous solution, acetic acid, and NaBH₃CN react to yield compound **10;**
      (B) In a solvent, compound **10,** an inorganic solid base, and compound **3** react to yield compound **11;** preferably, the inorganic solid base is Cs₂CO₃;
   wherein, R₂, n, m, X₂, A, and M are as defined above; 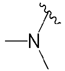 is in the ortho or meta position of R₂;
   alternatively, 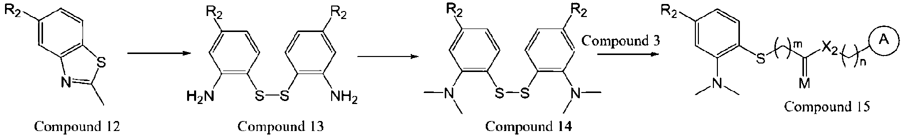
      (a) In a solvent, compound **12** reacts with an NaOH aqueous solution to yield compound **13;**
      (b) In a solvent, compound **13,** formaldehyde, and sodium cyanoborohydride react to yield compound **14;**
      (c) In a solvent, compound **14,** sodium formaldehyde sulfoxylate, and compound **3** react to
      yield compound **15.**

The present invention also provides the use of a compound mentioned above, or a stereoisomer thereof, or a pharmaceutically acceptable salt thereof, or a solvate thereof, or a crystal form thereof, or a prodrug thereof, or a metabolite thereof, or a deuterated derivative thereof in the manufacture of a medicament having an analgesic effect.

The present invention also provides a medicament, which is a preparation formed by a compound mentioned above, or a stereoisomer thereof, or a pharmaceutically acceptable salt thereof, or a solvate thereof, or a crystal form thereof, or a prodrug thereof, or a metabolite thereof, or a deuterated derivative thereof, in combination with pharmaceutically acceptable excipients.

The present invention also provides a pharmaceutical composition, which comprises a compound mentioned above, or a stereoisomer thereof, or a pharmaceutically acceptable salt thereof, or a solvate thereof, or a crystal form thereof, or a prodrug thereof, or a metabolite thereof, or a deuterated derivative thereof.

The compounds and derivatives provided in the present invention can be named according to the IUPAC (International Union of Pure and Applied Chemistry) or CAS (Chemical Abstracts Service, Columbus, OH) naming systems.

For the definition of terms used in the present invention: unless defined otherwise, the initial definition provided for the group or term herein applies to the group or term of the whole specification; for the terms that are not specifically defined herein, they should have the same meaning as commonly understood by one of ordinary skill in the art to which the disclosure belongs.

"Substitution" refers to the substitution of hydrogen atoms in a molecule with other different atoms or molecules.

The minimum and the maximum for the content of carbon atoms in hydrocarbon groups are represented by prefixes, such as the prefix Cₐ-C_{b} alkyl indicates any alkyl having "a"-"b" carbon atoms. Therefore, for example, "C₁-C₆ alkyl" refers to alkyls comprising 1-6 carbon atoms; "C₁-C₆ alkoxy" refers to alkoxys comprising 1-6 carbon atoms.

"Alkyl" refers to a saturated hydrocarbon chain with a specified number of carbon atoms. For example, C₁-C₆ alkyl refers to alkyl groups with 1-6 carbon atoms, namely 1, 2, 3, 4, 5, or 6 carbon atoms. Alkyl groups can be straight-chain or branched. Representative branched alkyl groups have one, two, or three branches. Alkyl includes methyl, ethyl, propyl (n-propyl and isopropyl), butyl (n-butyl, isobutyl, and tert-butyl), pentyl (n-pentyl, isopentyl, and neopentyl), hexyl, and so on.

"Halogen" refers to fluorine, chlorine, bromine, or iodine.

"Cycloalkyl" refers to a saturated or unsaturated all-carbon monocyclic or polycyclic ring (including fused, spiro, or bridged rings) without a conjugated π-electron system, including but not limited to: 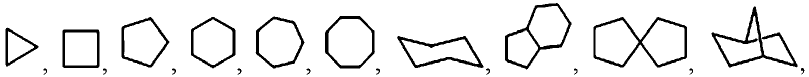 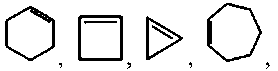 and the same.

"Heterocycloalkyl" refers to a cycloalkyl in which at least one carbon atom in the ring is replaced by a heteroatom, such as O, N, or S, and is a saturated or unsaturated monocyclic or polycyclic ring system (including fused, spiro, or bridged rings) without a conjugated π-electron system. Examples include but are not limited to: 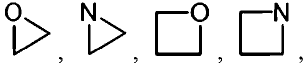 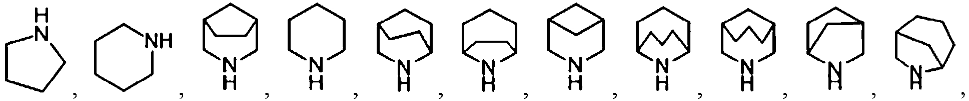 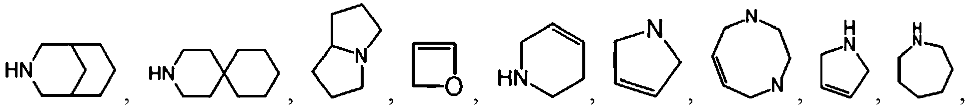 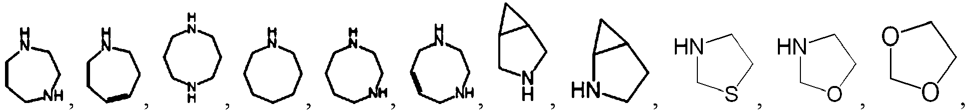 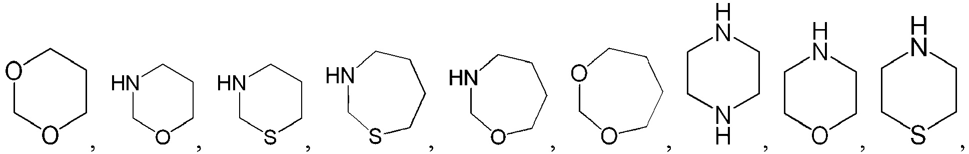 and the same.

"Aryl" refers to an all-carbon monocyclic or polycyclic ring (including fused, spiro, or bridged rings) with a conjugated π-electron system, such as but not limited to: phenyl, naphthyl, phenanthryl, anthryl, fluorenyl, and indenyl. The aromatic ring can be fused with other cyclic groups (including saturated and unsaturated rings), but it must not contain heteroatoms such as O, N, or S. Meanwhile, the point of attachment to the parent structure must be on a carbon atom in the ring with a conjugated π-electron system, such as but not limited to 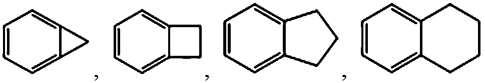 and the same.

"Heteroaryl" refers to an aryl in which at least one carbon atom in the conjugated π-electron system is replaced by a heteroatom, such as O, N, or S. Examples include but are not limited to thiophenyl, furanyl, and isothiazolyl.

The pharmaceutically acceptable salts in the present invention include acetate, adipate, aspartate, benzoate, benzenesulfonate, bicarbonate, carbonate, bisulfate, sulfate, borate, camphorsulfonate, citrate, cyclohexanesulfamate, ethanedisulfonate, ethanesulfonate, formate, fumarate, glucoheptonate, gluconate, glucuronate, hexafluorophosphate, hydrochloride, hydrobromide, hydroiodide, hydroxyethylsulfonate, lactate, malate, maleate, malonate, methanesulfonate, methylsulfate, naphthoate, naphthalenesulfonate, nicotinate, nitrate, orotate, oxalate, palmitate, pamoate hydrate, phosphate, hydrophosphate, dihydrogen phosphate, pyroglutamate, saccharate, stearate, succinate, tannate, tartrate, toluenesulfonate, trifluoroacetate, xinafoate, methanesulfonate, p-toluenesulfonate, quaternary ammonium salt, or succinate and the same.

The pharmaceutical composition of the present invention comprises a compound of the present invention or a pharmacologically acceptable salt thereof within a safe and effective dosage range, and a pharmacologically acceptable excipient or carrier.

The route of administration for the compounds or pharmaceutical compositions of the present invention include (but are not limited to): intragastric, enteral, parenteral (intravenous, intramuscular, or subcutaneous), oral, and various local administrations.

Compositions for parenteral (intravenous, intramuscular, subcutaneous) injection may comprise physiologically acceptable sterile aqueous or anhydrous solutions, dispersions, suspensions, or lotions, and sterile powders for reconstitution into sterile injectable solutions or dispersions. Suitable aqueous and non-aqueous carriers, diluents, solvents, or excipients include water, ethanol, polyols, and suitable mixtures thereof.

Solid dosage forms for oral administration include capsules, tablets, pills, powders, and granules. In these solid dosage forms, the active compound is mixed with at least one conventional inert excipient (or carrier), such as sodium citrate or dicalcium phosphate, or with the following components: (a) fillers or bulking agents, such as starch, lactose, sucrose, glucose, mannitol, and silicic acid; (b) binders, such as hydroxymethylcellulose, alginates, gelatin, polyvinylpyrrolidone, sucrose, and gum arabic; (c) Humectants, such as glycerin; (d) Disintegrants, such as agar, calcium carbonate, potato starch or cassava starch, alginic acid, certain composite silicates, and sodium carbonate; (e) Delayed-release agents, such as paraffin wax; (f) Absorption enhancers, such as quaternary ammonium compounds; (g) Wetting agents, such as cetyl alcohol and glyceryl monostearate; (h) Adsorbents, such as kaolin; and (i) Lubricants, such as talc, calcium stearate, magnesium stearate, solid polyethylene glycol, sodium lauryl sulfate, or mixtures thereof. In capsules, tablets, and pills, the dosage form may also include buffers.

Liquid dosage forms for oral administration include pharmaceutically acceptable lotions, solutions, suspensions, syrups, or tinctures. In addition to the active compound, the liquid dosage forms may contain inert diluents commonly used in the art, such as water or other solvents, solubilizers, and emulsifiers, for example, ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil, and sesame oil, or mixtures thereof.

Solid dosage forms such as tablets, sugar pills, capsules, pills, and granules can be prepared using coating and shell materials, such as enteric coatings and other materials well-known in the art. They can contain opaque agents, and the release of the active compound or compounds in such compositions can be delayed in a specific part of the digestive tract. Examples of embedding components that can be used are polymeric substances and wax-like substances. If necessary, the active compound can also be microencapsulated with one or more of the above excipients.

The dosage forms of the compounds of the present invention for local administration include ointments, powders, patches, sprays, and inhalants. Under sterile conditions, the active ingredient is mixed with a physiologically acceptable carrier, along with any preservatives, buffers, or propellants that may be necessary.

In addition to these inert diluents, the composition may also include auxiliary agents such as wetting agents, emulsifiers and suspending agents, sweeteners, flavoring agents, and fragrances.

In addition to the active compound, the suspension may comprise a suspending agent, such as ethoxylated isostearyl alcohol, polyoxyethylene sorbitol and sorbitan esters, microcrystalline cellulose, aluminum methoxide, and agar, or mixtures thereof.

The compound of the present invention can be administered alone or in combination with other pharmaceutically acceptable compounds.

When using the pharmaceutical composition, a safe and effective amount of the compound of the present invention is administered to a mammal (such as a human) in need of treatment, wherein the dosage at the time of administration is a dose considered safe and effective in pharmacology.

Compared with the prior art, the compounds provided in the present invention achieve the following beneficial effects:
The present invention provides a compound with analgesic effect. The compound of the present invention exhibits excellent analgesic effect, good safety during use, low toxicity and side effects, and no dependency during use. Therefore, the compound of the present invention has broad application prospects in the manufacture of analgesic medicaments, providing a new option for the preparation of medicaments with analgesic effects in clinical practice.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Examples

The raw materials and equipment used in the examples of the present invention are all known products, obtained by purchasing those commercially available.

The structure of the compound is determined by nuclear magnetic resonance (NMR) and/or mass spectrometry (MS). NMR shifts (δ) are given in units of 10⁻⁶ (ppm). NMR measurements were conducted using a spectrometer (Bruker Avance III 400 NMR), with the solvents being deuterated dimethyl sulfoxide (d₆-DMSO), deuterated chloroform (CDCl₃), deuterated methanol (d₄-MeOH), deuterated water (D₂O), and tetramethylsilane (TMS) as the internal standard.

The LCMS measurement was carried out using an Agilent LCMS 1200-6120 (ESI). The chromatographic column used was a Waters Xbridge Prep C18 OBD column with a size of 10 µm, 19*250 mm. The column temperature was maintained at 40°C, and the flow rate was set at 2.0 mL/min. The mobile phase consisted of a gradient from 95% [water + 10 mM ammonium bicarbonate] and 5% [CH₃CN] to 5% [water + 10 mM ammonium bicarbonate] and 95% [CH₃CN] over a period of 1.6 minutes, which was maintained for 1.4 minutes. Subsequently, the gradient was shifted to 95% [water + 10 mM ammonium bicarbonate] and 5% [CH₃CN] over a period of 0.05 minutes, and this condition was maintained for an additional 0.7 minutes.

### (1) Medicinal materials and reagents

The manufacturer of silica gel plates used in thin layer chromatography (TLC) is Qingdao Puke Separation Materials Co., Ltd., with specifications of 50*200 mm and a thickness ranging from 0.2 mm to 0.25 mm.

The silica gel used for column chromatography is 200-300 mesh silica gel from Taiyang Desiccant Co., Ltd. in Rushan City, Weihai, Shandong Province.

### (2) Main instruments

Electronic balance, FA2004, Shanghai Liangping Instrumentation Co., Ltd;
Magnetic stirrer (temperature-controlled and pressure-regulated), TY98-1, Shanghai Sile Instrument Co., Ltd;
Thiple-purpose UV analyzer, ZF-2 model, Shanghai Anting Scientific Instrument Factory;
Rotary evaporator, R201, Zhengzhou Huicheng Electronic Technology Co., Ltd;
Adjustable water bath, R201D, Zhengzhou Huicheng Electronic Technology Co., Ltd;
Circulating water vacuum pump (benchtop), SHB-III, Zhengzhou Huicheng Electronic Technology Co., Ltd.;
Circulating water vacuum pump (mobile), SHB-B95, Zhengzhou Huicheng Electronic Technology Co., Ltd.;
Low-temperature circulating pump, DLSB-5/20, Zhengzhou Huicheng Electronic Technology Co., Ltd;
Rotary vane vacuum pump (oil pump), 2XZ-4, Shanghai Vacuum Pump Factory.

### Example 1. Preparation of compound 1-1-249 of the present invention

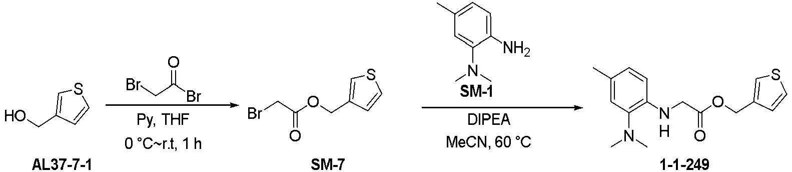

### 1. Synthesis of compound SM-7

To a 100 mL one-neck flask, was added **AL37-7-1** (1.2 g, 10.62 mmol), followed by adding tetrahydrofuran (THF) (40 mL) to dissolve, to which was then added pyridine (1.68 g, 21.24 mmol). The system was cooled to 0 °C with ice, and then bromoacetyl bromide (2.4 g, 11.68 mmol) was added dropwise. Subsequently, the mixture was stirred at room temperature for 1 h. After the reaction was complete by TLC monitoring, the solid was filterred out, and then the solvent was removed by evaporation under reduced pressure, to obtain the crude product, which was purified over silica gel column (petroleum ether: ethyl acetate = 100/1-10/1) to obtain **SM-7** (1.5 g, yield 61%) as a colorless oil.

### 2. Synthesis of compound 1-1-249

**SM-1** (400 mg, 1.71 mmol) and N,N-diisopropylethylamine (DIPEA) (441 mg, 3.42 mmol) were dissolved in acetonitrile (10 mL), to which was then added **SM-7** (257 mg, 1.71 mmol). The reaction was stirred for 6 h under reflux. After completion of the reaction according to LCMS monitoring, saturated saline (20 mL) was added to the reaction system. The resultant solution was extracted with dichloromethane (3 × 20 mL). The organic phase was washed with saturated saline (20 mL), dried over anhydrous sodium sulfate, filtered under suction, and concentrated under reduced pressure. The crude product was purified via prep-HPLC to obtain compound **1-1-249** (105.64 mg, yield 20%). MS Calcd.: 304.1; MS Found: 305.4 [M +H]⁺.

¹H NMR (400 MHz, DMSO-*d₆*) *δ*: 7.54 (q, *J* = 2.8 Hz, 1H), 7.48 (d, *J* = 2.0 Hz, 1H), 7.06 (dd, *J* = 1.2, 4.8 Hz, 1H), 6.81 (d, *J =* 1.6 Hz, 1H), 6.67 (d, *J* = 8.4 Hz, 1H), 6.32 (d, *J*= 8.0 Hz, 1H), 5.24 (t, *J* = 1.6 Hz, 1H), 5.13 (s, 2H), 3.97 (d, *J* = 6.4 Hz, 2H), 2.56 (s, 6H), 2.17 (s, 3H).

### Example 2. Preparation of compound 1-1-258 of the present invention

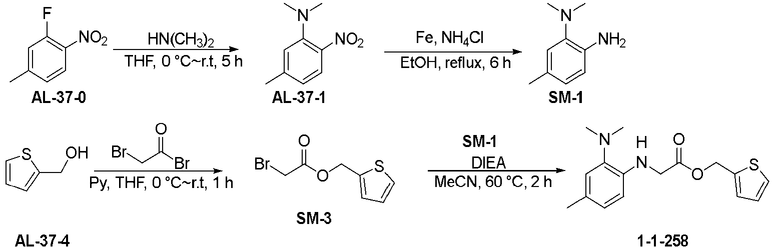

### 1. Synthesis of AL-37-1

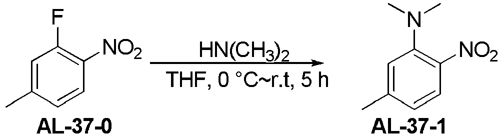

3-fluoro-4-nitrotoluene (10.0 g, 64.52 mmol) was dissolved in tetrahydrofuran (50 mL), to which was added dimethylamine (150 mL, 2 mol/L in THF, 60 mmol) at 0 °C, and then the mixture was allowed to react at room temperature for 5 h. After completion of the reaction by TLC monitoring, saturated saline (100 mL) was added to the reaction system. The resultant solution was extracted with ethyl acetate (3 × 100 mL). The organic phase was washed with saturated saline (100 mL), dried over anhydrous sodium sulfate, filtered under suction, and concentrated under reduced pressure. The crude product was purified by silica gel column chromatography to obtain compound **AL-37-1** (11.0 g, yield 95%). MS Calcd.: 180.1; MS Found: 181.4 [M +H]⁺.

### 2. Synthesis of SM-1

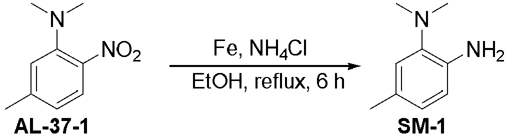

**AL-37-1** (10.0 g, 55.56 mmol), ammonium chloride (12.0 g, 222.22 mmol), and iron powder (6.2 g, 111.12 mmol) were dissolved in ethanol (280 mL), and then stirred under reflux for 6 h. After the reaction was complete via TLC monitoring, the reaction solution was filtered. The filtrate was concentrated under reduced pressure. The crude product was purified via silica gel column chromatography to obtain compound **SM-1** (6.5 g, yield 78.3%). MS Calcd.: 150.1; MS Found: 151.4 [M +H]⁺.

### 3. Synthesis of SM-3

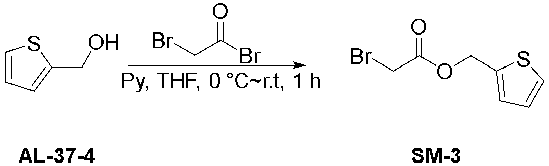

To a 250 mL one-neck flask, was added **AL-37-4** (5.0 g, 44.25 mmol), followed by adding THF (150 mL) to dissolve, to which was then added pyridine (7.0 g, 88.5 mmol). The system was cooled to 0 °C with ice, and then bromoacetyl bromide (10.73 g, 53.10 mmol) was added dropwise. Subsequently, the mixture was stirred and reacted at room temperature for 1 h. After completion of the reaction, the solid was filterred out, and then the solvent was removed by evaporation under reduced pressure, to obtain the crude product, which was purified over silica gel column (petroleum ether: ethyl acetate = 100/1-10/1) to obtain **SM-3** (8.0 g, yield 80%) as a colorless oil.

### 4. Synthesis of 1-1-258

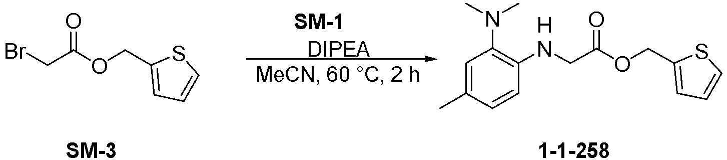

**SM-1** (400 mg, 2.67 mmol) and N,N-diisopropylethylamine (DIPEA) (688 mg, 5.33 mmol) were dissolved in MeCN (10 mL), to which was then added **SM-3** (622 mg, 2.67 mmol). The reaction was stirred under reflux for 6 h. After the reaction was complete via LCMS monitoring, saturated saline (20 mL) was added to the reaction system. The resultant solution was extracted with dichloromethane (3 × 20 mL). The organic phase was washed with saturated saline (20 mL), dried over anhydrous sodium sulfate, filtered under suction, and concentrated under reduced pressure. The crude product was purified via prep-HPLC, to obtain compound **1-1-258** (337.95 mg, yield 42%). MS Calcd.: 304.1; MS Found: 305.4 [M +H]⁺.

¹H NMR (400 MHz, DMSO-*d₆*) *δ*: 7.56 (dd, *J* = 1.2, 5.2 Hz, 1H), 7.17-7.16 (m, 1H), 7.02 (q, *J* = 4.4 Hz, 1H), 6.80 (d, *J* = 1.6 Hz, 1H), 6.66 (q, *J* = 0.4 Hz, 1H), 6.31 (d, *J* = 8.0 Hz, 1H), 5.31 (s, 2H), 5.24 (t, *J* =5.6Hz, 1H), 3.95 (d, *J* = 5.6 Hz, 2H), 2.56 (s, 6H), 2.16 (s, 3H).

### Example 3. Preparation of compound 1-1-259 of the present invention

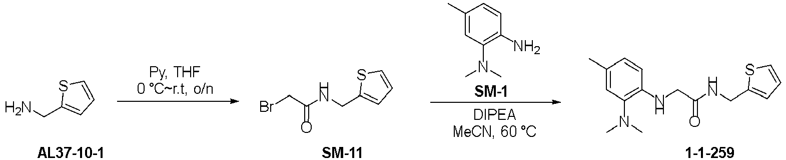

### 1. Preparation of compound SM-11

**AL37-10-1** (339 mg, 3 mmol), bromoacetyl bromide (606 mg, 3 mmol), and pyridine (237 mg, 3 mmol) were dissolved in tetrahydrofuran (50 mL) and stirred at room temperature overnight. After the reaction was complete via TLC monitoring, saturated saline (50 mL) was added to the reaction system. The reaction solution was extracted (3 × 100 mL). The organic phase was washed with saturated saline (50 mL), dried over anhydrous sodium sulfate, filtered under suction, and concentrated under reduced pressure. The crude product was purified by silica gel column chromatography (PE/EA=5/1), to obtain compound **SM-11** as a light yellow oil (500 mg, yield 71%).

### 2. Preparation of compound 1-1-259

**SM-11** (232 mg, 1.0 mmol), **SM-1** (150 mg, 1.0 mmol), and DIPEA (136 mg, 1.0 mmol) were dissolved in acetonitrile (15 mL) and stirred at 60 °C overnight. After the reaction was complete via TLC monitoring, saturated saline (50 mL) was added to the reaction system, and then the resultant solution was extracted with CH₂Cl₂ (3 × 100 mL). The organic phase was washed with saturated saline (50 mL), dried over anhydrous sodium sulfate, filtered under suction, and concentrated under reduced pressure. The crude product was purified via reversed-phase prep-HPLC to obtain compound **1-1-259** as a colorless oil (100 mg, yield 33%). ESI [M + H]⁺ = 289.2.

¹H NMR (400 MHz, DMSO-*d₆*) *δ* 8.44 (t, *J =* 6.4 Hz, 1H), 7.35 (dd, *J* = 3.6,2.4 Hz, 1H), 6.92 (dd, *J* = 4.8, 3.2 Hz, 2H), 6.82 (d, *J =* 8.0 Hz, 1H), 6.10 (dd, *J* = 8.0, 2.4 Hz, 1H),5.73 (t, *J* = 6.0 Hz, 1H), 4.44 (d, *J* = 6.0 Hz, 2H), 3.60 (d, *J =* 6.0 Hz, 2H), 2.53 (s, 6H), 2.09 (s, 3H).

### Example 4. Preparation of compound 1-1-276 of the present invention

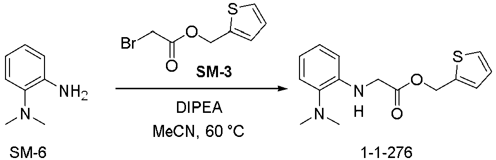

### 1. Synthesis of compound 1-1-276

**SM-6** (600 mg, 4.0 mmol) and N,N-diisopropylethylamine (DIPEA) (1.03 g, 8.0 mmol) were dissolved in MeCN (10 mL), to which was then added **SM-3** (932 mg, 4.0 mmol). The reaction was stirred under reflux at 60 °C for 6 h. After the reaction was complete via LCMS monitoring, saturated saline (20 mL) was added to the reaction system. The resultant solution was extracted with dichloromethane (3 × 20 mL). The organic phase was washed with saturated saline (20 mL), dried over anhydrous sodium sulfate, filtered under suction, and concentrated under reduced pressure. The crude product was purified via prep-HPLC, to obtain compound **1-1-276** (420.02 mg, yield 36%). MS Calcd.: 290.1; MS Found: 291.4 [M +H]⁺.

¹H NMR (400 MHz, DMSO-*d*₆) *δ*: 7.56 (d, *J* = 4.8 Hz, 1H), 7.16 (d, *J* = 3.2 Hz, 1H), 7.03-6.96 (m, 2H), 6.85 (t, *J* = 7.6 Hz, 1H), 6.60 (t, *J =* 7.6 Hz, 1H), 6.40 (d, *J* = 8.0 Hz, 1H), 5.43 (t, *J* = 6.0 Hz, 1H), 5.32 (s, 2H), 4.00 (d, *J* = 6.0 Hz, 2H), 2.57 (s, 6H).

### Example 5. Preparation of compound 1-1-321 of the present invention

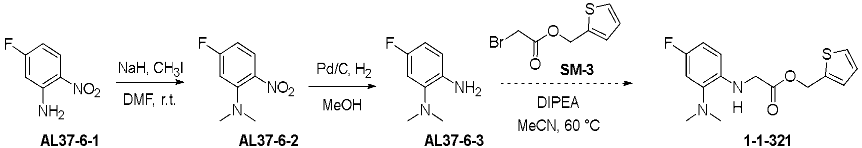

### 1. Preparation of compound AL37-6-2

**AL37-6-1** (468 mg, 3 mmol) was dissolved in N,N-dimethylformamide (DMF) (15 mL), to which was added 60% sodium hydride (240 mg, 6 mmol), and then the reaction was stirred at room temperature for 2 h, followed by addition of iodomethane (1.06 g, 7.5 mmol). After completion of the reaction via TLC monitoring, saturated saline (50 mL) was added to the reaction system, and the resultant solution was extracted with ethyl acetate (3 × 100 mL). The organic phase was washed with saturated saline (50 mL), dried over anhydrous sodium sulfate, filtered under suction, and concentrated under reduced pressure. The crude product was purified by silica gel column chromatography (PE/EA=5/1) to obtain compound **AL37-6-2** as a light yellow oil (400 mg, yield 72%). ESI [M + H]⁺ = 185.2.

### 2. Preparation of compound AL37-6-3

To a 30 mL methanol solution containing **AL37-6-2** (368 mg, 2 mmol), Pd/C (50 mg) was added, and the reaction solution was stirred at room temperature overnight. After completion of the reaction by TLC monitoring, the reaction solution was filtered and concentrated under reduced pressure to obtain the crude product, which was purified by silica gel column chromatography (PE/EA=5/1) to yield compound **AL37-6-3** as a light yellow oil (155 mg, yield 50%). ESI [M + H]⁺ =155.2.

### 3. Preparation of compound 1-1-321

**AL37-6-3** (154 mg, 1.0 mmol), **SM-3** (233 mg, 1.0 mmol), and DIPEA (129 mg, 1.0 mmol) were dissolved in acetonitrile (15 mL) and stirred at 60 °C overnight. After the reaction was complete via TLC monitoring, saturated saline (50 mL) was added to the reaction system, and then the resultant solution was extracted with CH₂Cl₂ (3 × 100 mL). The organic phase was washed with saturated saline (50 mL), dried over anhydrous sodium sulfate, filtered under suction, and concentrated under reduced pressure. The crude product was purified via reversed-phase prep-HPLC to obtain compound **1-1-321** as a colorless oil (100 mg, yield 33%). ESI [M + H]⁺ = 309.2.

¹H NMR (400 MHz, DMSO-*d₆*) *δ* 7.57 (dd, *J* = 5.2, 1.2 Hz, 1H), 7.16 (d, *J* = 3.0 Hz, 1H), 7.01 (dd, *J* = 5.2, 2.0 Hz, 1H), 6.83 (dd, *J* = 10.4, 2.8 Hz, 1H), 6.69-6.64 (m, 1H), 6.37 (dd, *J* = 8.8, 6.4 Hz, 1H), 5.31 (d, 2H), 5.25 (t, *J* = 6.0 Hz, 1H), 3.98 (d, *J* = 5.4 Hz, 2H), 2.56 (s, 6H).

### Example 6. Preparation of compound 1-1-312 of the present invention

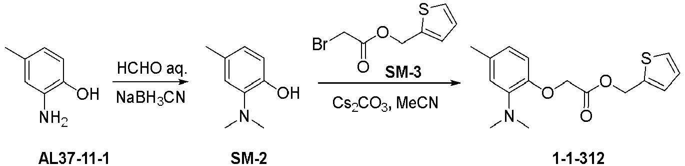

### 1. Synthesis of compound SM-2

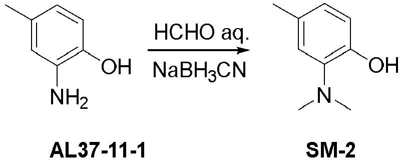

**AL37-11-1** (1.23 g, 10 mmol), aqueous formaldehyde solution (7.5 g, 100 mmol), and acetic acid (5 mL) were dissolved in 45 mL of methanol, to which was added NaBH₃CN (3.1 g, 50 mmol), and then stirred at room temperature overnight. After completion of the reaction via TLC monitoring, saturated saline (50 mL) was added to the reaction system, and the resultant solution was extracted with dichloromethane (3 × 100 mL). The organic phase was washed with saturated saline (50 mL), dried over anhydrous sodium sulfate, filtered under suction, and concentrated under reduced pressure. The crude product was purified by silica gel column chromatography (PE/EA=1/1) to obtain compound **SM-2** as a pale yellow oil (1.5 g, yield 99%). MS Found: 152.2 [M + H]⁺.

### 2. Synthesis of compound 1-1-312

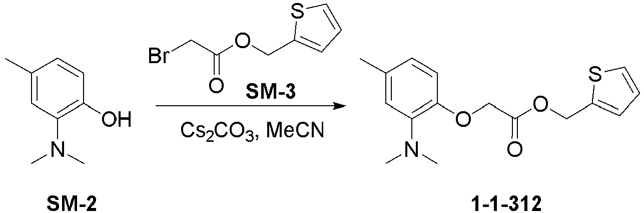

**SM-2** (302 mg, 2.0 mmol), **SM-3** (466 mg, 2.0 mmol), and cesium carbonate (750 mg, 2.0 mmol) were dissolved in acetonitrile (15 mL) and stirred at room temperature overnight. After completion of the reaction via TLC monitoring, saturated saline (50 mL) was added to the reaction system, and then the resultant solution was extracted with CH₂Cl₂ (3 × 100 mL). The organic phase was washed with saturated saline (50 mL), dried over anhydrous sodium sulfate, filtered under suction, and concentrated under reduced pressure. The crude product was purified by reversed-phase prep-HPLC to obtain compound **1-1-312** as a colorless oil (103.06 mg, yield 17%). ESI [M + H]⁺ = 306.2.

¹H NMR (400 MHz, DMSO-*d₆*) *δ* 7.58 (dd, *J =* 5.2, 1.2 Hz, 1H), 7.20-7.19 (m, 1H), 7.03 (dd, *J* = 5.2*,* 3.2 Hz, 1H), 6.67-6.66 (m, 2H), 6.63-6.60 (m, 1H), 5.37 (s, 2H), 4.75 (s, 2H), 2.69 (s, 6H), 2.20 (s, 3H).

### Example 7. Preparation of compound 1-1-322 of the present invention

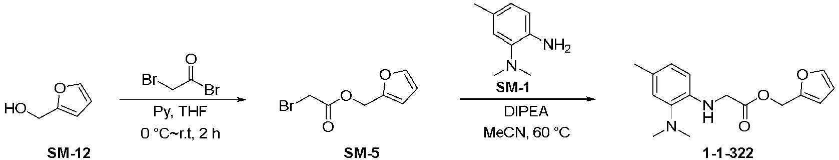

### 1. Preparation of compound SM-5

**SM-12** (980 mg, 10 mmol), bromoacetyl bromide (2.01 g, 10 mmol), and pyridine (790 mg, 10 mmol) were dissolved in tetrahydrofuran (50 mL) and stirred at room temperature overnight. After the reaction was complete via TLC monitoring, saturated saline (50 mL) was added to the reaction system. The reaction solution was extracted with dichloromethane (3 × 100 mL). The organic phase was washed with saturated saline (50 mL), dried over anhydrous sodium sulfate, filtered under suction, and concentrated under reduced pressure. The crude product was purified by silica gel column chromatography (PE/EA=5/1), to obtain compound SM-5 (1.2 g, yield 60%).

### 2. Preparation of compound 1-1-322

**SM-5** (200 mg, 1.0 mmol), **SM-1** (150 mg, 1.0 mmol), and N,N-diisopropylethylamine (DIPEA) (136 mg, 1.0 mmol) were dissolved in acetonitrile (15 mL) and stirred at 60 °C overnight. After the reaction was complete via TLC monitoring, saturated saline (50 mL) was added to the reaction system, and then the resultant solution was extracted with CH₂Cl₂ (3 × 100 mL). The organic phase was washed with saturated saline (50 mL), dried over anhydrous sodium sulfate, filtered under suction, and concentrated under reduced pressure. The crude product was purified via reversed-phase prep-HPLC to obtain compound **1-1-322** as a colorless oil (100 mg, yield 34%). ESI [M + H]⁺ = 289.2.

¹H NMR (400 MHz, DMSO-*d₆*) *δ* 7.69 (dd, *J =* 2.0, 0.8 Hz, 1H), 6.80 (d, *J* = 1.6 Hz, 1H), 6.66 (dd, *J* = 8.0, 1.2 Hz, 1H), 6.52 (d, *J* = 3.2 Hz, 1H), 6.46 (dd, *J* = 0.9, 3.2 Hz, 1H), 6.30 (d, *J* = 8.0 Hz, 1H), 5.22 (t, *J* = 6.4 Hz, 1H), 5.10 (s, 2H), 3.95 (d, *J* = 6.4 Hz, 2H), 2.56 (s, 6H), 2.16 (s, 3H).

### Example 8. Preparation of compound 1-2-283 of the present invention

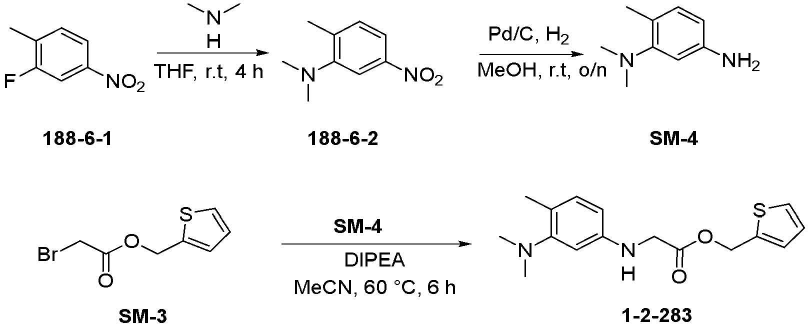

### 1. Synthesis of 188-6-2

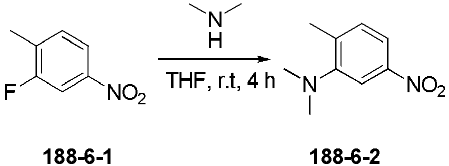

**188-6-1** (1.0 g, 6.45 mmol) was dissolved in dimethylamine (30 mL, 2 mol/L in tetrahydrofuran (THF), 60 mmol) in a sealed tube, and the mixture was allowed to react at room temperature for 4 h. After completion of the reaction via TLC monitoring, saturated saline (30 mL) was added to the reaction system, and then the resultant solution was extracted with ethyl acetate (3 × 30 mL). The organic phase was washed with saturated saline (30 mL), dried over anhydrous sodium sulfate, filtered under suction, and concentrated under reduced pressure. The crude product was purified via reversed-phase prep-HPLC to obtain compound **188-6-2** (1.1 g, yield 95%). MS Calcd.: 180.1; MS Found: 181.3 [M +H]⁺.

### 2. Synthesis of SM-4

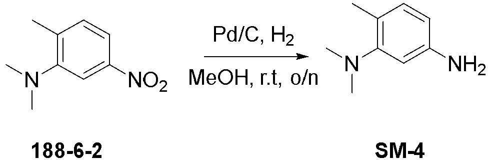

**188-6-2** (1.1 g, 6.11 mmol) was dissolved in MeOH (100 mL), to which was added Pd/C (200 mg), and then the system was purged with hydrogen three times. The system was stirred and reacted under hydrogen atmosphere overnight. After completion of the reaction by LCMS monitoring, the reaction solution was filtered. The filtrate was concentrated under reduced pressure. The crude product was purified by silica gel column chromatography to obtain compound **SM-4** (700 mg, yield 76%). MS Calcd.: 150.1; MS Found: 151.4 [M +H]⁺.

### 3. Synthesis of 1-2-283

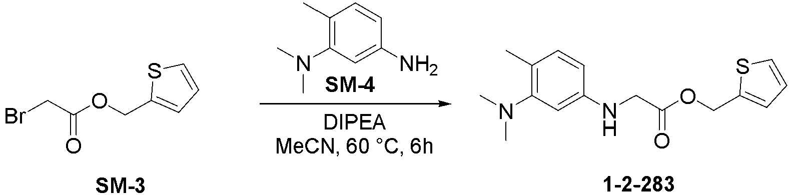

**SM-4** (400 mg, 1.71 mmol) and N,N-diisopropylethylamine (DIPEA) (441 mg, 3.42 mmol) were dissolved in MeCN (10 mL), to which was then added **SM-3** (257 mg, 1.71 mmol). The reaction was stirred at 60 °C for 6 h. After the reaction was complete via LCMS monitoring, saturated saline (20 mL) was added to the reaction system. The resultant solution was extracted with dichloromethane (3 × 20 mL). The organic phase was washed with saturated saline (20 mL), dried over anhydrous sodium sulfate, filtered under suction, and concentrated under reduced pressure. The crude product was purified via prep-HPLC, to obtain compound **1-2-283** (107.37 mg, yield 21%). MS Calcd.: 304.1; MS Found: 305.4 [M +H]⁺.

¹H NMR (400 MHz, DMSO-*d₆*) *δ*: 7.56 (dd, *J* = 1.2, 4.8 Hz, 1H), 7.16(d, *J* = 0.8 Hz, 1H), 7.02 (dd, *J* = 3.2, 5.2 Hz, 1H), 6.80 (d, *J* = 8.0 Hz, 1H), 6.22 (d, *J =* 2.4 Hz, 1H), 6.08 (dd, *J* = 2.4, 8.0 Hz, 1H), 5.80 (t, *J* = 6.4 Hz, 1H), 5.30(s, 2H), 3.86 (d, *J* = 6.4 Hz, 2H), 2.52 (s, 6H), 2.08 (s, 3H).

### Example 9. Preparation of compound 1-7-001 of the present invention

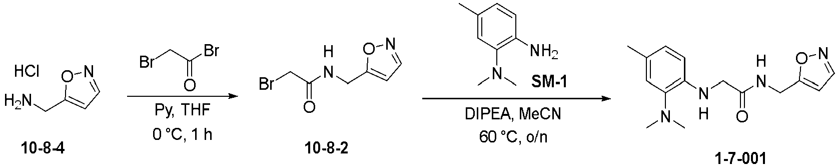

### 1. Synthesis of compound 10-8-2

To a 100 mL one-neck flask, was added **10-8-4** (200 mg, 1.48 mmol), followed by adding tetrahydrofuran (THF) (8 mL) to dissolve, to which was then added pyridine (351 mg, 4.44 mmol). The system was cooled to 0 °C with ice, and then bromoacetyl bromide (299 mg, 1.48 mmol) was added dropwise. Subsequently, the mixture was stirred at room temperature for 1 h. After the reaction was complete by TLC monitoring, the solid was filterred out, and then the solvent was removed by evaporation under reduced pressure, to obtain the crude product, which was purified over silica gel column (petroleum ether: ethyl acetate = 100/1-10/1) to obtain **10-8-2** (180 mg, yield 55%) as a colorless oil. ESI [M + H]⁺ = 219.4 [M+H]⁺.

### 2. Synthesis of compound 1-7-001

**SM-1** (124 mg, 0.826 mmol) and DIPEA(213 mg, 1.65 mmol) were dissolved in acetonitrile (5 mL), to which was then added **10-8-2** (180 mg, 0.826 mmol). The reaction was stirred at 60 °C overnight. After completion of the reaction according to LCMS monitoring, saturated saline (20 mL) was added to the reaction system. The resultant solution was extracted with dichloromethane (3 × 20 mL). The organic phase was washed with saturated saline (10 mL), dried over anhydrous sodium sulfate, filtered under suction, and concentrated under reduced pressure. The crude product was purified via prep-HPLC to obtain compound **1-7-001** (41.26 mg, yield 17%). ESI [M + H]⁺ = 289.4 [M+H]⁺.

¹H NMR (400 MHz, DMSO-*d₆*) *δ*: 8.58 (t, *J* = 6.0 Hz, 1H), 8.47 (d, *J* = 1.6 Hz, 1H), 6.80 (d, *J* =1.6 Hz, 1H), 6.71 (t, *J* = 1.2 Hz, 1H), 6.29-6.25 (m, 2H), 5.31 (t, *J* = 6.0 Hz, 1H), 4.46 (d, *J* = 6.0 Hz, 2H),3.70 (d, *J* = 5.6 Hz, 2H), 2.58 (s, 6H), 2.17 (s, 3H).

### Example 10. Preparation of compound 1-1-313 of the present inventi

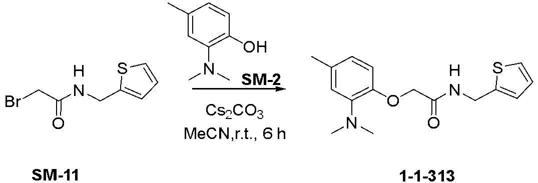

### 1. Synthesis of compound 1-1-313

**SM-11** (600 mg, 2.57 mmol) and **SM-2** (388 mg, 2.57 mmol) were added to a 100 mL one-neck flask, and then dissolved by adding MeCN (30 mL), followed by addition of cesium carbonate (837 mg, 2.57 mmol). The reaction was stirred at room temperature for 6 h. After completion of the reaction, water (20 mL) was added to the system, and the resultant solution was extracted with ethyl acetate (3 × 20 mL). The organic phase was dried over anhydrous sodium sulfate, and then filtered under suction. The solvent was removed by evaporation under reduced pressure to obtain the crude product, which was purified by silica gel column (petroleum ether: ethyl acetate = 100/1-40/1) and reversed-phase prep-HPLC, to obtain **1-1-313** as white solid (188.77 mg, yield 24%). ESI [M + H]⁺ = 305.2 [M+H] ⁺.

¹H NMR (400 MHz, DMSO-*d*₆) *δ* 9.02 (d, *J* = 5.6 Hz, 1H), 7.4 (d, *J* = 3.2 Hz, 1H), 6.95 (d, *J* = 3.6 Hz, 2H), 6.87 (d, *J* = 8 Hz, 1H), 6.74-6.70 (m, 2H), 4.52 (s, 2H), 4.49 (d, *J* = 5.6 Hz, 2H), 2.62 (s, 6H), 2.21 (s, 3H).

### Example 11. Preparation of compound 1-7-002 of the present invention

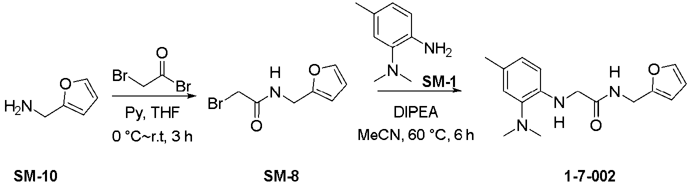

### 1. Synthesis of compound SM-8

In an ice-water bath at 0 °C, 2-furylmethylamine **SM-10** (500 mg, 5.15 mmol) and pyridine (1.22 g, 15.45 mmol) were dispersed in dry tetrahydrofuran (20 mL). Bromoacetyl bromide (1.040 g, 5.15 mmol) was gradually added, and the reaction was stirred at room temperature for 3 h. After completion of the reaction by LCMS monitoring, n-hexane (20 mL) was added to the reaction system. The reaction solution was stirred for 10 min, filtered with suction, and the solid was washed with ethyl acetate (3 × 30 mL). After drying, a white-like solid compound **SM-8** (400 mg, yield 36%) was obtained. ESI [M]⁺ = 218.0.

### 2. Synthesis of compound 1-7-002

**SM-8** (400 mg, 1.84 mmol) and **SM-1** (276 mg, 1.84 mmol) were added to a 100 mL one-neck flask, and then dissolved with MeCN (30 mL), followed by addition of N,N-diisopropylethylamine (DIPEA) (814 mg, 5.52 mmol). The system was heated to 60 °C in an oil bath and reacted under stirring for 6 h. After completion of the reaction, water (30 mL) was added to the system, and then the resultant solution was extracted with ethyl acetate (3 × 30 mL). The organic phase was dried over anhydrous sodium sulfate, and then filtered under suction. The solvent was removed by evaporation under reduced pressure to obtain a crude product, which was purified by silica gel column (petroleum ether: ethyl acetate = 100/1-30/1) to obtain **1-7-002** (163.06 mg, yield 31%) as white solid. ESI [M + H]⁺ = 288.2 [M+H]⁺.

¹H NMR (400 MHz, DMSO-d₆), δ 8.38 (t, *J =* 5.6 Hz, 1H), 7.56 (t, *J =* 0.8 Hz, 1H), 6.79 (d, *J* = 2 Hz, 1H), 6.69(d, *J* = 8.0 Hz,1H), 6.37 (q, *J*₁ = 1.6 Hz, *J*₂ = 2.8 Hz, 1H), 6.27 (d, *J* = 8.0 Hz, 1H), 6.20 (d, *J* = 3.2 Hz, 1H), 5.27 (t, *J* = 5.6 Hz, 1H), 4.30 (d, *J* = 5.6 Hz, 2H), 3.66 (d, *J* = 6.0 Hz, 2H), 2.57 (s, 6H), 2.16 (s, 3H).

### Example 12. Preparation of compound 1-7-003 of the present invention

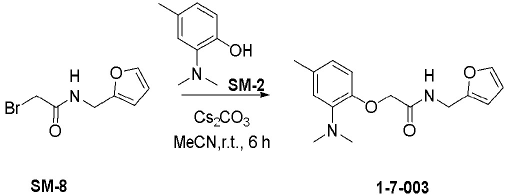

### 1. Synthesis of compound 1-7-003

**SM-8** (450 mg, 2.07 mmol) and **SM-2** (312 mg, 2.07 mmol) were added to a 100 mL one-neck flask, and then dissolved by adding MeCN (30 mL), followed by addition of cesium carbonate (672 mg, 2.07 mmol). The reaction was stirred at room temperature for 6 h. After completion of the reaction, water (20 mL) was added to the system, and the resultant solution was extracted with ethyl acetate (3 × 20 mL). The organic phase was dried over anhydrous sodium sulfate, and then filtered under suction. The solvent was removed by evaporation under reduced pressure to obtain the crude product, which was purified by silica gel column (petroleum ether: ethyl acetate = 100/1-40/1) and reversed-phase prep-HPLC, to obtain **1-7-003** as white solid (100.24 mg, yield 34%). ESI [M + H]⁺ = 289.1 [M+H]⁺.

¹H NMR (400 MHz, DMSO-*d*₆) *δ* 8.93 (d, *J* = 5.6 Hz, 1H), 7.58 (q, *J*₁ = 0.8 Hz, 1H), 6.88 (d, *J* = 8 Hz, 1H), 6.76~6.71 (m, 2H), 6.39 (q, *J*₁ = 2 Hz, 1H), 6.21(dd, *J*₁ = 0.8 Hz, *J*₂ = 3.2 Hz, 1H), 4.52 (s , 2H), 4.32 (d, *J* = 5.6 Hz, 2H), 2.64 (s, 6H), 2.22 (s, 3H).

### Example 13. Preparation of compound 1-2-301 of the present invention

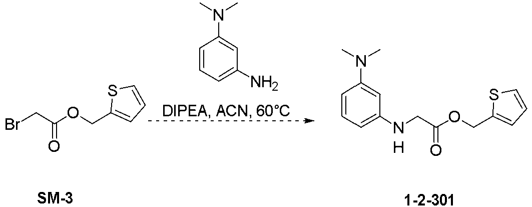

### 1. Synthesis of compound 1-2-301

N,N-dimethyl-m-phenylenediamine (300 mg, 2.20 mmol) was added to a reaction flask, and then dissolved with acetonitrile (10 mL), followed by addition of N,N-diisopropylethylamine (DIPEA) (569 mg, 4.41 mmol) and **SM-3** (518 mg, 2.20 mmol). The reaction system was purged with nitrogen three times, heated to 60 °C, and then stirred for 6 h. After completion of the reaction, the reaction solution was cooled to room temperature. The solvent was removed by rotatory evaporation at 40 °C. Water (15 mL) and dichloromethane (15 mL) were added to perform extraction three times. The organic phases were combined, washed with saturated saline, dried over anhydrous sodium sulfate, and filtered. The solvent was removed by evaporation under reduced pressure. The residue was purified by prep-HPLC to obtain **1-2-301** as white solid (127 mg, yield: 20%). ESI [M+H]⁺ = 291.1.

¹H NMR (400 MHz, DMSO-*d*₆) *δ* 7.56 (dd, *J =* 4.8, 1.2 Hz, 1H), 7.16 (d, *J* = 3.6 Hz, 1H), 1.02 (dd, *J* = 1.8, 3.2Hz, 1H), 6.85 (t, *J* = 8.4 1H), 6.00 (m, 1H), 5.88 (m, 2H), 5.81 (t, *J* = 6.4 Hz, 1H) ,5.31 (s, 2H), 3.87 (d, *J* = 6.4 Hz, 2H), 2.78 (s, 6H).

### Example 14. Preparation of compound 1-7-004 of the present invention

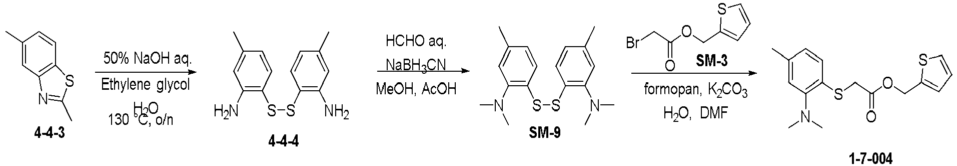

### 1. Synthesis of compound 4-4-4

To a solution of **4-4-3** (1.63 g, 10 mmol) in 20 mL of ethylene glycol, 50% aqueous NaOH solution (20 mL) was added, and the reaction solution was stirred at 130 °C overnight. After completion of the reaction by TLC monitoring, water (100 mL) was added to the reaction system, and the resultant solution was extracted with ethyl acetate (3 × 100 mL). The organic phase was washed with saturated saline (50 mL), dried over anhydrous sodium sulfate, and filtered under suction. The crude product obtained after concentration under reduced pressure was purified by silica gel column chromatography (PE/EA=5/1) to obtain compound **4-4-4** as yellow solid (1.1 g, yield 80%). ESI [M + H]⁺ = 277.2.

### 2. Synthesis of compound SM-9

**4-4-4** (1.1 g, 4.0 mmol), formaldehyde aqueous solution (3.0 g, 40.0 mmol), and acetic acid (5 mL) were dissolved in 45 mL of methanol, to which was added NaBH₃CN (1.3 g, 20 mmol), and then the reaction solution was stirred at room temperature overnight. After completion of the reaction via TLC monitoring, saturated saline (50 mL) was added to the reaction system, and then the resultant solution was extracted with dichloromethane (3 × 100 mL). The organic phase was washed with saturated saline (50 mL), dried over anhydrous sodium sulfate, filtered under suction, and concentrated under reduced pressure. The crude product was purified over silica gel column chromatography (PE/EA=5/1) to obtain compound **SM-9** as a light yellow oil (900 mg, yield 68%). ESI [M + H]⁺ = 333.2.

### 3. Synthesis of compound 1-7-004

**SM-9** (332 mg, 1.0 mmol), **SM-3** (466 mg, 2.0 mmol), sodium formaldehyde sulfoxylate (1.2 g, 10 mmol), and potassium carbonate (278 mg, 2.0 mmol) were dissolved in N,N-dimethylformamide (DMF) (30 mL) and water (3 mL), and then the reaction solution was stirred at room temperature overnight. After completion of the reaction by TLC monitoring, water (50 mL) was added to the reaction system, and then the resultant solution was extracted with ethyl acetate (3 × 100 mL). The organic phase was washed with saturated saline (50 mL), dried over anhydrous sodium sulfate, filtered under suction, and concentrated under reduced pressure. The crude product was purified by reversed-phase prep-HPLC to obtain compound **1-7-004** as a colorless oil (130 mg, yield 41%). ESI [M + H]⁺ = 322.1 [M + H]⁺.

¹H NMR (400 MHz, DMSO-*d₆*) *δ* 7.56 (dd, *J*₁ = 1.2 Hz, *J* = 5.2 Hz, 1H), 7.12 (d, *J =* 3.6 Hz, 1H), 7.04~6..99 (m, 2H), 6.92 (d, *J =* 1.2 Hz, 1H), 6.78 (d, *J =* 8 Hz, 1H), 5.27 (s, 2H), 3.81 (s, 2H), 2.62 (s, 6H), 2.24 (s, 3H).

### Example 15. Preparation of compound 1-1-267 of the present invention

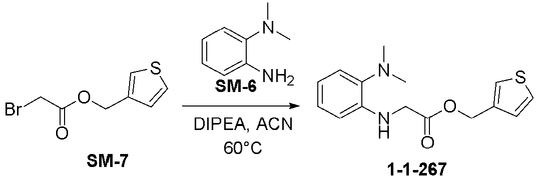

### 1. Synthesis of compound 1-1-267

*N,N*-dimethyl-o-phenylenediamine **SM-6** (440 mg, 1.87 mmol) and N,N-diisopropylethylamine (DIPEA) (484 mg, 3.74 mmol) were dissolved in acetonitrile (10 mL), to which was then added **SM-7** (255 mg, 1.87 mmol), and then the mixture was stirred under reflux for 6 h. After completion of the reaction via LCMS monitoring, saturated saline (20 mL) was added to the reaction system, and then the resultant solution was extracted with dichloromethane (3 × 20 mL). The organic phase was washed with saturated saline (20 mL), dried over anhydrous sodium sulfate, filtered under suction, and concentrated under reduced pressure. The crude product was purified over silica gel column chromatography (EtOAc/petrol ether (v/v) = 0-60%) to obtain compound **1-1-267** (105.64 mg, yield 20%). ESI [M + H]⁺ = 291.2.

¹H NMR (400 MHz, CDCl₃) *δ*: 7.31 (dd, *J* = 5.2, 3.2 Hz, 1H), 7.29-7.26 (m, 1H), 7.15-6.91 (m, 3H), 6.74 (dd, *J* = 7.6, 7.6 Hz, 1H), 6.50 (d, *J* = 7.6 Hz, 1H), 5.61-5.23 (m, 1H), 5.20 (s, 2H), 4.00 (s, 2H), 2.53 (s, 6H).

### Example 16. Preparation of compound 1-7-005 of the present invention

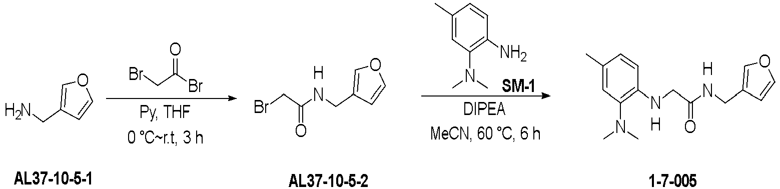

### 1. Synthesis of compound AL37-10-5-2

To a 100 mL one-neck flask, was added **AL37-10-5-1** (500 mg, 5.15 mmol), followed by adding tetrahydrofuran (THF) (30 mL) to dissolve, to which was then added pyridine (814 mg, 10.3 mmol). The system was cooled to 0 °C in an ice bath, and then bromoacetyl bromide (1040 mg, 5.15 mmol) was slowly added dropwise. Subsequently, the mixture was stirred and reacted at room temperature overnight. After the reaction was complete by LCMS monitoring, n-hexane (20 mL) was added to the reaction system. The reaction solution was stirred for 10 min, and then filtered with suction. The solid was washed with ethyl acetate (3 × 30 mL), and dried to obtain compound **AL37-10-5-2** as off-white solid (400 mg, yield 35%). ESI [M + H]⁺ = 219.0 [M+H]⁺.

### 2. Synthesis of compound 1-7-005

**AL37-10-5-2** (400 mg, 1.84 mmol) and **SM-1** (276 mg, 1.84 mmol) were added to a 100 mL one-neck flask, and then dissolved by adding MeCN (30 mL), followed by addition of N,N-diisopropylethylamine (DIPEA) (814 mg, 5.52 mmol). The system was heated to 60 °C in an oil bath, and then the reaction was stirred for 6 h. After completion of the reaction, water (30 mL) was added to the system, and the resultant solution was extracted with ethyl acetate (3 × 30 mL). The organic phase was dried over anhydrous sodium sulfate, and then filtered. The solvent was removed by evaporation under reduced pressure to obtain the crude product, which was purified by silica gel column (petroleum ether: ethyl acetate = 100/1-30/1), to obtain **1-7-005** as white solid (204.20 mg, yield 38%). ESI [M + H]⁺ = 288.0 [M+H]⁺.

¹H NMR (400 MHz, DMSO-*d*₆) *δ* 8.24 (t, *J* = 5.6 Hz, 1H), 7.57 (t, *J* = 1.6 Hz, 1H), 7.49 (s, 1H), 6.79 (d, *J* = 1.6 Hz, 1H), 6.68 (dd, *J*₁ = 1.2 Hz, *J*₂ = 8 Hz, 1H), 6.38 (s, 1H), 6.27 (d, *J* = 8 Hz, 1H), 5.27 (t, *J* = 5.6 Hz, 1H), 4.14(d, *J* = 5.6 Hz, 2H), 3.64 (d, *J =* 5.6 Hz, 2H), 2.57 (s, 6H), 2.16 (s, 3H).

### Example 17. Preparation of compound 1-7-006 of the present invention

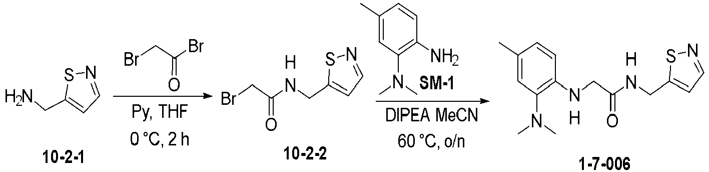

### 1. Synthesis of compound 10-2-2

To a 100 mL one-neck flask, was added **10-2-1** (400 mg, 3.51 mmol), followed by adding tetrahydrofuran (THF) (20 mL) to dissolve, to which was then added pyridine (555 mg, 7.02 mmol). The system was cooled to 0 °C in an ice bath, and then bromoacetyl bromide (709 mg, 3.51 mmol) was slowly added dropwise. Subsequently, the mixture was stirred and reacted at room temperature for 2 h. After completion of the reaction, the solid were filtered off. The solvent was removed by evaporation under reduced pressure to obtain the crude product, which was purified by silica gel column (petroleum ether: ethyl acetate = 100/1 to 10/1) to obtain **10-2-2** (340 mg, yield 41%) as a colorless oil. ESI [M + H]⁺ = 234.4 [M+H]⁺.

### 2. Synthesis of compound 1-7-006

SM-1 (218 mg, 1.45 mmol) and N,N-diisopropylethylamine (DIPEA) (561 mg, 4.35 mmol) were dissolved in acetonitrile (10 mL), to which was then added **10-2-2** (340 mg, 1.45 mmol), and then the mixture was stirred under reflux overnight. After completion of the reaction via LCMS monitoring, saturated saline (10 mL) was added to the reaction system, and then the resultant solution was extracted with dichloromethane (3 × 20 mL). The organic phase was washed with saturated saline (10 mL), dried over anhydrous sodium sulfate, filtered under suction, and concentrated under reduced pressure. Then, the crude product was purified by prep-HPLC to obtain compound **1-7-006** (228.68 mg, yield 52%). ESI [M + H]⁺ = 305.4 [M+H] ⁺. ¹H NMR (400 MHz, DMSO-*d*₆) *δ*: 8.69 (t, *J* = 7.6 Hz, 1H), 8.42 (d, *J* = 1.6Hz, 1H), 7.23 (d, *J* = 1.2 Hz, 1H), 6.80 (d, *J* = 1.6 Hz, 1H), 6.69 (d, *J=* 8.0 Hz, 1H), 6.26 (d, *J* =8.0Hz, 1H), 5.33 (t, *J* = 6.0 Hz, 1H), 4.59 (d, *J* =6.0Hz, 2H), 3.69 (d, *J* =5.6Hz, 2H), 2.59 (s, 6H), 2.17 (s, 3H).

### Example 18. Preparation of compound 1-1-324 of the present invention

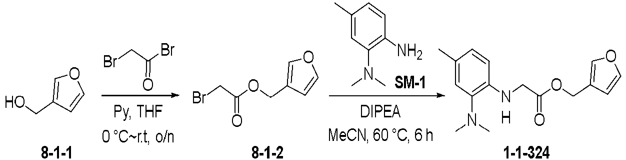

### 1. Synthesis of compound 8-1-2

To a 100 mL one-neck flask, was added **8-1-1** (500 mg, 5.10 mmol), followed by adding tetrahydrofuran (THF) (40 mL) to dissolve, to which was then added pyridine (806 mg, 10.20 mmol). The system was cooled to 0 °C in an ice bath, and then bromoacetyl bromide (1.03 g, 5.10 mmol) was added dropwise. Subsequently, the mixture was stirred and reacted at room temperature overnight. After completion of the reaction by TLC monitoring, the solid were filtered off. The solvent was removed by evaporation under reduced pressure to obtain the crude product, which was purified by silica gel column (petroleum ether: ethyl acetate = 100/1 to 10/1) to obtain **8-1-2** (600 mg, yield 55%) as a colorless oil. ESI [M + H]⁺ = 218.3 [M+H]⁺.

### 2. Synthesis of compound 1-1-324

**SM-1** (413 mg, 2.75 mmol) and DIPEA (1.06 g, 8.25 mmol) were dissolved in acetonitrile (14 mL), to which was then added **8-1-2** (600 mg, 2.75 mmol), and then the mixture was stirred under reflux for 6 h. After completion of the reaction via LCMS monitoring, saturated saline (20 mL) was added to the reaction system, and then the resultant solution was extracted with dichloromethane (3 × 20 mL). The organic phase was washed with saturated saline (20 mL), dried over anhydrous sodium sulfate, filtered under suction, and concentrated under reduced pressure. Then, the crude product was purified by silica gel column (petroleum ether: ethyl acetate = 100/1 to 5/1) and prep-HPLC to obtain compound **1-1-324** (227.92 mg, yield 29%). ESI [M + H]⁺ = 288.4 [M+H]⁺.

¹H NMR (400 MHz, DMSO-*d*₆) *δ*: 7.70 (s, 1H), 7.65 (t, *J* = 1.6 Hz, 1H), 6.80 (d, *J* = 1.6 Hz, 1H), 6.67 (dd, *J =* 1.2, 8.0 Hz, 1H), 6.46 (d, *J* = 0.8 Hz, 1H), 6.32 (d, *J* = 8.4 Hz, 1H), 5.22 (s, 1H), 5.00 (s, 2H), 3.95 (d, *J* = 2.4 Hz, 2H), 2.56 (s, 6H), 2.16 (s, 3H).

### Example 19. Preparation of compound 1-7-007 of the present invention

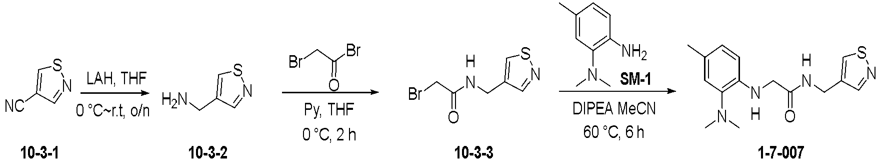

### 1. Synthesis of 10-3-2

**10-3-1** (1.0 g, 9.09 mmol) was added to a reaction flask (150 mL), and dissolved with tetrahydrofuran (THF) (45 mL). Then, the system was cooled to 0 °C, and lithium aluminum hydride (7.3 mL, 18.18 mmol) was slowly added into the reaction solution dropwise. After that, the reaction was stirred at room temperature overnight. After completion of the reaction via LCMS monitoring, 1 mL of water, 1 mL of 15% NaOH solution, and 3 mL of water were slowly added to the reaction system in sequence to quench the reaction. The resultant solution was stirred at room temperature for 20 min, filtered, and concentrated to obtain the crude product, which was purified by column chromatography (DCM:MeOH=20: 1) to obtain 10-3-2 (240 mg, yield 24%) as a yellow oil. ESI [M + H]⁺ = 115.4 [M+H]⁺.

### 2. Synthesis of 10-3-3

To a 100 mL one-neck flask, was added **10-3-2** (240 mg, 2.11 mmol), followed by adding tetrahydrofuran (THF) (20 mL) to dissolve, to which was then added pyridine (333 mg, 4.22 mmol). The system was cooled to 0 °C in an ice bath, and then bromoacetyl bromide (426 mg, 2.11 mmol) was added dropwise. Subsequently, the mixture was stirred and reacted at room temperature for 2 h. After completion of the reaction, the solid were filtered off. The solvent was removed by evaporation under reduced pressure to obtain the crude product, which was purified by silica gel column (petroleum ether: ethyl acetate = 100/1 to 10/1) to obtain **10-3-3** (200 mg, yield 41%) as a colorless oil. ESI [M + H]⁺ = 234.3 [M+H]⁺.

### 3. Synthesis of compound 1-7-007

**SM-1** (128 mg, 0.855 mmol) and N,N-diisopropylethylamine (DIPEA) (221 mg, 1.71 mmol) were dissolved in acetonitrile (10 mL), to which was then added **10-3-3** (200 mg, 0.855 mmol), and then the mixture was stirred under reflux for 6 h. After completion of the reaction via LCMS monitoring, saturated saline (10 mL) was added to the reaction system, and then the resultant solution was extracted with dichloromethane CH₂Cl₂ (3 × 20 mL). The organic phase was washed with saturated saline (10 mL), dried over anhydrous sodium sulfate, filtered under suction, and concentrated under reduced pressure. Then, the crude product was purified by prep-HPLC to obtain compound 1-7-007 (170.57 mg, yield 66%). ESI [M + H]⁺ = 305.4 [M+H] ⁺. ¹H NMR (400 MHz, DMSO-*d*₆) *δ*: 8.75 (s, 1H), 8.48 (t, *J* = 6.0 Hz, 1H), 8.45 (s, 1H), 6.80 (s, 1H), 6.70 (d, *J =* 8.0 Hz, 1H), 6.27 (d, *J =* 8.0 Hz, 1H), 5.30 (t, *J* = 5.6 Hz, 1H), 4.39 (d, *J* =6.0 Hz, 2H), 3.68 (d, *J*=5.6 Hz, 2H), 2.59 (s, 6H), 2.17 (s, 3H).

### Example 20. Preparation of compound 1-7-008 of the present invention

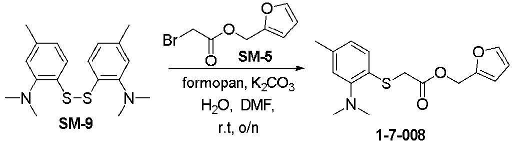

### 1. Synthesis of compound 1-7-008

**SM-9** (400 mg, 1.20 mmol), **SM-5** (528 mg, 2.41 mmol), sodium formaldehyde sulfoxylate (1.42 g, 12.0 mmol), and K₂CO₃ (333 mg, 2.41 mmol) were dissolved in N,N-dimethylformamide (DMF) (30 mL) and H₂O (3 mL), and then the reaction solution was stirred at room temperature overnight. After completion of the reaction by TLC monitoring, water (50 mL) was added to the reaction system, followed by extraction with EA (3 × 100 mL). The organic phase was washed with saturated saline (50 mL), dried over anhydrous sodium sulfate, filtered under suction, and concentrated under reduced pressure. The crude product was purified by reversed-phase prep-HPLC to obtain compound **1-7-008** as a colorless oil (230.22 mg, yield 63%). ESI [M + H]⁺ = 306.1 [M + H]⁺.

¹H NMR (400 MHz, DMSO-*d₆*) *δ* 7.56 (dd, *J* = 1.2 Hz, 2.0 Hz, 1H), 7.04 (d, *J =* 8.0 Hz, 1H), 6.92 (d, *J* = 1.2 Hz, 1H), 6.79 (dd, *J* = 1.2,7.6 Hz, 1H), 6.49 (d, *J* = 3.2 Hz,, 1H), 6.46 (dd, *J =* 1.6,3.2 Hz, 1H), 5.07 (s, 2H),3.82 (s, 2H), 2.63 (s, 6H), 2.24 (s, 3H).

### Example 21. Preparation of compound 1-7-009 of the present invention

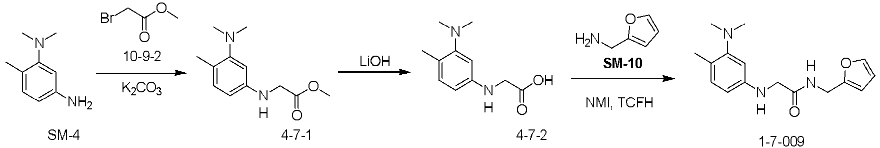

### 1. Synthesis of compound 4-7-1

To a solution of compound **SM-4** (1 g, 6.66 mmol) in N,N-dimethylformamide (10 mL), bromoacetic acid methyl ester **10-9-2** (1.16 mL, 7.20 mmol) and potassium carbonate (1.38 g, 9.99 mmol) were added, and then the reaction was stirred at 25 °C for 14. After completion of the reaction indicated by LC-MS, the reaction mixture was concentrated under reduced pressure. H2O (40 mL) was added, followed by extraction with ethyl acetate (20 mL x 3). The organic phase was washed with concentrated brine, dried over anhydrous sodium sulfate, and concentrated. The residue was purified by silica gel column chromatography (ISCO@; 12 g SepaFlash@ Silica Flash Column, eluent of 0 - 5% PE/EA gradient @ 45 mL/min) to obtain compound **4-7-1** as a brown oil (0.6 g, yield 40.55%). *m*/*z* calculated for [M+H]⁺ 223.3, found 223.1.

### 2. Synthesis of compound 4-7-2

To a solution of compound **4-7-1** (600 mg, 2.699 mmol) in tetrahydrofuran (12 mL) and water (3 mL), lithium hydroxide (193.92 mg, 8.097 mmol) was added, and the reaction was stirred at room temperature for 2 h. Afte completion of the reaction as indicated by LC-MS, the mixture was neutralized with dilute hydrochloric acid and concentrated under reduced pressure to obtain compound **4-7-2** as a brown oil (550 mg, yield 97.84%). m/z calculated for [M+H]⁺ 209.3, found 209.2.

### 3. Synthesis of compound 1-7-009

To a solution of compound **4-7-2** (600 mg, 2.881 mmol) in acetonitrile (ACN) (10 mL), N-methylimidazole (NMI) (0.5 mL), N,N,N',N'-tetramethylchloroformamidinium hexafluorophosphate (TCFH) (3226.74 mg, 11.524 mmol), and 2-furylmethylamine (SM-10) (559.61 mg, 5.762 mmol) were added, and then the mixture was stirred at room temperature for 20 min. After LC-MS indicated completion of the reaction, water (20 mL) was added to the reaction mixture, followed by extraction with ethyl acetate (20 mL x 3). The organic phase was washed with concentrated brine, dried over anhydrous sodium sulfate, and concentrated *in vacuum.* The residue was purified by pre-HPLC to obtain compound **1-7-009** as a colorless oil (244.6 mg, yield 29.55%). m/z calculated for [M+H]⁺ 288.4, found 288.1.

¹H NMR (400 MHz, DMSO-*d*₆) δ 8.30-8.27 (m, 1H), 7.55-7.54 (m, 1H), 6.84-6.82 (m, 1H), 6.37-6.36 (m, 1H), 6.28-6.27 (m, 1H), 6.17 - 6.16 (m, 1H), 6.12-6.09 (m, 1H), 5.71-5.68 (m, 1H), 4.28 (d, *J* = 4 Hz, 2H), 3.62 (d, *J* = 4 Hz, 2H), 2.55 (s, 6H), 2.09 (s, 3H).

### Example 22. Preparation of compound 1-7-010 of the present invention

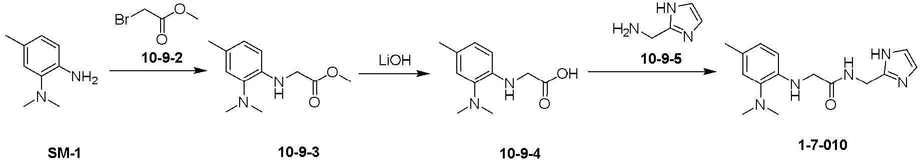

### 1. Synthesis of compound 10-9-3

At 0 °C, to a solution of compound **SM-1** (1000 mg, 6.66 mmol) in N,N-dimethylformamide (10 mL), were added compound **10-9-2** (1.17 mL, 7.32 mmol) and potassium carbonate (1839.91 mg, 13.31 mmol), followed by stirring at 60°C for 16 h. After completion of the reaction indicated by LC-MS, the reaction mixture was concentrated under reduced pressure. H₂O (200 mL) was added, and the resultant solution was extracted with ethyl acetate (200 mL x 3). The organic layer was washed with concentrated brine, dried over anhydrous sodium sulfate, and concentrated. The residue was purified by silica gel column chromatography to obtain compound **10-9-3** as a yellow oil (1200 mg, yield 81.10%). m/z calculated for [M+H] ⁺ 223.29, found 223.1.

### 2. Synthesis of compound 10-9-4

To a solution of compound **10-9-3** (1200 mg, 5.40 mmol) in tetrahydrofuran (10 mL), was added lithium hydroxide (0.65 g, 26.99 mmol), and then the reaction was stirred at 0 °C for 30 min. After LC-MS indicated completion of the reaction, the reaction mixture was concentrated under reduced pressure to obtain compound **10-9-4** as a yellow oil (800 mg, yield 80.09%). m/z calculated for [M+H]⁺ 209.26, found 209.1.

### 3. Synthesis of compound 1-7-010

To a solution of compound **10-9-4** (800 mg, 3.84 mmol) in acetonitrile (10 mL), were added N-methylimidazole (629.98 mg, 7.68 mmol), tetramethylchloroformamidinium hexafluorophosphate (1077.80 mg, 3.84 mmol), and compound **10-9-5** (559.61 mg, 5.76 mmol), and then the mixture was stirred at 25 °C for 30 min. After LC-MS indicated completion of the reaction, water was added, followed by extraction with ethyl acetate (20 mL x 3). The organic phase was washed with concentrated brine, dried over anhydrous sodium sulfate, and concentrated *in vacuum.* The residue was purified by pre-HPLC to obtain compound **1-7-010** as white solid (155.6 mg, yield 14.10%). m/z calculated for [M+H] ⁺ 288.37, found 288.1.

¹H NMR (400 MHz, DMSO-*d*₆) *δ* 8.43 - 8.31 (m, 1H), 6.99 (s, 2H), 6.83 - 6.75 (m, 1H), 6.73 - 6.60 (m, 1H), 6.43 - 6.23 (m, 1H), 5.28 (s, 1H), 4.47 - 4.25 (m, 2H), 3.89 - 3.59 (m, 2H), 2.58 (s, 6H), 2.17 (s, 3H).

### Example 23. Preparation of compound 1-7-011 of the present invention

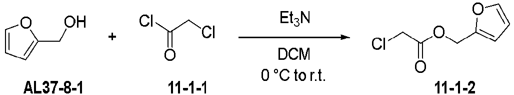

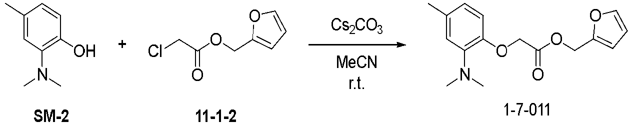

### 1. Synthesis of compound 11-1-2

**AL37-8-1** (0.86 mL, 10 mmol, 1.0 equiv.) was dissolved in dichloromethane (5 mL), to which were added **11-1-1** (0.88 mL, 11 mmol, 1.1 equiv.) and Et₃N (1.67 mL, 12 mmol, 1.2 equiv.) at 0 °C, and then the mixture was allowed to react at room temperature for 2 h. After completion of the reaction via TLC monitoring, the reaction was quenched with water, and then the resultant solution was extracted three times with dichloromethane. The organic phase was washed with concentrated brine, dried over anhydrous sodium sulfate, and concentrated under reduced pressure. Then, the residue was purified by column chromatography (PE/EtOAc = 15:1) to obtain compound **11-1-2** as a yellow oil (1.45 g, yield 83%).

### 2. Synthesis of compound 1-7-011

**SM-2** (302 mg, 2 mmol, 1.0 equiv.) and **11-1-2** (349 mg, 2 mmol, 1.0 equiv.) were dissolved in MeCN (10 mL), to which was then added Cs₂CO₃ (652 mg, 2 mmol, 1.0 equiv.). The mixture was allowed to react at room temperature overnight. After completion of the reaction via TLC monitoring, EtOAc and water were added to carry out extraction three times. The organic phase was dried over anhydrous sodium sulfate, concentrated under reduced pressure, and then subjected to column chromatography (PE/EtOAc = 15:1) to obtain compound **1-7-011** as a yellow oil: 355 mg, 1.23 mmol, with a yield of 61%. HRMS (ESI-TOF) *m*/*z:* [M + H]⁺ Calcd for C₁₆H₁₉NO₄H⁺ 290.1389; Found 290.1389.

¹H NMR (400 MHz, CDCl₃) *δ* 7.45-7.40 (m, 1H), 6.75 (s, 1H), 6.71-6.67 (m, 1H), 6.64 (d, *J* = 8.2 Hz, 1H), 6.43 (d, *J* = 3.2 Hz, 1H), 6.36 (dd, *J* = 3.2, 1.8 Hz, 1H), 5.18 (s, 2H), 4.69 (s, 2H), 2.79 (s, 6H), 2.27 (s, 3H).

### Example 24. Preparation of compound 1-2-337 of the present invention

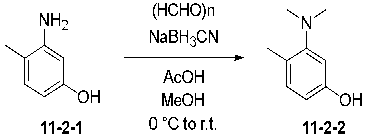

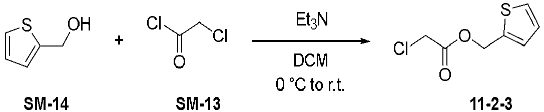

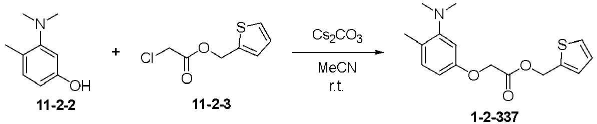

### 1. Synthesis of compound 11-2-2

To a solution of **11-2-1** (1.23 g, 10 mmol) in methanol (25 mL), were added acetic acid (3 mL, 50 mmol) and paraformaldehyde (3 g, 100 mmol), and then the mixture was cooled to 0 °C, to which was gradually added NaBH₃CN (3.15 g, 50 mmol, 5.0 equiv.) in batches. After that, the mixture was warmed to room temperature and reacted overnight. The reaction was monitored by TLC to ensure completion, and then the reaction was quenched using saturated NaHCO₃ (aq). The pH value was adjusted to approximately 7, and the reaction solution was extracted three times with ethyl acetate and water. The organic phase was dried over anhydrous sodium sulfate, concentrated under reduced pressure, and purified by column chromatography (PE/EtOAc = 10:1) to obtain compound **11-2-2** as a colorless oil (1.26 g, yield 83%).

### 2. Synthesis of compound 11-2-3

The solution of **SM-14** (0.95 mL, 10 mmol, 1.0 equiv.) in dichloromethane (DCM) (5 mL) was cooled to 0 °C, to which were added **SM-13** (0.88 mL, 11 mmol, 1.1 equiv.) and Et₃N (1.67 mL, 12 mmol, 1.2 equiv.), and then the mixture was allowed to react at room temperature for 2 h. After monitoring the reaction to completion by TLC, the reaction was quenched with water, and then extracted three times with dichloromethane. The organic phase was washed with concentrated brine, dried over anhydrous sodium sulfate, and concentrated under reduced pressure. The residue was purified by column chromatography (PE/EtOAc = 15:1) to obtain compound **11-2-3** as a yellow oil (1.81 g, 90% yield).

### 3. Synthesis of compound 1-2-337

To a solution of **11-2-2** (435 mg, 2.6 mmol) and **11-2-3** (611 mg, 2.6 mmol) in acetonitrile (20 mL), was added cesium carbonate (938 mg, 2.88 mmol), and then the mixture was allowed to react overnight at room temperature. After completion of the reaction via TLC monitoring, ethyl acetate and water were added to perform extraction three times. The organic phase was dried over anhydrous sodium sulfate, concentrated under reduced pressure, and then purified by column chromatography (PE/EtOAc = 15:1) to obtain compound **1-2-337** as a yellow oil: 782 mg, 2.56 mmol, with a yield of 85%. HRMS (ESI-TO F) *m*/*z:* [M + H]⁺ Calcd for C₁₆H₁₉NO₃SH⁺ 306.1158; Found 306.1157.

¹H NMR (400 MHz, CDCl₃) *δ* 7.37-7.31 (m, 1H), 7.12 (d, *J* = 3.2 Hz, 1H), 7.05-6.96 (m, 2H), 6.63 (d, *J* = 2.6 Hz, 1H), 6.41 (dd, *J =* 8.4, 2.6 Hz, 1H), 5.39 (s, 2H), 4.61 (s, 2H), 2.65 (s, 6H), 2.23 (s, 3H).

### Example 25. Preparation of compound 1-7-012 of the present invention

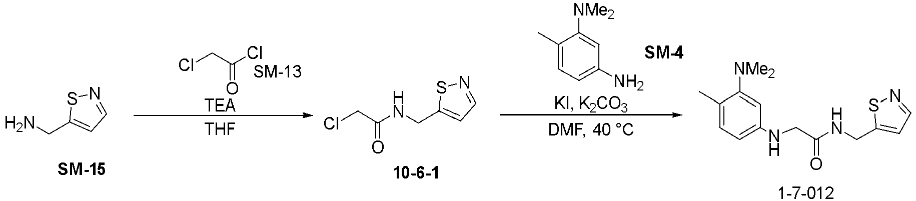

### 1. Synthesis of compound 10-6-1

Chloroacetyl chloride (160 µL, 2 mmol) was placed in a 100 mL round-bottom flask, to which was added 5 mL of tetrahydrofuran, and then the reaction was cooled to -70 °C, followed by adding a solution of **SM-15** (1.13 g, 10 mmol) and Et₃N (0.56 mL, 4 mmol) in tetrahydrofuran (5 mL) dropwise. After that, the reaction solution was warmed to room temperature and stirred. After 5 hours, the reaction solution was filtered and washed with ethyl acetate. The filtrate was concentrated. The residue was purified by silica gel column chromatography to obtain the product as a yellow oil, and then recrystallization provided product **10-6-1** as white solid (133 mg, 35% yield).

### 2. Synthesis of compound 1-7-012

**10-6-1** (133 mg, 0.7 mmol, 1.0 equiv) was dissolved in *N*,*N-*dimethylformamide (5 mL), to which were added potassium iodide (67 mg, 0.4 mmol, 0.5 equiv), potassium carbonate (304 mg, 0.88 mmol, 1.1 equiv), and **SM-4** (144 mg, 1 mmol, 1.2 equiv) in sequence. The reaction solution was stirred at 40 °C in an oil bath overnight. After diluting with ethyl acetate, the reaction solution was washed twice with saturated saline. The organic phase was dried over anhydrous sodium sulfate and concentrated. Then, the residue was purified by column chromatography, to obtain product **1-7-012** as white solid: 160 mg, 0.52 mmol, yield 75%.

¹H NMR (400 MHz, CDCl₃) *δ* 8.34 (d, *J =* 1.7 Hz, 1H), 7.38 (s, 1H), 7.06 (d, *J =* 1.7 Hz, 1H), 6.99 (d, *J* = 8.0 Hz, 1H), 6.26 (d, *J* = 2.4 Hz, 1H), 6.21 (dd, *J* = 8.0, 2.5 Hz, 1H), 4.74 (d, *J* = 6.3 Hz, 2H), 4.08 (s, 1H), 3.84 (d, *J =* 4.2 Hz, 2H), 2.64 (s, 6H), 2.21 (s, 3H). HRMS (ESI-TOF) *m*/*z:* [M + H]⁺ Calcd for C₁₅H₂₁N₄OS⁺ 305.1431; Found 305.1444.

### Example 26. Preparation of compound 1-1-250 of the present invention

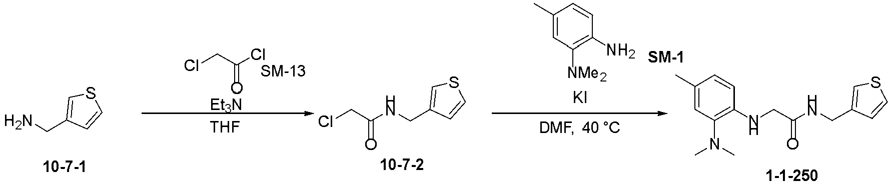

### 1. Synthesis of compound 10-7-2

Chloroacetyl chloride was placed in a 100 mL round-bottom flask, to which was added tetrahydrofuran (10 mL), and then the reaction was cooled to -70 °C, followed by adding a solution of **10-7-1** (1.13 g, 10 mmol, 1.0 equiv) and Et₃N (1.39 mL, 10 mmol, 1.0 equiv) in tetrahydrofuran (5 mL) dropwise. After that, the reaction solution was warmed to room temperature and stirred. After 3.5 hours, the reaction solution was filtered. After most of the solvent was removed by rotary evaporation of the filtrate, EtOAc was added for dilution. The resultant solution was washed with a 1:1 mixture of a saturated NaHCO₃ aqueous solution and saturated saline. The organic phase was dried over anhydrous sodium sulfate, concentrated at room temperature, and purified by silica gel column chromatography to obtain a light yellow solid. After recrystallization, a white solid product **10-7-2** (800 mg, yield 42.3%) was obtained.

### 2. Synthesis of compound 1-1-250

**10-7-2** (378 mg, 2 mmol, 1.0 equiv) was dissolved in *N,N*-dimethylformamide (5 mL), to which were added potassium iodide (166 mg, 1.0 mmol, 0.5 equiv), followed by addition of potassium carbonate (304 mg, 2.2 mmol, 1.1 equiv) and **SM-1** (300 mg, 2 mmol, 1.0 equiv). The reaction solution was stirred at 50 °C in an oil bath overnight. After diluting with ethyl acetate, the reaction solution was washed twice with saturated saline. Then, the organic phase was dried over anhydrous sodium sulfate and concentrated. The residue was purified by column chromatography, to obtain product **1-1-250** as white solid: 160 mg, 1.1 mmol, with a yield of 54%. HRMS (ESI-TOF) *m*/*z:* [M + H]⁺ Calcd for C₁₆H₁₂N₃NaOS⁺ 326.1298; Found 326.1306.

¹H NMR (400 MHz, CDCl₃) *δ* 7.26 (q, *J =* 2.9 Hz, 2H), 7.10 (s, 1H), 7.07 - 7.02 (m, 1H), 6.96 (dd, *J* = 4.9, 1.4 Hz, 1H), 6.89 (d, *J* = 2.0 Hz, 1H), 6.82 (dd, *J =* 8.1, 2.0 Hz, 1H), 6.45 (d, *J* = 8.1 Hz, 1H), 5.18 (t, *J* = 5.6 Hz, 1H), 4.49 (d, *J =* 6.0 Hz, 2H), 3.81 (d, *J =* 5.6 Hz, 2H), 2.63 (s, 6H), 2.26 (s, 3H).

### Example 27. Preparation of compound 1-7-013 of the present invention

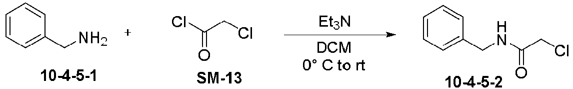

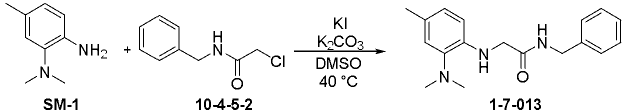

### 1. Synthesis of compound 10-4-5-2

**10-4-5-1** (2.7 mL, 20.0 mmol, 1.0 equiv) was placed in a 25 mL flask, to which was added dichloromethane (10 mL), and then **SM-13** (1.75 mL, 22.0 mmol, 1.1 equiv) and triethylamine (3.35 mL, 24.0 mmol, 1.2 equiv) were slowly added dropwise at 0 °C. The reaction was warmed to room temperature and stirred for 30 min. After completion of the reaction via TLC monitoring, the reaction was quenched by adding water, and then extracted with dichloromethane. The organic phase was washed with saturated saline, dried over anhydrous sodium sulfate, and rotatory evaporated. The residue was purified by silica gel column chromatography (DCM) to obtain white solid **10-4-5-2** (3.1 g, 16.8 mmol, yield 85%).

### 2. Synthesis of compound 1-7-013

**SM-1** (300 mg, 2.3 mmol, 1.0 equiv) was placed into a 50 mL flask, to which were added DMSO (3 mL), KI (200 mg, 1.2 mmol, 0.5 equiv), potassium carbonate (345 mg, 2.5 mmol, 1.1 equiv), and **10-4-5-2** (367 mg, 2.3 mmol, 1.0 equiv), and then the reaction was stirred at 40 °C overnight. After completion of the reaction via TLC monitoring, the reaction solution was diluted with ethyl acetate, washed twice with saturated saline, dried over anhydrous sodium sulfate, and rotatory evaporated. The residue was purified by silica gel column chromatography (PE: EtOAc = 4:1) to obtain product 1-7-013 as white solid: 384 mg, 1.3 mmol, yield 56%. HRMS (ESI-TOF) *m*/*z:* [M + H]⁺ Calcd for C₁₈H₂₄N₃O⁺ 298.1914; Found 298.1947.

¹H NMR (400 MHz, CDCl₃) *δ* 7.37 - 7.11 (m, 7H), 6.91 (d, *J* = 2.0 Hz, 1H), 6.86 (d, *J* = 8.1 Hz, 1H), 6.50 (d, *J* = 8.0 Hz, 1H), 4.52 (d, *J =* 6.1 Hz, 2H), 3.85 (d, *J* = 4.6 Hz, 2H), 2.64 (s, 6H), 2.29 (s, 3H).

### Example 28. Preparation of compound 1-7-014 of the present invention

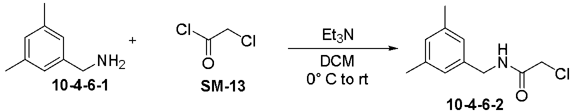

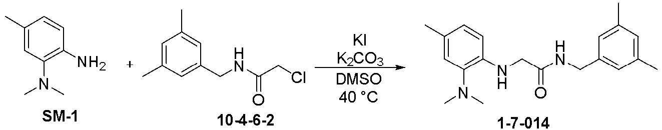

### 1. Preparation of compound 10-4-6-2

**10-4-6-1** (810 mg, 6.0 mmol, 1.0 equiv) was transferred into a 25 mL flask, to which was added dichloromethane (3 mL), and then **SM-13** (0.53 mL, 6.6 mmol, 1.1 equiv) and triethylamine (1.0 mL, 1.2 mmol, 1.2 equiv) were slowly added dropwise at 0 °C. The reaction was warmed to room temperature and stirred for 30 min. After completion of the reaction via TLC monitoring, the reaction was quenched by adding water, and then extracted with dichloromethane. The organic phase was washed with saturated saline, dried over anhydrous sodium sulfate, and rotatory evaporated. The residue was purified by silica gel column chromatography to obtain white solid **10-4-6-2** (732 mg, yield 58%).

### 2. Preparation of compound 1-7-014

**SM-1** (375 mg, 2.0 mmol) was placed into a 50 mL flask, to which were added DMSO (3 mL) and KI (208 mg, 0.5 mmol), and then stirred at room temperature for 10 min, followed by adding potassium carbonate (345 mg, 2.5 mmol) and **10-4-6-2** (530 mg, 2.5 mmol). The reaction was stirred at 40 °C overnight. After completion of the reaction via TLC monitoring, the reaction solution was diluted with ethyl acetate, washed twice with saturated saline, dried over anhydrous sodium sulfate, and then evaporated to dryness. The residue was purified by silica gel column chromatography (PE: EtOAc = 4:1) to obtain white solid **1-7-014:** 665 mg, with a yield of 82%.

¹H NMR (400 MHz, CDCl₃) *δ* 7.10 (s, 1H), 6.93 - 6.88 (m, 2H), 6.88 - 6.81 (m, 3H), 6.51 (d, *J* = 8.0 Hz, 1H), 5.21 (s, 1H), 4.44 (d, *J =* 6.0 Hz, 2H), 3.91 - 3.80 (m, 2H), 2.64 (s, 6H), 2.28 (d, *J* = 3.4 Hz, 9H). HRMS (ESI-TOF) *m*/*z:* [M + H]⁺ Calcd for C₂₀H₂₈N₃O⁺ 326.2227; Found 326.2310.

### Example 29. Preparation of compound 1-2-284 of the present invention

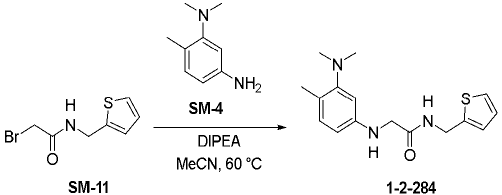

### Preparation of compound 1-2-284

**SM-11** (464 mg, 2.0 mmol), **SM-4** (300 mg, 2.0 mmol), and DIPEA (272 mg, 2.0 mmol) were dissolved in acetonitrile (30 mL) and stirred at 60 °C overnight. After completion of the reaction via TLC monitoring, saturated saline (50 mL) was added to the reaction system, and the resultant solution was extracted with CH₂Cl₂ (3 × 100 mL). The organic phase was washed with saturated saline (50 mL), dried over anhydrous sodium sulfate, filtered under suction, and concentrated under reduced pressure. The crude product was purified by reversed-phase prep-HPLC to obtain compound **1-2-284** (228 mg, yield 38%) as an oil. ESI [M + H]⁺ = 304.2.

¹H NMR (400 MHz, DMSO-*d₆*) *δ* 8.45 (t, *J* = 6.0 Hz, 1H), 7.36 (dd, *J* = 3.6, 2.4 Hz, 1H), 6.92 (dd, *J =* 4.8, 3.6 Hz, 2H), 6.82 (d, *J* = 7.6 Hz, 1H), 6.25 (d, *J* = 2.4 Hz, 1H), 6.09 (dd, *J* = 8.0, 2.4 Hz, 1H), 5.74 (t, *J* = 6.0 Hz, 1H), 4.43 (d, *J* = 6.0 Hz, 2H), 3.60 (d, *J =* 6.0 Hz, 2H), 2.53 (s, 6H), 2.09 (s, 3H)_{∘}

### Example 30. Preparation of compound 1-2-350 of the present invention

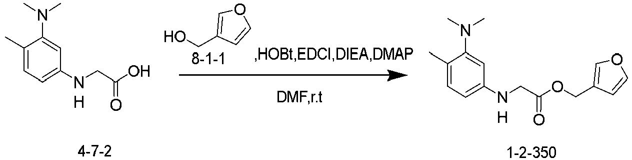

### 1. Preparation of compound 1-2-350

To a solution of **4-7-2** (3.1 g, 14.89 mmol) in N,N-dimethylformamide (DMF) (50 mL), furan-3-ylmethanol **8-1-1** (1.46 g, 14.89 mmol), HOBt (4.02 g, 29.77 mmol), EDCI (5.71 g, 29.77 mmol), DIEA (7.70 g, 59.54 mmol), and DMAP (1 g, 8.19 mmol) were slowly added. The mixture was stirred at room temperature overnight. After the reaction was complete, it was quenched with water and extracted with ethyl acetate. The organic phase was washed with saturated saline, dried over anhydrous sodium sulfate, and concentrated. After purification by column chromatography, a colorless oil, **1-2-350** (146.1 mg, yield 3.40%), was obtained. m/z calculated for [M+H]⁺ 289.2, found 289.2.

¹H NMR (400 MHz, CD₃OD_SPE) *δ 7.53* - 7.51 (m, 1H), 7.47 - 7.45 (m, 1H), 6.90 (d, *J* = 8.1 Hz, 1H), 6.43 - 6.40 (m, 1H), 6.38 (d, *J =* 2.3 Hz, 1H), 6.23 (dd, *J =* 8.1, 2.3 Hz, 1H), 5.07 (s, 2H), 3.90 (s, 2H), 2.62 (s, 6H), 2.18 (s, 3H).

### Example 31. Preparation of compound 1-7-015 of the present invention

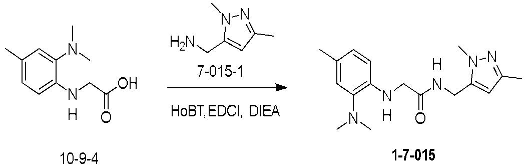

### 1. Synthesis of compound 1-7-015

To a solution of **10-9-4** (500 mg, 2.40 mmol) in dichloromethane (10 mL), were added **7-015-1** (300.52 mg, 2.40 mmol), HOBT (648.23 mg, 4.80 mmol), EDCI (917.12 mg, 4.80 mmol), and DIEA (1.58 mL, 9.60 mmol). The mixture was stirred at room temperature overnight. After completion of the reaction, water was added, and the resultant solution was extracted with dichloromethane. The organic phase was dried over anhydrous sodium sulfate and concentrated. The crude product was purified by prep-HPLC to obtain **1-7-015** as a colorless oil (135.9 mg, yield 17.95%). m/z calculated for [M+H] ⁺316.42, found 316.1.

¹H NMR (400 MHz, DMSO-*d*₆) *δ* 8.34-8.31 (m, 1H), 6.80-6.79 (m, 1H), 6.70-6.68 (m, 1H), 6.28 (d, *J* = 8.0 Hz, 1H), 5.84 (s, 1H), 5.27 (t, *J* = 12 Hz, 1H), 4.28 (d, *J* = 4 Hz, 2H), 3.67 (d, *J* = 8 Hz, 2H), 3.62 (s, 3H), 2.57 (s, 6H), 2.16 (s, 3H), 2.07-2.05 (m, 3H).

### Example 32. Preparation of compound 1-7-016 of the present invention

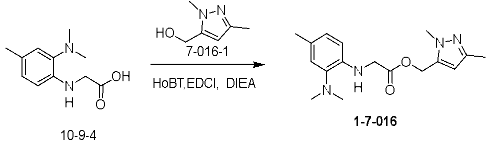

### 1. Synthesis of compound 1-7-016

To a solution of **10-9-4** (500 mg, 2.40 mmol) in dichloromethane (15 mL), were added 7-**016-1** (302.89 mg, 2.40 mmol), HOBT (648.23 mg, 4.80 mmol), EDCI (917.12 mg, 4.80 mmol), and DIEA (1.58 mL, 9.60 mmol). The mixture was stirred at room temperature overnight. After completion of the reaction, water was added, and the resultant solution was extracted with dichloromethane. The organic phase was dried over anhydrous sodium sulfate and concentrated. The crude product was purified by prep-HPLC to obtain **AL37-7-7** as a colorless oil (175.1 mg, yield 23.05%). m/z calculated for [M+H] ⁺317.40, found 317.2.

¹H NMR (400 MHz, DMSO-*d*₆) *δ* 6.81-6.80 (m, 1H), 6.69 - 6.64 (m, 1H), 6.33-6.31 (m, 1H), 6.04 (s, 1H), 5.25-5.22 (m, 1H), 5.11 (s, 2H), 3.97 (d, *J* = 8 Hz, 2H), 3.62 (s, 3H), 2.57-2.55 (m, 6H), 2.16 (s, 3H), 2.09 (s, 3H).

### Example 33. Preparation of compound 1-7-017 of the present invention

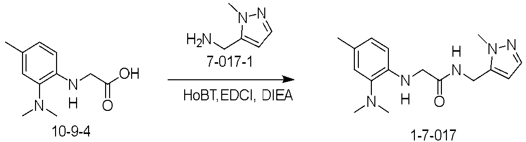

### 1. Synthesis of compound 1-7-017

To a solution of **10-9-4** (500 mg, 2.40 mmol) in dichloromethane (15 mL), were added 7-**017-1** (266.85 mg, 2.40 mmol), HOBT (648.23 mg, 4.80 mmol), EDCI (917.12 mg, 4.80 mmol), and DIEA (2 mL, 9.60 mmol). The mixture was stirred at room temperature overnight. After completion of the reaction, water was added, and the resultant solution was extracted with dichloromethane. The organic phase was dried over anhydrous sodium sulfate and concentrated. The crude product was purified by prep-HPLC to obtain**1-7-017** as a colorless oil (178.8 mg, yield 24.7%). m/z calculated for [M+H] ⁺302.39, found 302.2.

¹H NMR (400 MHz, DMSO-*d*₆) *δ* 8.58-8.55 (m, 1H), 8.47-8.46 (m, 1H), 6.81-6.80 (m, 1H), 6.72-6.69 (m, 1H), 6.30 - 6.25 (m, 2H), 5.32-5.29 (m, 1H), 4.47 (d, *J* = 4 Hz, 2H), 3.71 (d, *J* = 8 Hz, 2H), 2.58 (s, 6H), 2.17 (s, 3H).

### Example 34. Preparation of compound 1-7-018 of the present invention

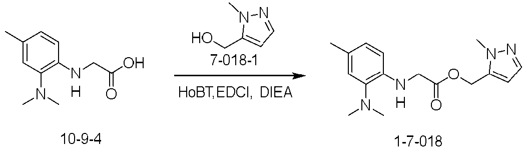

### 1. Synthesis of compound 1-7-018

To a solution of **10-9-4** (700 mg, 3.36 mmol) in dichloromethane (15 mL), were added 7-**018-1** (565.34 mg, 5.04 mmol), HOBT (907.52 mg, 6.72 mmol), EDCI (1.28 g, 6.72 mmol), and DIEA (1.3 g, 10.08 mmol). The mixture was stirred at room temperature overnight. After completion of the reaction, the reaction was quenched by adding water, and the resultant solution was extracted with dichloromethane. The organic phase was washed with saturated saline, and dried over anhydrous sodium sulfate, followed by purification over column chromatography to obtain **1-7-018** as white solid (152.52 mg, yiled 15.01%). m/z calculated for [M+H]⁺ 303.1, found 303.1.

¹H NMR (400 MHz, DMSO-*d*₆) *δ* 7.35 (d, J = 1.8 Hz, 1H), 6.81 (d, J = 1.6 Hz, 1H), 6.67 (d, J = 8.0 Hz, 1H), 6.33 (d, J = 8.0 Hz, 1H), 6.30 (d, J = 1.8 Hz, 1H), 5.24 (t, J = 6.5 Hz, 1H), 5.18 (s, 2H), 3.99 (d, J = 6.6 Hz, 2H), 3.71 (s, 3H), 2.55 (s, 6H), 2.16 (s, 3H).

### Example 35. Preparation of compound 1-7-019 of the present invention

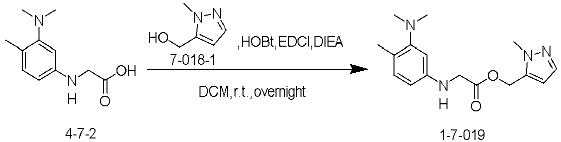

### 1. Synthesis of compound 1-7-019

To a solution of **4-7-2** (3.2 g, 15.37 mmol) in DMF (40 mL), were slowly added **7-018-1** (3.45 g, 30.73 mmol), HOBt (4.15 g, 30.73 mmol), EDCI (5.89 g, 30.73 mmol), and DIEA (7.94 g, 61.46 mmol). The mixture was stirred at room temperature overnight. After completion of the reaction, the reaction was quenched by adding water (200 mL), and the resultant solution was extracted with dichloromethane. The organic phase was washed with saturated saline, and dried over anhydrous sodium sulfate, followed by purification over column chromatography to obtain **1-7-019** as colorless oil (183.1 mg, yiled 3.94%). m/z calculated for [M+H] ⁺303.1, found 303.1. ¹H NMR (400 MHz, CD₃OD_SPE) *δ* 8.07 - 8.01 (m, 1H), 7.36 (d, *J* = 1.9 Hz, 1H), 6.86 (d, *J* = 8.1 Hz, 1H), 6.34 (d, *J* = 2.4 Hz, 1H), 6.30 (d, *J* = 1.9 Hz, 1H), 6.21 - 6.15 (m, 1H), 5.20 (s, 2H), 3.92 (s, 2H), 3.70 (s, 3H), 2.58 (s, 6H), 2.15 (s, 3H).

### Example 36. Preparation of compound 1-1-304 of the present invention

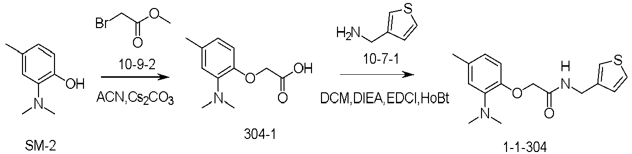

### 1. Synthesis of compound 304-1

To a solution of **SM-2** (2 g, 13.23 mmol) in acetonitrile (20 mL), were added cesium carbonate (12.93 g, 39.68 mmol) and ethyl bromoacetate **10-9-2** (2.55 mL, 15.87 mmol), and then the reaction was stirred at 85 °C for 16 h. The solvent was removed under reduced pressure to obtain the crude product, which was purified by prep-HPLC to yield a brown oily substance **304-1** (1.6 g, yield 57.81%). m/z calculated for [M+H]⁺ 210.1, found 210.1.

### 2. Synthesis of compound 1-1-304

To a solution of **304-1** (1 g, 4.78 mmol) in dichloromethane (10 mL), were added DIEA (2.47 g, 19.12 mmol), EDCI (1.29 g, 9.56 mmol), HOBT (1.83 g, 9.56 mmol), and 3-thiophenemethylamine **10-7-1** (0.65 g, 5.73 mmol). The reaction was stirred at room temperature for 16 h. The solvent was removed under reduced pressure to obtain the crude product, which was then purified by prep-HPLC to yield a colorless oily substance **1-1-304** (279.4 g, yield 19.21%). m/z calculated for [M+H]⁺ 305.0, found 305.0.

¹H NMR (400 MHz, DMSO-*d*₆) *δ* 8.83 (s, 1H), 7.52 - 7.44 (m, 1H), 7.24 (s, 1H), 6.99 (d, *J* = 4.8 Hz, 1H), 6.89 (d, *J =* 7.8 Hz, 1H), 6.78 - 6.68 (m, 2H), 4.53 (s, 2H), 4.32 (d, *J =* 5.7 Hz, 2H), 2.61 (s, 6H), 2.22 (s, 3H).

### Example 37. Preparation of compound 1-7-020 of the present invention

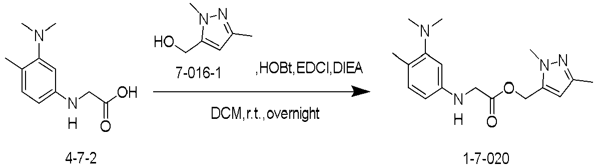

### 1. Synthesis of compound 1-7-020

To a solution of **4-7-2** (3.2 g, 15.37 mmol) in DMF (40 mL), were gradually added **7-016-1** (3.88 g, 30.73 mmol), HOBt (4.15 g, 30.73 mmol), EDCI (5.89 g, 30.73 mmol), and DIEA (7.94 g, 61.46 mmol). The mixed solution was stirred at room temperature overnight. After the reaction was complete, 150 mL of water was added to quench the reaction, followed by extraction with dichloromethane. The organic phase was washed with saturated saline solution and dried over anhydrous sodium sulfate. Purification via column chromatography yielded a colorless oily substance **1-7-020** (110.8 mg, yield 2.28%). m/z calculated for [M+H]⁺ 317.1, found 317.1. ¹HNMR (400 MHz, CD₃OD_SPE) *δ* 6.89 (d, *J* = 8.1 Hz, 1H), 6.35 (d, *J* = 2.3 Hz, 1H), 6.21 (dd, *J* = 8.1, 2.3 Hz, 1H), 6.08 (s, 1H), 5.16 (s, 2H), 3.94 (s, 2H), 3.64 (s, 3H), 2.61 (s, 6H), 2.18 (s, 6H).

### Example 38. Preparation of compound 1-2-274 of the present invention

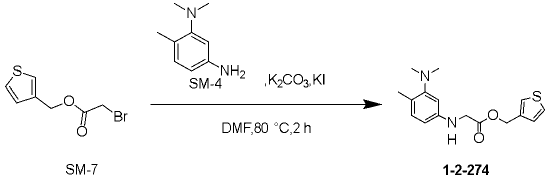

### 1. Synthesis of compound 1-2-274

To a solution of **SM-7** (2.03 g, 8.65 mmol) in N,N-dimethylformamide, were gradually added **SM-4** (1.3 g, 8.65 mmol), potassium carbonate (2.39 g, 17.31 mmol), and potassium iodide (1.44 g, 8.65 mmol). The mixed solution was stirred at 80 °C for 2 h. The reaction was quenched by adding 60 mL of water and then extracted with ethyl acetate. The organic phase was washed with saturated saline and dried over anhydrous sodium sulfate. After removing the solvent under reduced pressure, compound **1-2-274** (668.5 mg, yield 25.4%) was obtained as an orange oil after purification by column chromatography. m/z calculated for [M+H] ⁺305.0, found 305.0. ¹HNMR (400 MHz, CD₃OD) *δ* 7.35 - 7.30 (m, 1H), 7.30 - 7.25 (m, 1H), 7.03 (dd, *J* = 5.0, 1.2 Hz, 1H), 6.91 (d, *J =* 8.0 Hz, 1H), 6.39 (d, *J* = 2.4 Hz, 1H), 6.22 (dd, *J =* 8.0, 2.4 Hz, 1H), 5.16 (s, 2H), 3.89 (s, 2H), 2.60 (s, 6H), 2.20 (s, 3H).

### Example 39. Preparation of compound 1-7-021 of the present invention

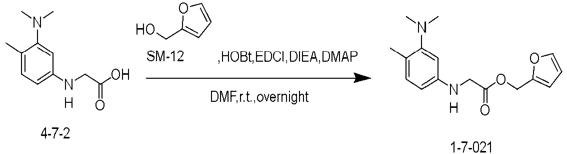

### 1. Synthesis of compound 1-7-021

To a solution of **4-7-2** (3.0 g, 14.41 mmol) in DMF (50 mL), 2-furylmethanol **SM-12** (2.83 g, 28.81 mmol), HOBt (3.89 g, 28.81 mmol), EDCI (5.52 g, 28.81 mmol), DIEA (7.45 g, 57.62 mmol), and DMAP (1 g, 8.19 mmol) were gradually added. The mixed solution was stirred at room temperature overnight. After the reaction was complete, 150 mL of water was added to quench the reaction, followed by extraction with dichloromethane. The organic phase was washed with saturated saline and dried over anhydrous sodium sulfate. Purification by column chromatography yielded a colorless oily substance **1-7-021** (235.5 mg, yield 5.67%). m/z calculated for [M+H]⁺ 289.1, found 289.1.

¹H NMR (400 MHz, CD₃OD) *δ* 7.52 - 7.49 (m, 1H), 6.90 (d, *J* = 8.2 Hz, 1H), 6.46 - 6.43 (m, 1H), 6.41 - 6.39 (m, 1H), 6.38 (d, *J* = 2.4 Hz, 1H), 6.22 (dd, *J =* 8.0, 2.4 Hz, 1H), 5.14 (s, 2H), 3.90 (s, 2H), 2.63 (s, 6H), 2.18 (s, 3H).

### Example 40. Preparation of compound 1-7-022 of the present invention

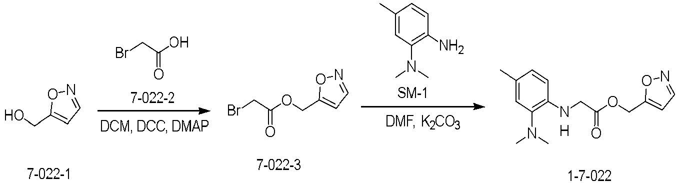

### 1. Synthesis of compound 7-022-3

To a solution of compound **7-022-1** (500 mg, 5.046 mmol), DCC (911.47 mg, 6.560 mmol), and DMAP (61.65 mg, 0.505 mmol) in DCM (10 mL), was added bromoacetic acid **7-022-2** (6.560 mmol) at 0 °C, and then the reaction was stirred for 0.5 h. After the mixed solution was heated to room temperature, it was stirred for 2 h. The residue was concentrated to dryness to obtain the crude product, which was purified by silica gel chromatography to give compound **7-022-3** (900 mg, yield 81.07%) as a brown oil. m/z calculated for [M+H]+220.1, found 221.9.

### 2. Synthesis of compound 1-7-022

To a solution of compound **7-022-3** (400 mg, 1.818 mmol) and **SM-1** (2.182 mmol) in DMF (10 mL), was added K₂CO₃ (502.51 mg, 3.636 mmol), and then the reaction was stirred at 60 °C for 1 h. After LC-MS indicated completion of the reaction, water was added, followed by extraction with ethyl acetate (10 mL x 3). The organic phase was washed with concentrated brine, dried over anhydrous sodium sulfate, and concentrated *in vacuum.* Compound **1-7-022** (196.3 mg, yield 37.32%) was obtained as a brown oil after purification by pre-HPLC. m/z calculated for [M+H]⁺ 290.1, found 290.2.

¹H NMR (400 MHz, CDCl₃) *δ* 8.25 (d, *J =* 1.6 Hz, 1H), 6.89 (s, 1H), 6.81 (s, 1H), 6.42 (d, *J* = 8.0 Hz, 1H), 6.29 (d, *J* = 1.5 Hz, 1H), 5.31 (s, 2H), 4.04 (s, 2H), 2.69 (s, 6H), 2.27 (s, 3H).

The beneficial effects of the present invention were demonstrated by the following specific experimental example.

### Experimental example 1: Study on the potent analgesic effect of the compound according to the present invention

### 1 Experimental method

Using the loss of tail withdrawal reflex (LOTWR) in rats as an indicator, the potent analgesic effect of the compounds according to the present invention was evaluated.

**1.1 Experimental animals:** Adult male SD rats weighing between 220-300 g were selected for the experiment. The rats were housed in sawdust-bedded cages with a temperature of 25+1°C, humidity ranging from 40% to 60%, and a 12-hour light/12-hour dark cycle. Each cage contained no more than 5 rats, with free access to water and food.

### 1.2 Experimental protocol:

(1) The compound of the present invention, along with the positive control drug remifentanil and the negative control (vehicle), were administered in fixed volumes. A dose escalation approach was employed to determine the minimum effective dose at which the compound of the present invention produces potent analgesic effects, as well as the minimum dose at which severe adverse reactions first appear.
(2) LOTWR lasting for 30 seconds was used as the criterion for generating potent analgesic effects. During the experiment, drugs were administered via the tail vein of rats, with a dosage volume of 0.6 mL per rat and a dosage rate of 0.02 mL/s. Subsequently, an alligator clip was applied at a distance of 1 cm from the base of the rat's tail, clamping once longitudinally and once transversely before being left in place. Meanwhile, the distal end of the alligator clip was continuously oscillated (30 times/min) to continuously generate stimulation. When the rat exhibited escape reactions (i.e., avoidance movements, struggling, and squeaking) or when the alligator clip remained on the tail for 30 seconds (to avoid tissue damage), the clip was removed. If the rat exhibited escape reactions, that time point was recorded as "no LOTWR," and the process was repeated at a 2-minute interval. When all results were "no LOTWR" after 10 minutes of testing (5 trials), the test was stopped and recorded as "ineffective."
(3) All compounds would undergo a dose escalation for efficacy evaluation, starting from 1 mg/kg (due to the high potency of remifentanil, the lowest dose was set at 1 µg/kg), followed by 5 mg/kg, 10 mg/kg, 20 mg/kg, and so on (increasing by 20 mg/kg increments after 20 mg/kg). The dose escalation would be stopped when rats show signs of death, and the lowest effective dose and the lowest dose at which severe adverse reactions occur would be recorded throughout the process. In this study, severe adverse reactions are defined as apnea, generalized tonic rigidity, convulsions, opisthotonus, seizures, etc.

**1.3 Evaluation indicators:** the minimum effective dose; the minimum dose resulting in serious adverse reactions; the safety therapeutic index = "the minimum dose resulting in serious adverse reactions" / "the minimum effective dose".

### 2 Experimental results

**Table 1. The efficacy and safety margin (single intravenous injection) of the compounds of the present invention in rats.**

| Example No. | The minimum effective dose | The safety therapeutic index |
|---|---|---|
| Remifentanil | A (10 µg/kg) | +(2) |
| Vehicle | - | - |
| Example 1 | A | +++ |
| Example 2 | D | ++ |
| Example 3 | A | +++ |
| Example 4 | A | +++ |
| Example 5 | C | + |
| Example 6 | A | ++ |
| Example 7 | C | ++ |
| Example 8 | B | +++ |
| Example 9 | A | +++ |
| Example 10 | A | +++ |
| Example 11 | A | +++ |
| Example 12 | B | +++ |
| Example 13 | D | + |
| Example 14 | D | ++ |
| Example 15 | D | ++ |
| Example 16 | D | + |
| Example 17 | B | +++ |
| Example 18 | D | + |
| Example 19 | D | ++ |
| Example 20 | D | + |
| Example 21 | D | + |
| Example 22 | D | + |
| Example 23 | D | + |
| Example 24 | D | + |
| Example 25 | B | ++ |
| Example 26 | B | ++ |
| Example 27 | A | + |
| Example 28 | C | + |
| Example 29 | D | + |
| Example 30 | D | ++ |
| Example 31 | B | ++ |
| Example 32 | A | +++ |
| Example 33 | D | + |
| Example 34 | A | +++ |
| Example 35 | A | +++ |
| Example 36 | B | ++ |
| Example 37 | A | +++ |
| Example 38 | D | + |
| Example 39 | B | +++ |
| Example 40 | D | + |

| | | |
|---|---|---|
| Note: "-" indicates that the test has been completed, but the effect has not occurred or is not present; "A" indicates that the minimum effective dose is ≤ 10.00 mg/kg; "B" indicates that the minimum effective dose is between 10.00 mg/kg and 20.00 mg/kg inclusive; "C" indicates that the minimum effective dose is between 20.00 mg/kg and 30.00 mg/kg inclusive; "D" indicates that the minimum effective dose is >30.00 mg/kg. "+" indicates a safty therapeutic index of ≤2; "++" indicates that the safety therapeutic index is between 2 and 4 inclusive; "+++" indicates a safety therapeutic index of >4. | | |

As shown in Table 1, both the compounds of the present invention and the µ-type opioid receptor agonist remifentanil can produce precise and potent systemic analgesic effects. At the same time, as indicated in the table, compared to the µ-type opioid receptor agonist remifentanil, most of the compounds of the present invention have significantly higher therapeutic safety indices, indicating that the compounds of the present invention are safer.

The present invention provided a compound with analgesic effect. The compound of the present invention exhibited excellent analgesic effect, good safety during use, minimal toxic side effects, and no dependency during use. Therefore, the compound of the present invention had broad application prospects in the manufacture of analgesic medicaments, providing a new option for the preparation of drugs with analgesic effect in clinical practice.

## Claims

1. A compound represented by formula I, or a stereoisomer thereof, or a pharmaceutically acceptable salt thereof, or a solvate thereof, or a crystal form thereof, or a prodrug thereof, or a metabolite thereof, or a deuterated derivative thereof: 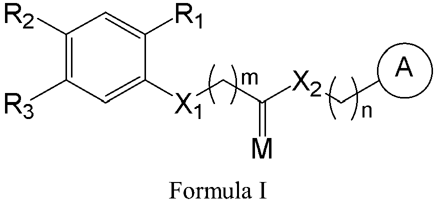 wherein,
R₁ and R₃ are each independently selected from H or NR₄R₅, and only one of R₁ and R₃ is selected from NR₄R₅;
R₂ is selected from the group consisting of H, substituted or unsubstituted C₁-C₆ alkyl, halogen, and hydroxyl;
R₄ and R₅ are each independently selected from the group consisting of substituted or unsubstituted C₁-C₆ alkyl, and substituted or unsubstituted C₁-C₆ alkoxy;
X₁ and X₂ are each independently selected from the group consisting of O, S, and NR₆;
R₆ is selected from the group consisting of H and substituted or unsubstituted C₁-C₆ alkyl;
m is selected from an integer of 1 to 5; n is selected from an integer of 0 to 5;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms ranges from 1 to 5.

2. The compound according to claim 1, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is as represented by formula II: 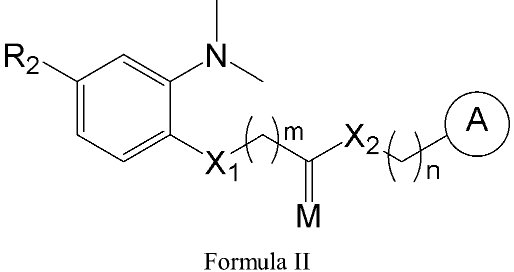 wherein,
R₂ is selected from the group consisting of H, substituted or unsubstituted C₁-C₆ alkyl, halogen, and hydroxyl;
X₁ and X₂ are each independently selected from the group consisting of O, S, and NR₆;
R₆ is selected from the group consisting of H and substituted or unsubstituted C₁-C₆ alkyl;
m is selected from 1, 2, 3, 4, or 5; n is selected from 0, 1, 2, 3, 4, or 5;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

3. The compound according to claim 2, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is as represented by formula III: 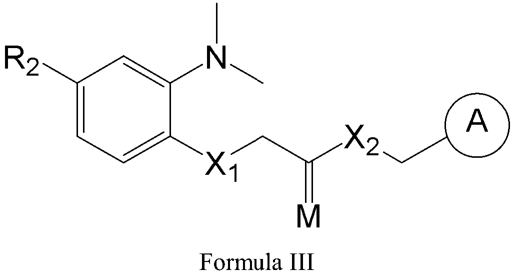 wherein,
R₂ is selected from the group consisting of H, substituted or unsubstituted C₁-C₆ alkyl, halogen, and hydroxyl;
X₁ and X₂ are each independently selected from the group consisting of O, S, and NR₆;
R₆ is selected from the group consisting of H and substituted or unsubstituted C₁-C₆ alkyl;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

4. The compound according to claim 3, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is as represented by formula Iva, formula IVb, or formula IVc: 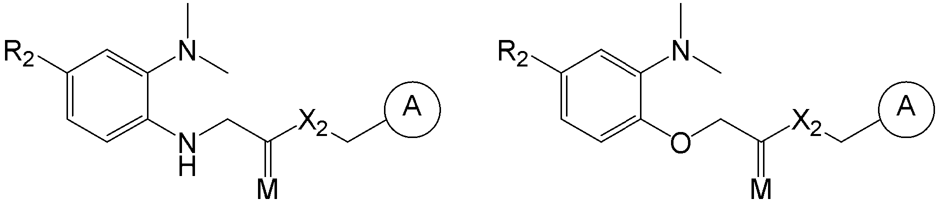 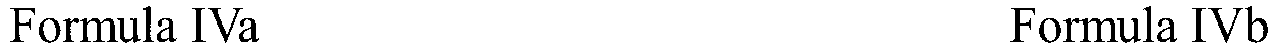 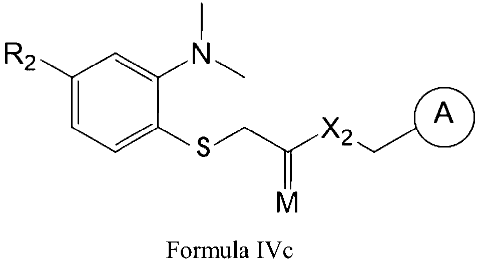 wherein,
R₂ is selected from the group consisting of H, substituted or unsubstituted C₁-C₆ alkyl, halogen, and hydroxyl;
X₂ is selected from the group consisting of O, S, and NR₆;
R₆ is selected from the group consisting of H and substituted or unsubstituted C₁-C₆ alkyl;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

5. The compound according to claim 1, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is as represented by formula V: 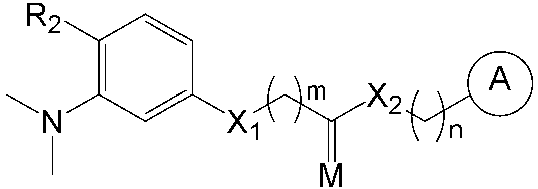 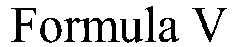 wherein,
R₂ is selected from the group consisting of H, substituted or unsubstituted C₁-C₆ alkyl, halogen, and hydroxyl;
X₁ and X₂ are each independently selected from the group consisting of O, S, and NR₆;
R₆ is selected from the group consisting of H and substituted or unsubstituted C₁-C₆ alkyl;
m is selected from 1, 2, 3, 4, or 5; n is selected from 0, 1, 2, 3, 4, or 5;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

6. The compound according to claim 5, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is as represented by formula VI: 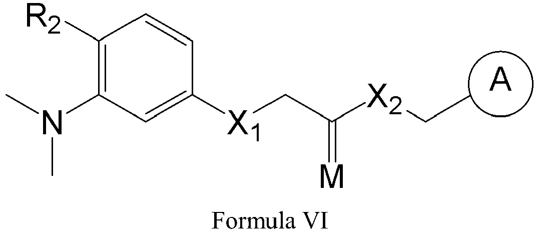 wherein,
R₂ is selected from the group consisting of H, substituted or unsubstituted C₁-C₆ alkyl, halogen, and hydroxyl;
X₁ and X₂ are each independently selected from the group consisting of O, S, and NR₆;
R₆ is selected from the group consisting of H and substituted or unsubstituted C₁-C₆ alkyl;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

7. The compound according to claim 6, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is as represented by formula VIIa, formula VIIb, or formula VIIc: 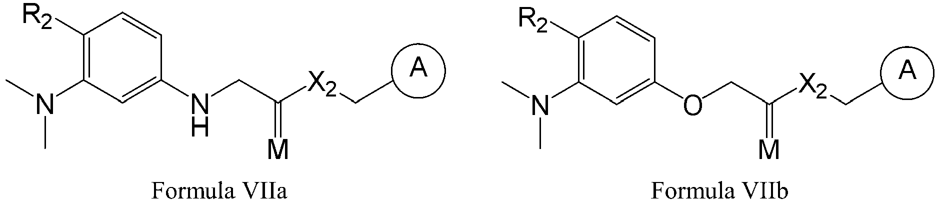 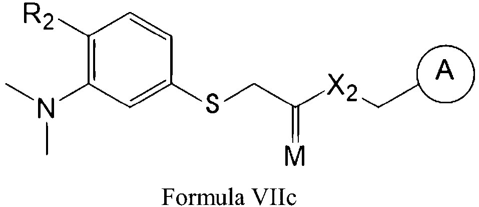 wherein,
R₂ is selected from the group consisting of H, substituted or unsubstituted C₁-C₆ alkyl, halogen, and hydroxyl;
X₂ is selected from the group consisting of O, S, and NR₆;
R₆ is selected from the group consisting of H and substituted or unsubstituted C₁-C₆ alkyl;
M is selected from the group consisting of O, S, and NR₇;
R₇ is selected from the group consisting of H, substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, and substituted or unsubstituted (5-8)-membered heterocycloalkyl;
the substituents of said alkyl and alkoxy are selected from the group consisting of deuterium, halogen, amino, nitro, cyano, carboxyl, hydroxyl, and mercapto;
the substituents of said aryl, heteroaryl, cycloalkyl, and heterocycloalkyl are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatom in said heteroaryl is selected from O, S, or N, and the number of heteroatoms is selected from 1, 2, 3, 4, or 5.

8. The compound according to any one of claims 1 to 7, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that**:
X₂ is selected from the group consisting of O, S, and NH;
and/or, M is selected from the group consisting of O, S, and NH.

9. The compound according to any one of claims 1 to 7, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that**:
ring A is selected from the group consisting of following substituted or unsubstituted groups: 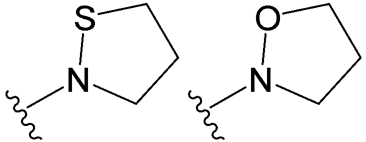 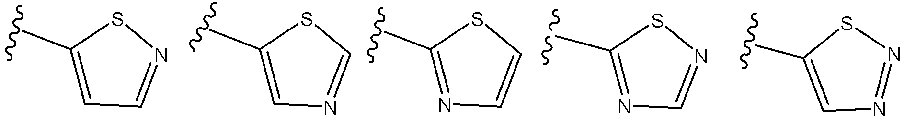 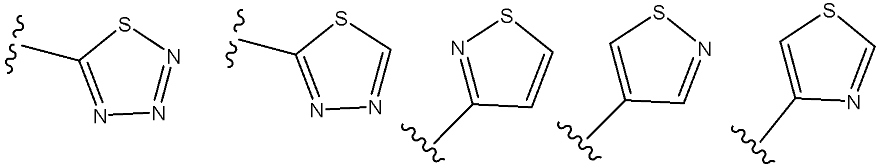 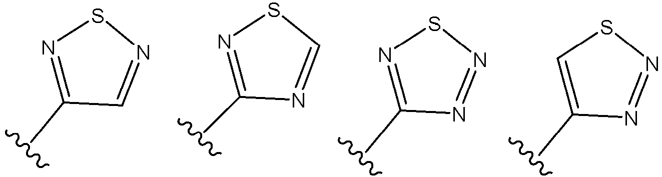 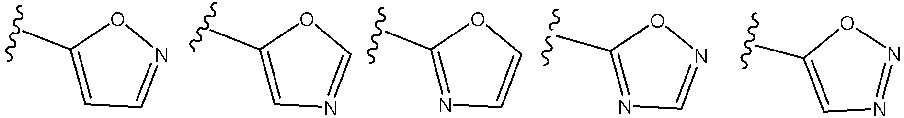 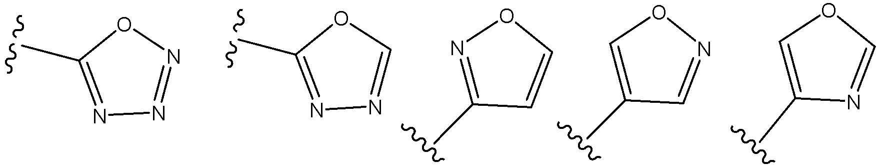 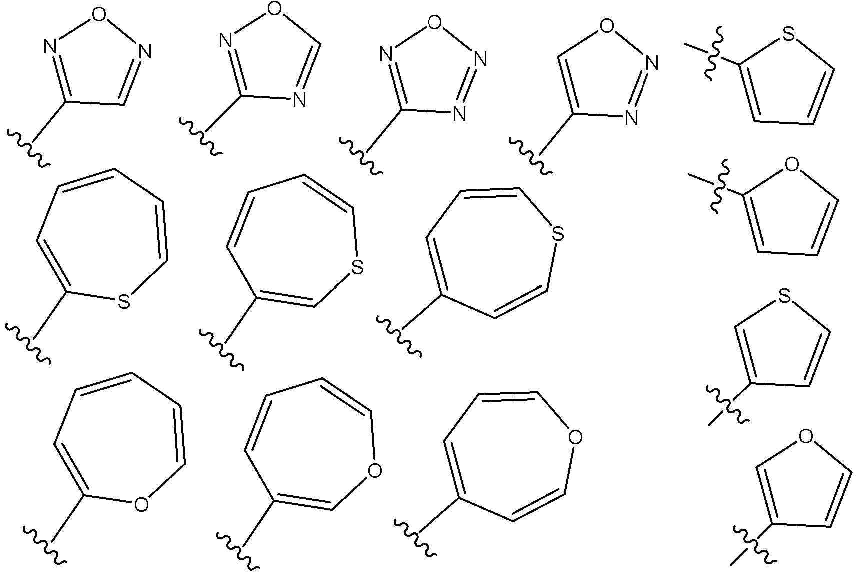 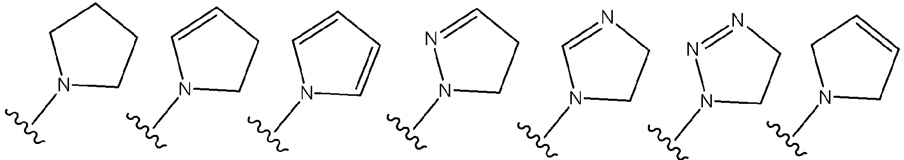 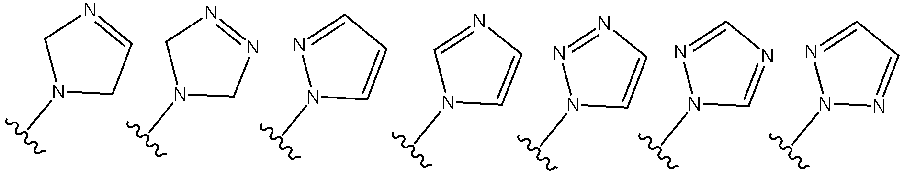 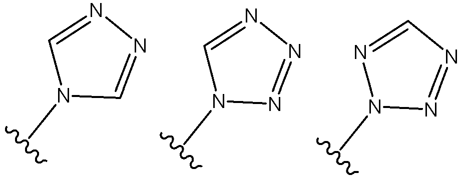 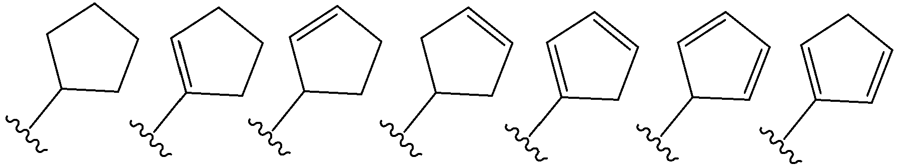 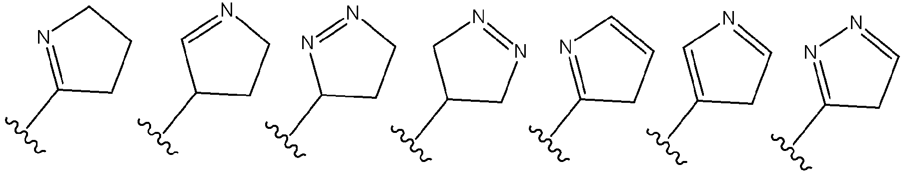 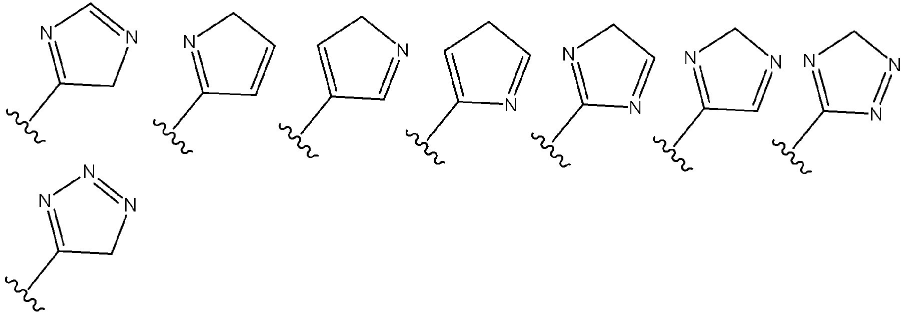 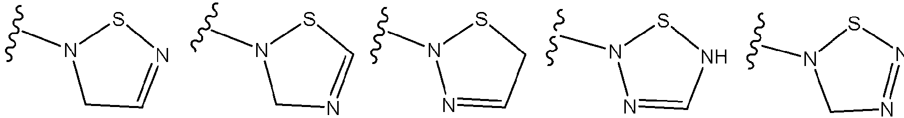 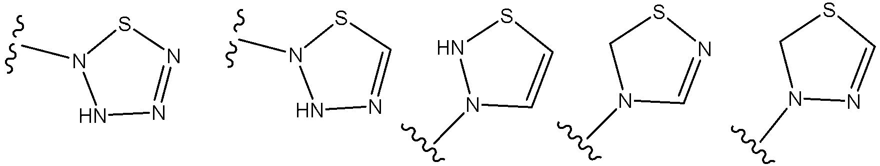 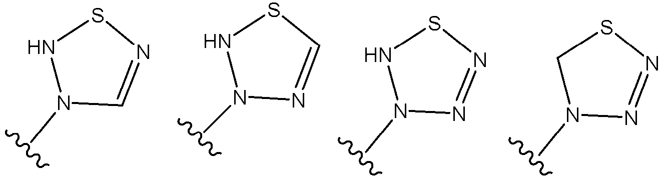 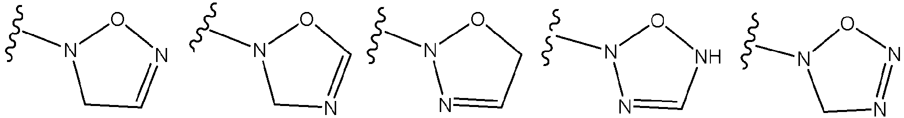 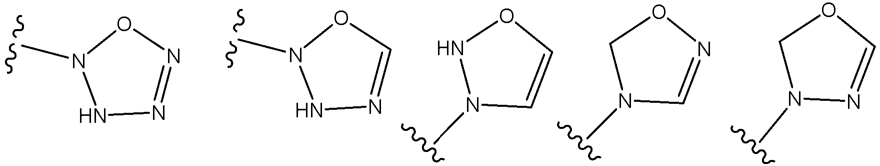 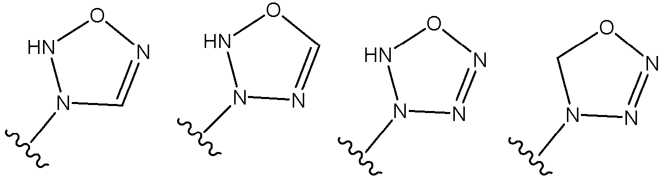 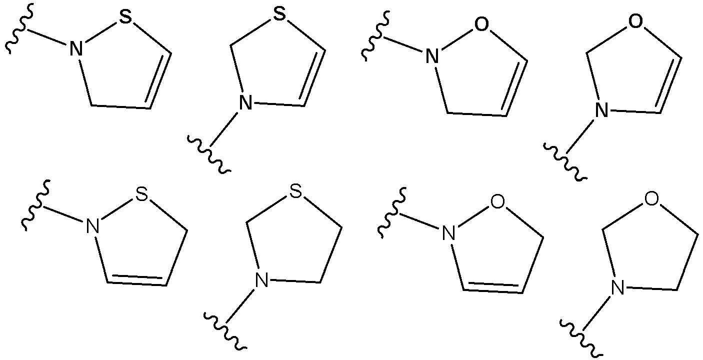 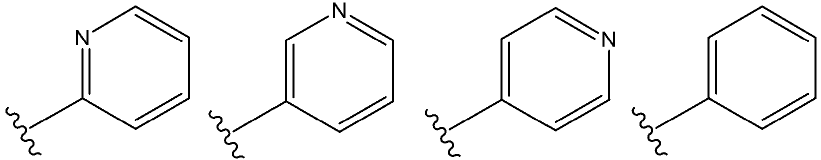 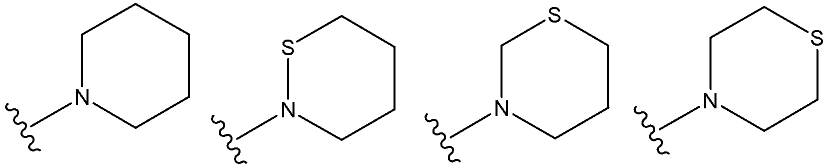 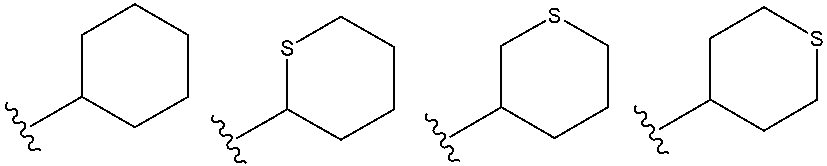 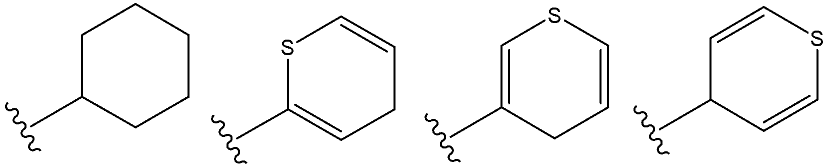 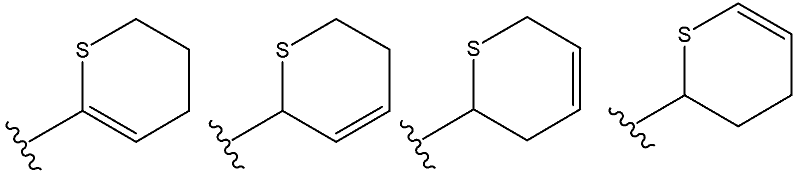 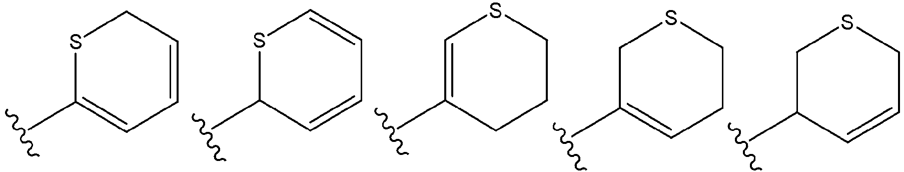 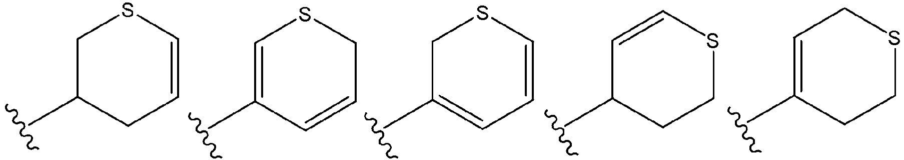 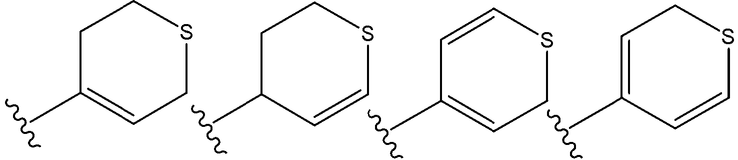 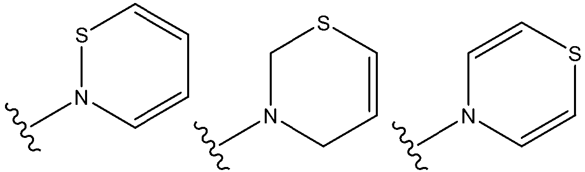 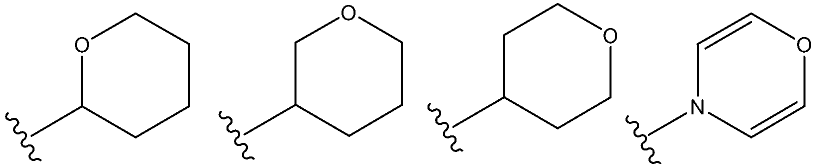 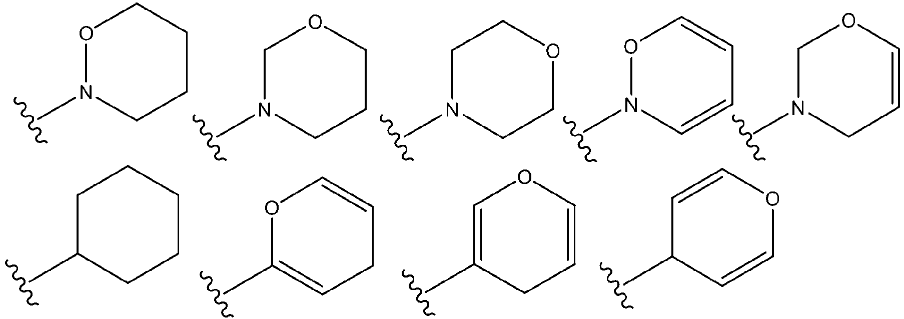 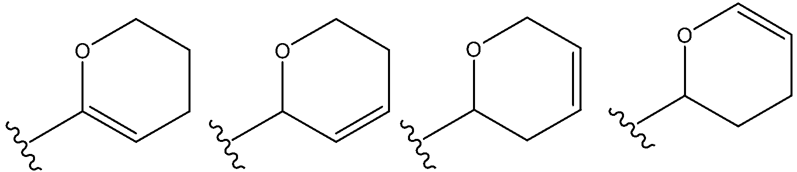 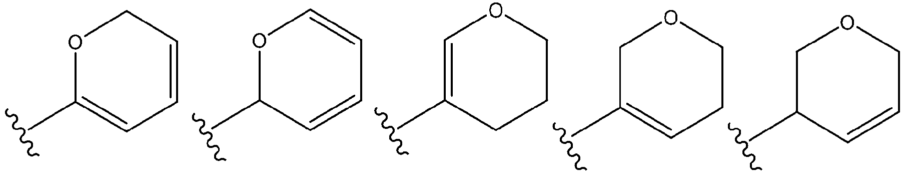 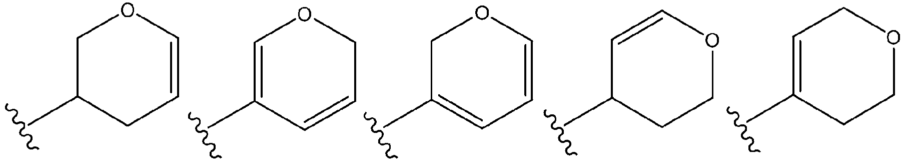 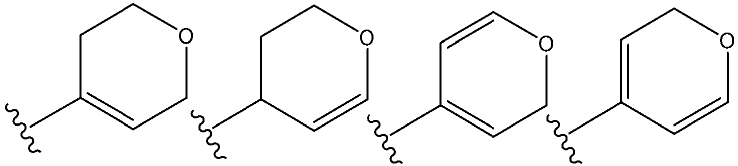 ;
the substituents of said ring A are selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the substituents of said alkyl and alkoxy are selected from the group consisting of deuterium, halogen, amino, nitro, cyano, carboxyl, hydroxyl, and mercapto;

10. The compound according to claim 9, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that**:
the substituents of said ring A are selected from the group consisting of deuterium, halogen, amino, nitro, cyano, carboxyl, hydroxyl, mercapto, substituted or unsubstituted C₁-C₄ alkyl, and substituted or unsubstituted C₁-C₄ alkoxy;
the substituents of said alkyl and alkoxy are selected from the group consisting of deuterium, halogen, amino, nitro, cyano, carboxyl, hydroxyl, and mercapto

11. The compound according to any one of claims 1 to 7, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that**: said compound is selected from the group consisting of following compounds:

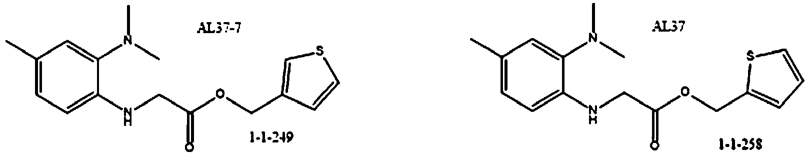

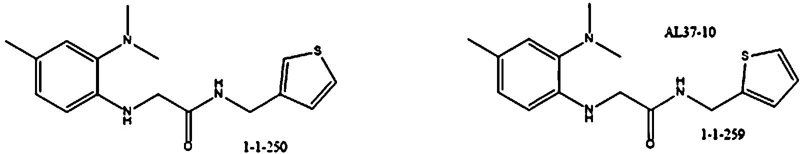

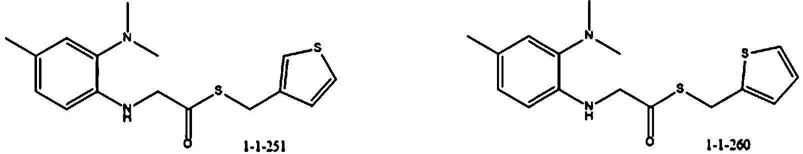

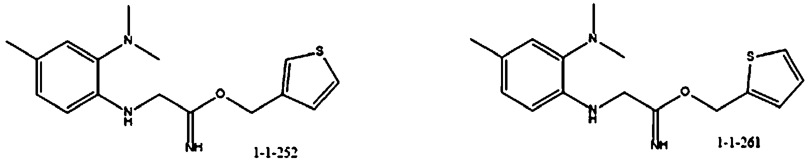

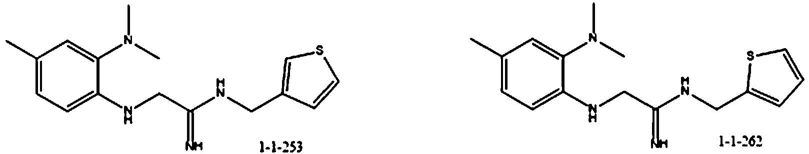

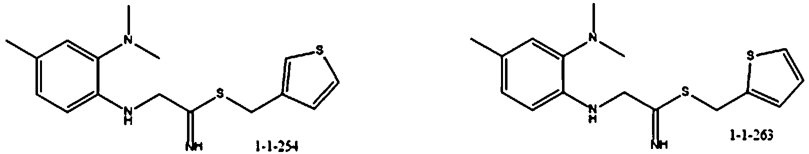

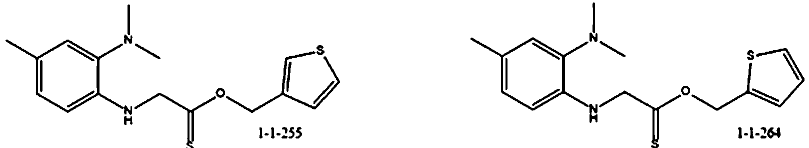

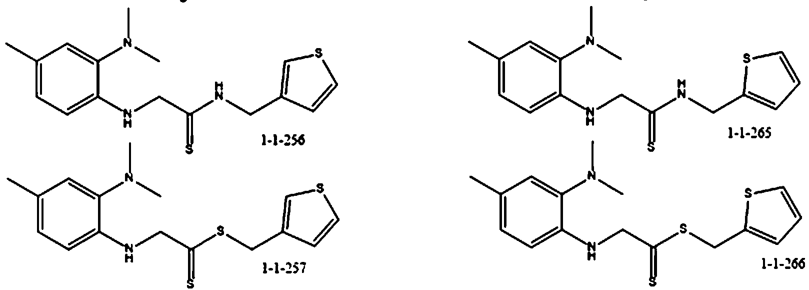

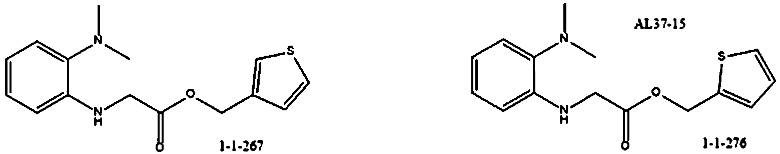

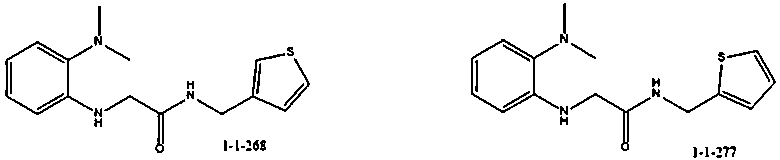

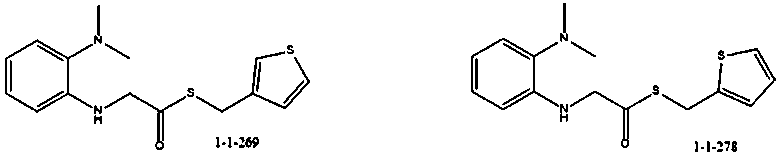

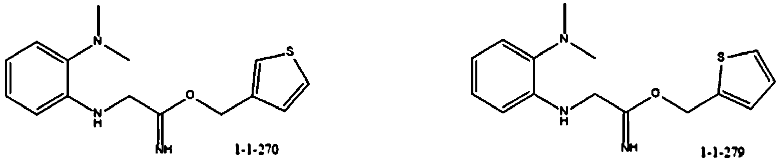

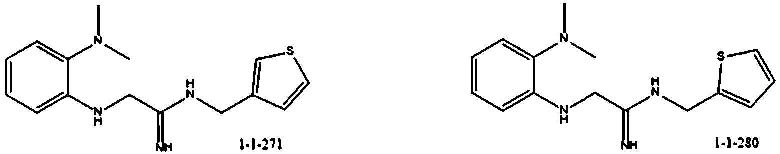

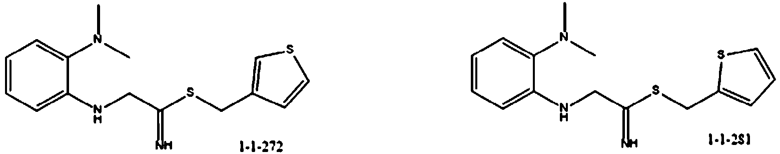

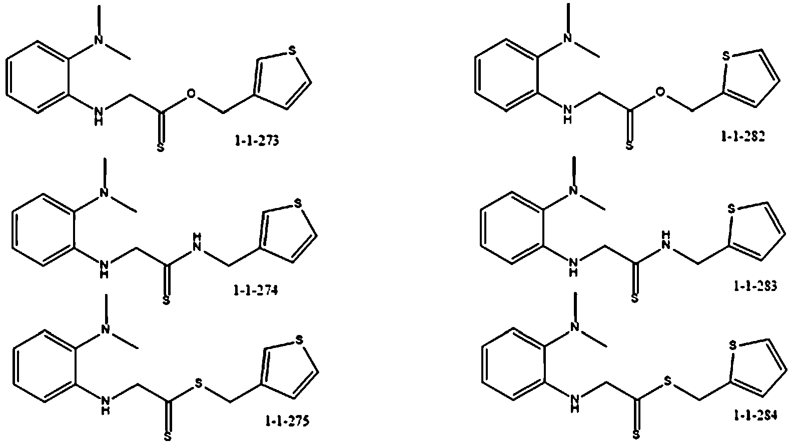

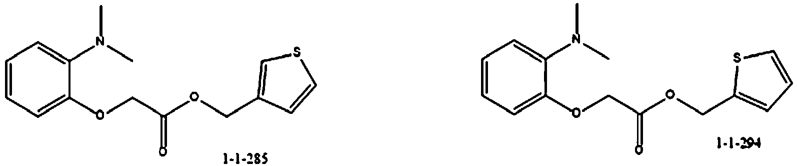

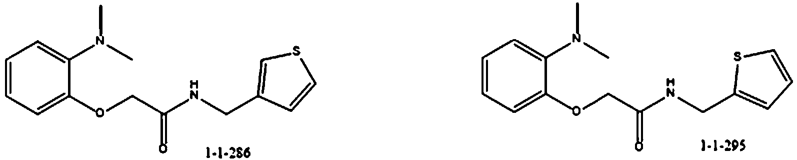

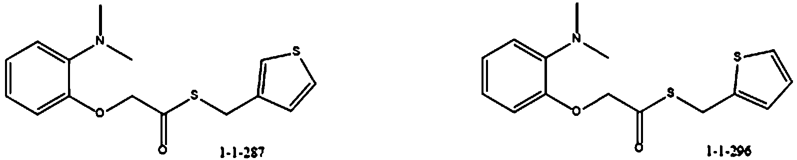

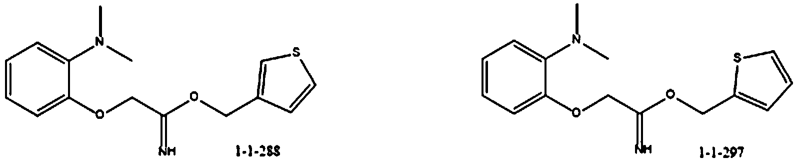

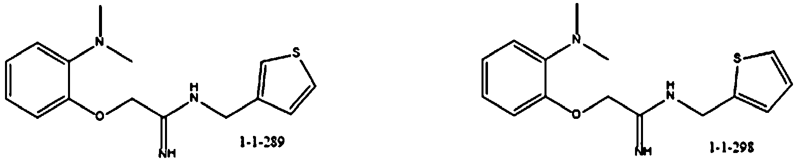

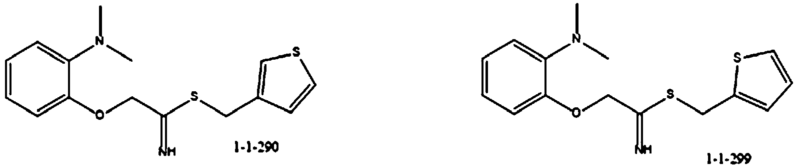

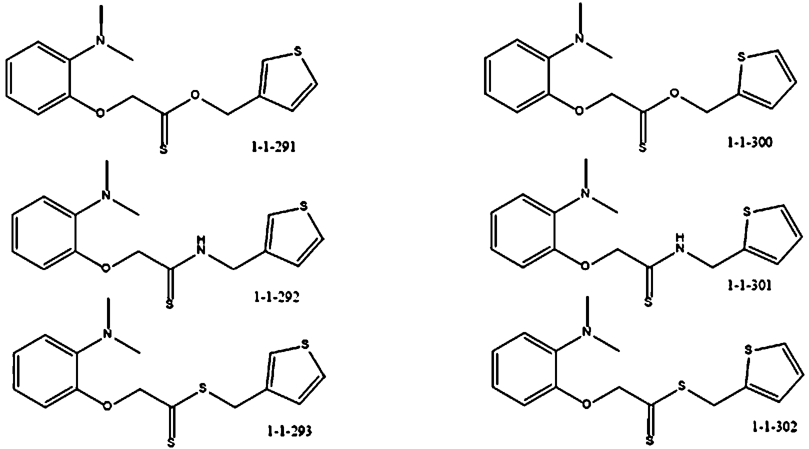

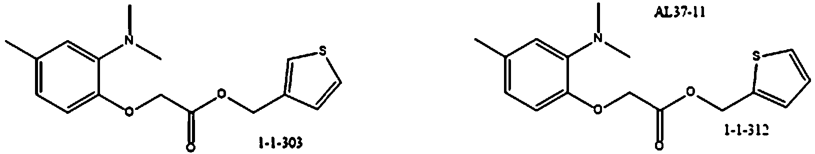

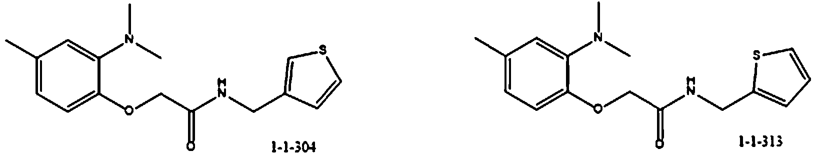

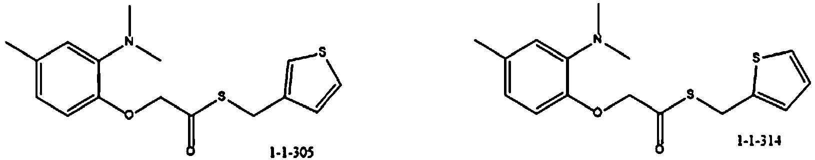

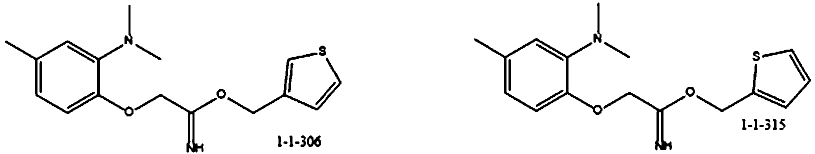

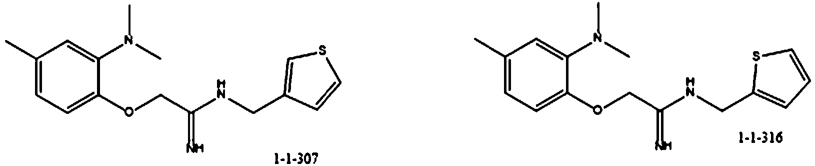

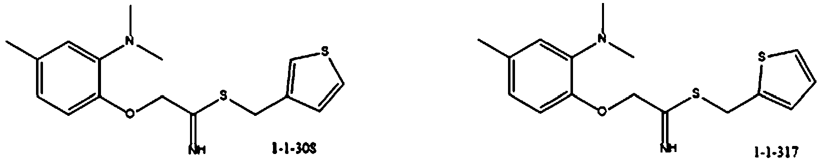

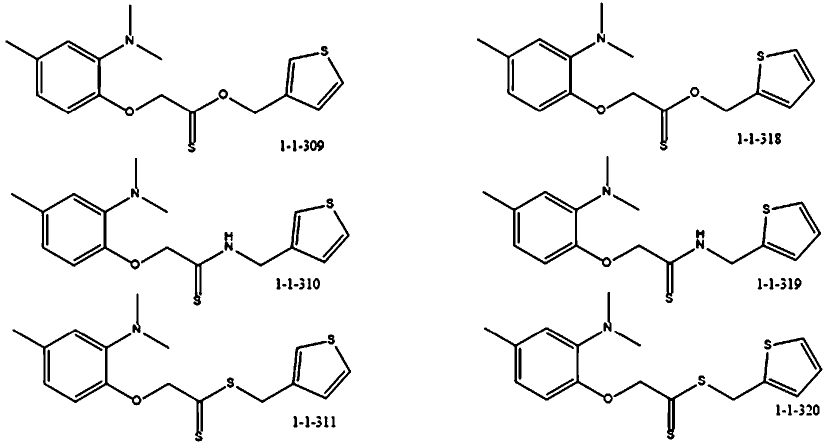

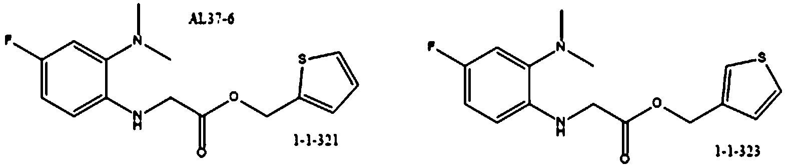

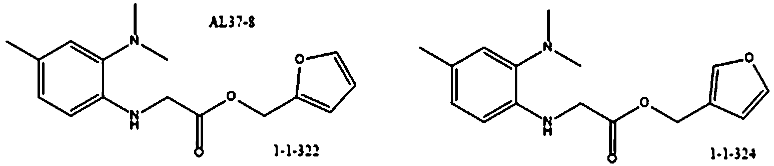

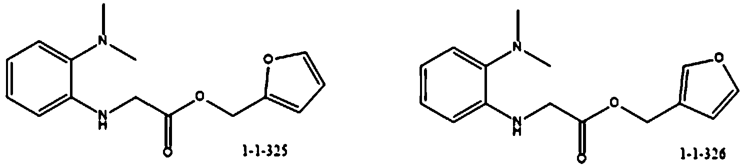

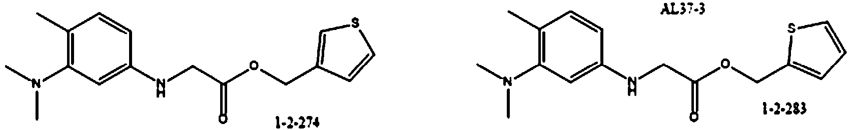

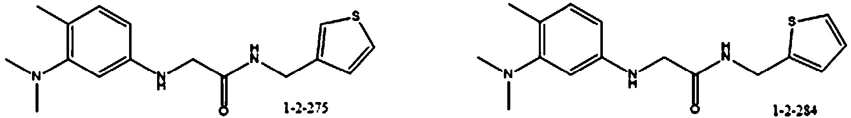

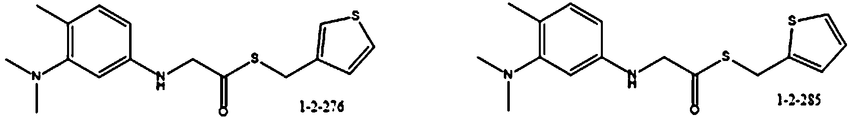

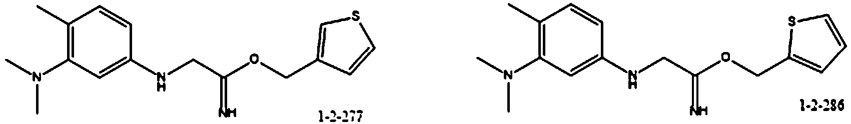

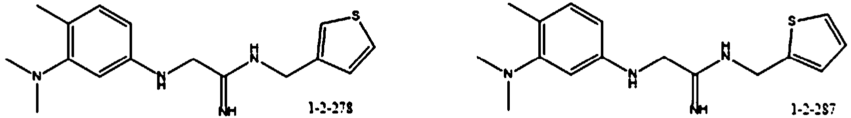

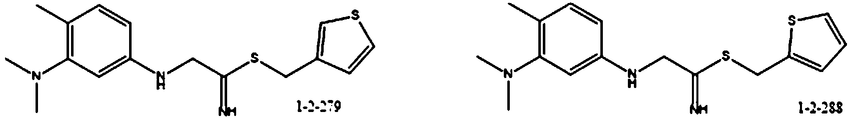

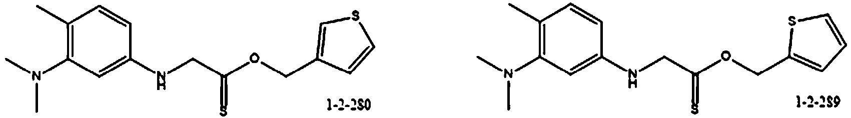

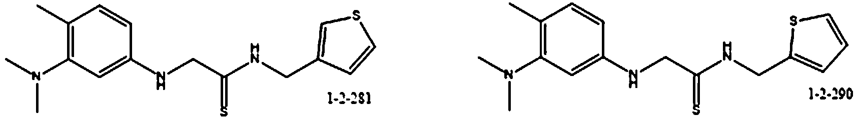

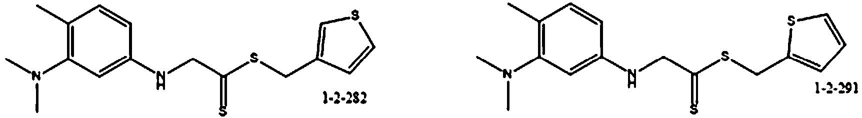

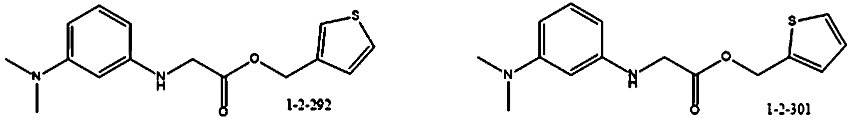

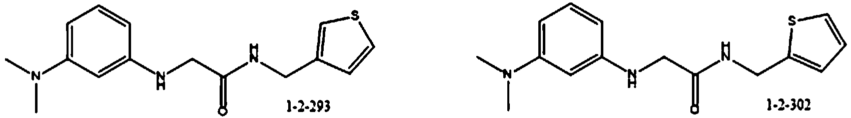

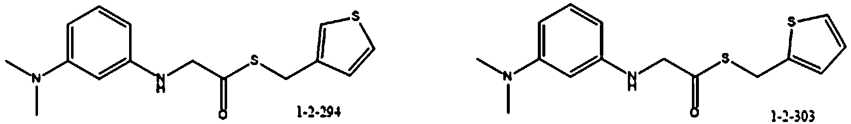

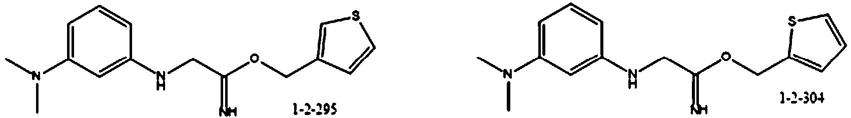

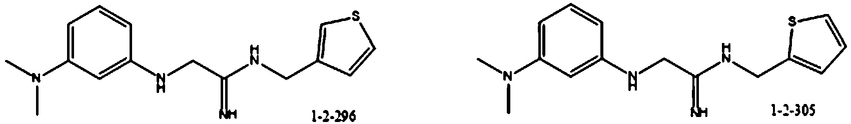

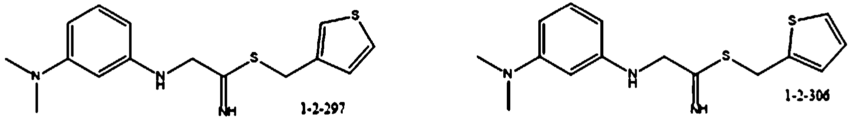

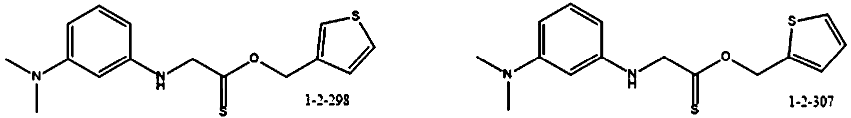

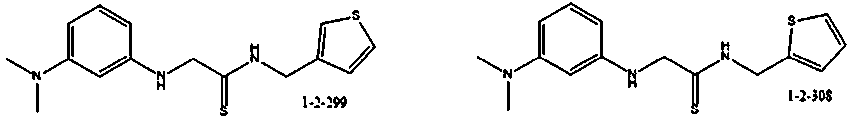

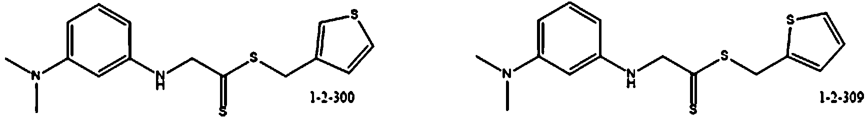

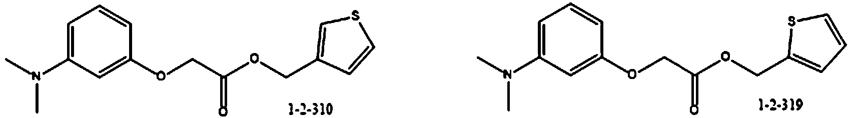

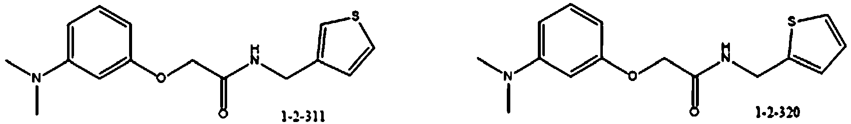

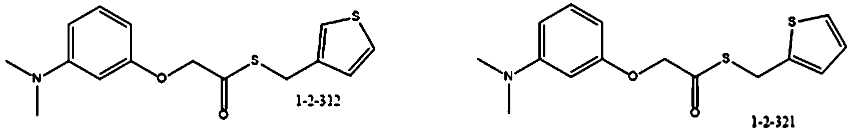

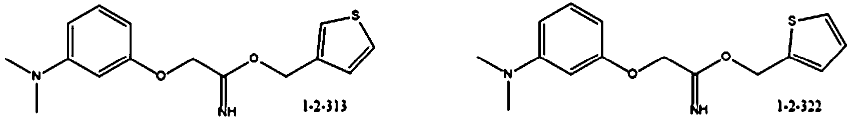

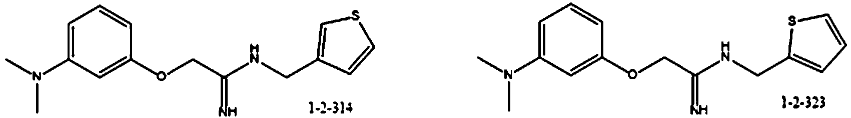

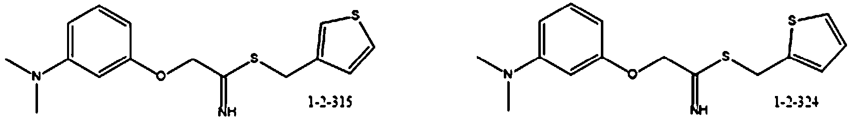

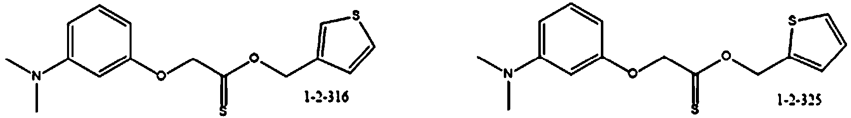

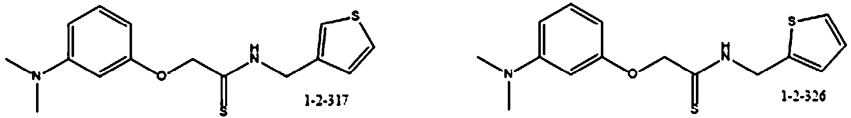

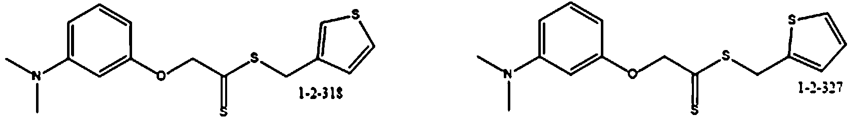

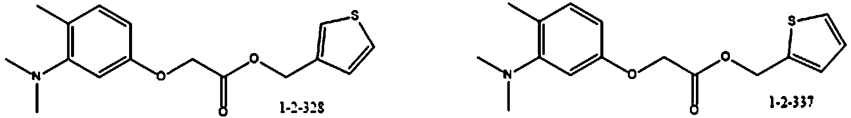

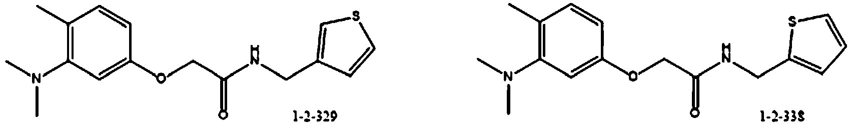

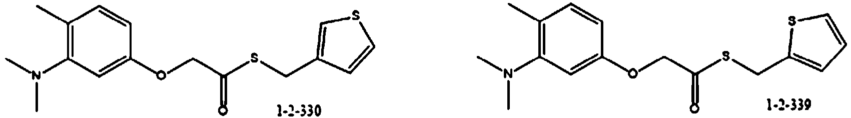

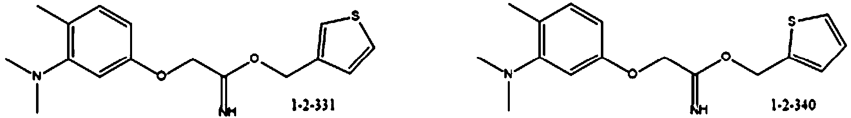

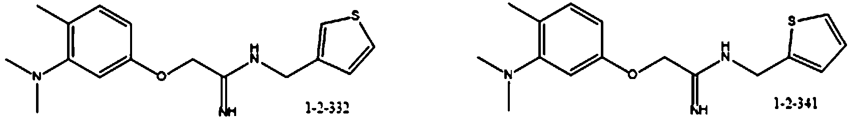

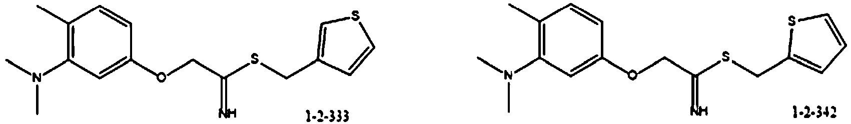

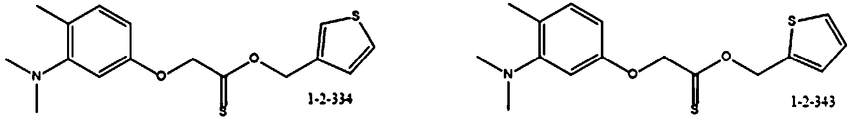

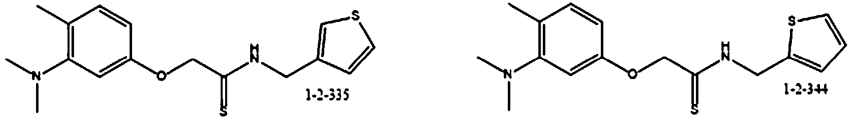

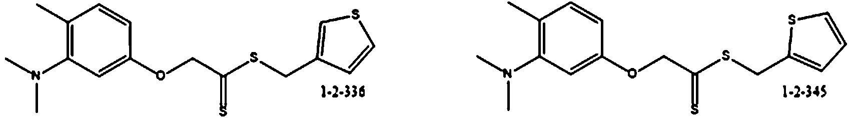

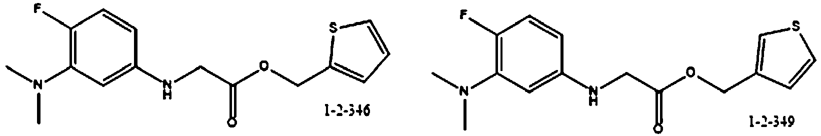

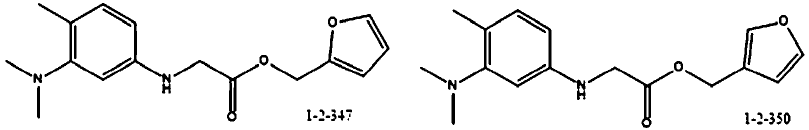

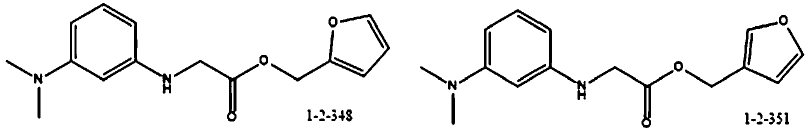

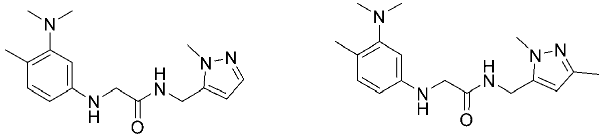

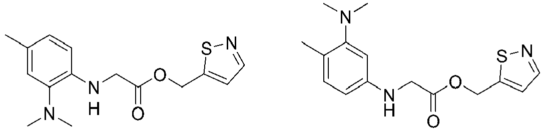

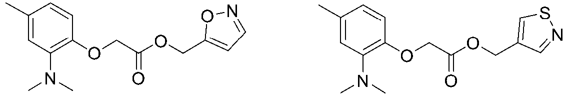

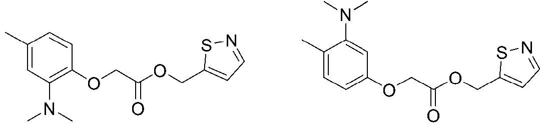

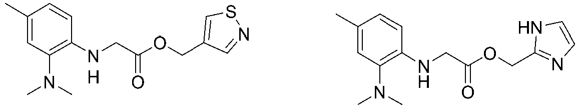

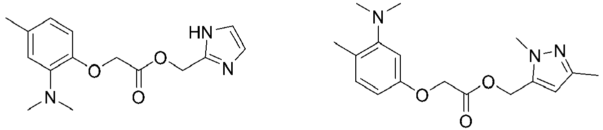

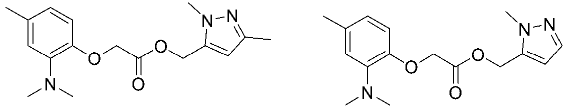

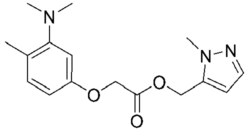

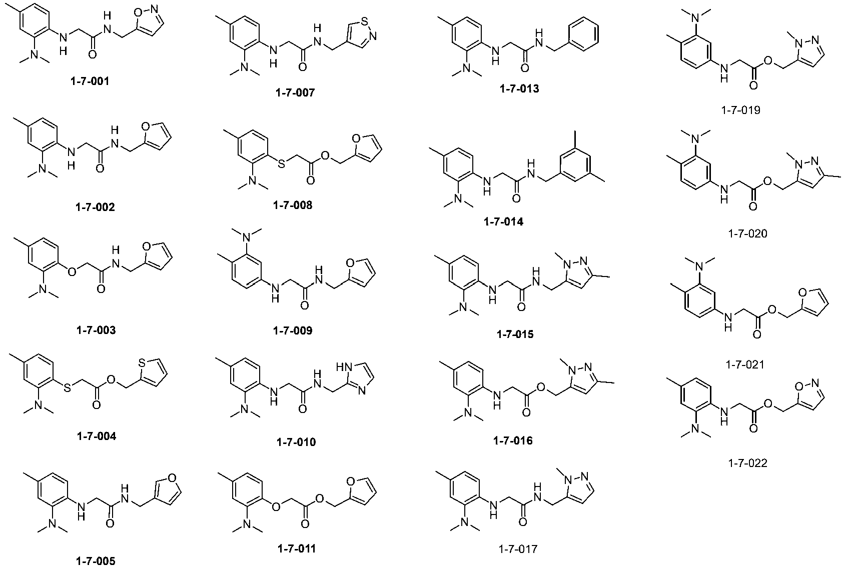

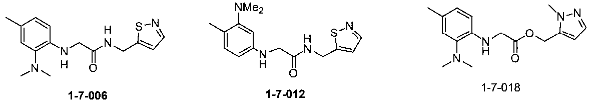

12. A method for preparing the compound according to any one of claims 2 to 11, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** it comprises the following steps:
Step 1: 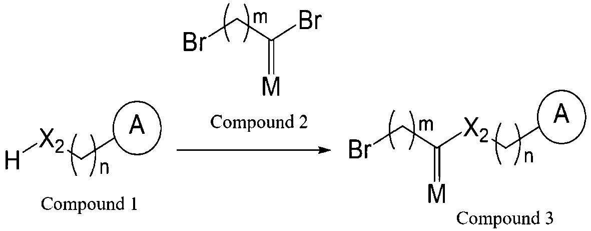 In a solvent, compound 1, an organic base, and compound 2 react to obtain compound 3; preferably, the organic base is pyridine;
wherein, n, m, X₂, A, and M are as defined in any one of claims 2 to 11;
Step 2: 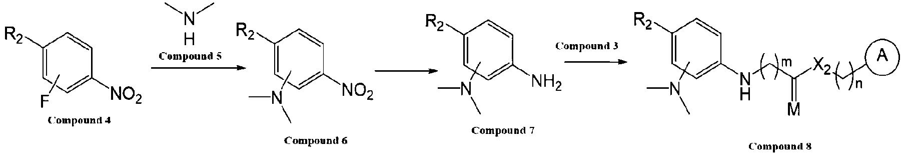
(1) In a solvent, compound 4 reacts with compound 5 to yield compound 6;
(2) In a solvent, compound 6 reacts with Pd/C in a hydrogen atmosphere to yield compound 7;
(3) In a solvent, compound 7, an organic base and compound 3 react to yield compound 8; preferably, the organic base is DIPEA;
wherein, R₂, n, m, X₂, A, and M are as defined in any one of claims 2 to 11; 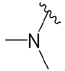
is in the ortho or meta position of R₂;
alternatively, 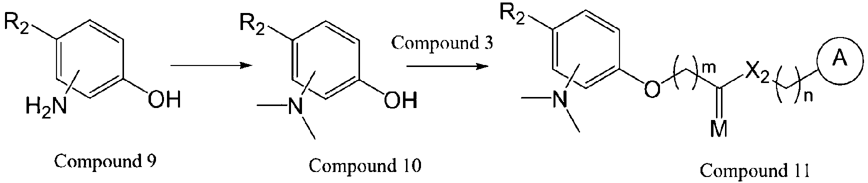
(A) In a solvent, a compound, formaldehyde aqueous solution, acetic acid, and NaBH₃CN react to yield compound 10;
(B) In a solvent, compound 10, an inorganic solid base, and compound 3 react to yield compound 11; preferably, the inorganic solid base is Cs₂CO₃;
wherein, R₂, n, m, X₂, A, and M are as defined in any one of claims 2 to 11; 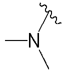
is in the ortho or meta position of R₂;
alternatively, 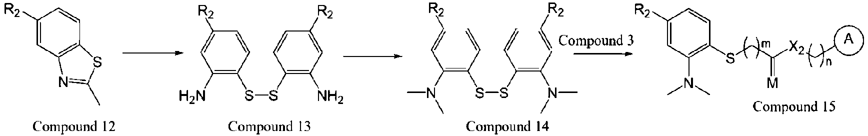
(a) In a solvent, compound 12 reacts with an NaOH aqueous solution to yield compound 13;
(b) In a solvent, compound 13, formaldehyde, and sodium cyanoborohydride react to yield compound 14;
(c) In a solvent, compound 14, sodium formaldehyde sulfoxylate, and compound 3 react to yield compound 15.

13. The use of a compound according to any one of claims 1 to 11, or a stereoisomer thereof, or a pharmaceutically acceptable salt thereof, or a solvate thereof, or a crystal form thereof, or a prodrug thereof, or a metabolite thereof, or a deuterated derivative thereof in the manufacture of a medicament having an analgesic effect.

14. A medicament, **characterized in that** it is a preparation formed by a compound according to any one of claims 1 to 11, or a stereoisomer thereof, or a pharmaceutically acceptable salt thereof, or a solvate thereof, or a crystal form thereof, or a prodrug thereof, or a metabolite thereof, or a deuterated derivative thereof, in combination with pharmaceutically acceptable excipients.

15. A pharmaceutical composition, **characterized in that** it comprises a compound according to any one of claims 1 to 11, or a stereoisomer thereof, or a pharmaceutically acceptable salt thereof, or a solvate thereof, or a crystal form thereof, or a prodrug thereof, or a metabolite thereof, or a deuterated derivative thereof.
